# EUROPÄISCHE PATENTSCHRIFT

(11) **EP 2 139 347 B1**
(45) Veröffentlichungstag und Bekanntmachung des Hinweises auf die Patenterteilung: **12.01.2011**
(21) Anmeldenummer: 08717107.0
(22) Anmeldetag: 25.02.2008
(51) Int. Cl.: A23L 1/22, A23L 1/275

(54) **VERWENDUNG VON WASSERDISPERGIERBAREN CAROTINOID-NANOPARTIKELN ALS GESCHMACKSMODULATOREN, GESCHMACKSMODULATOREN, ENTHALTEND WASSERDISPERGIERBARE CAROTINOID-NANOPARTIKEL, UND VERFAHREN ZUR GESCHMACKSMODULATION**
USE OF WATER-DISPERSIBLE CAROTINOID NANOPARTICLES AS TASTE MODULATORS, TASTE MODULATORS CONTAINING WATER-DISPERSIBLE CAROTINOID NANOPARTICLES, AND METHOD FOR TASTE MODULATION
UTILISATION DE NANOPARTICULES DE CAROTÉNOÏDES HYDRODISPERSABLES COMME MODULATEURS DE GOÛT, MODULATEURS DE GOÛT CONTENANT DES NANOPARTICULES DE CAROTÉNOÏDES HYDRODISPERSABLES ET PROCÉDÉ DE MODULATION DU GOÛT

(30) Priorität: 23.02.2007 EP 07102977; 26.11.2007 EP 07121529
(43) Veröffentlichungstag der Anmeldung: 06.01.2010
(73) Patentinhaber: B.R.A.I.N. Biotechnology Research And Information Network AG, 64673 Zwingenberg (DE)
(72) Erfinder: MATUSCHEK, Markus, 69469 Weinheim (DE); ERNST, Andreas, 67551 Worms (DE); KÖPSEL, Christian, 69469 Weinheim (DE); JAGER, Martin, B., 67677 Enkenbach-Alsenborn (DE); KLEBER, Alice, 64625 Bensheim (DE); KROHN, Michael, 64653 Lorsch (DE); ZINKE, Holger, 64673 Zwingenberg (DE)
(74) Vertreter: Vossius & Partner
(86) Internationale Anmeldenummer: PCT/EP2008/052273
(87) Internationale Veröffentlichungsnummer: WO 2008/102019

(56) Entgegenhaltungen:
- EP-A- 0 848 913
- WO-A-91/06292
- WO-A-94/19411
- WO-A-03/047550
- WO-A-03/086293
- WO-A-2007/003543
- US-A- 4 522 743
- US-A- 5 028 625

## Beschreibung

### Gebiet der Erfindung

Die vorliegende Erfindung betrifft die neuartige Verwendung von wasserdispergierbaren Carotinoid-Nanopartikeln als Geschmacksmodulatoren insbesondere zur Reduktion von bitterem Geschmack und Nachgeschmack in stofflichen Zusammensetzungen, vorzugsweise in Lebensmitteln, Getränken, Genussmitteln, Süßungsmitteln, Tiernahrungsmitteln und Kosmetika, bevorzugt in Lebensmitteln, Getränken, Genussmitteln, Süßungsmitteln, Tiernahrungsmitteln, Kosmetika und Pharmazeutika, die mindestens einen HIS (High Intensity Sweetener) enthalten.

Außerdem betrifft die vorliegende Erfindung ein neues Verfahren zur Geschmacksmodulation insbesondere zur Reduktion von bitterem Geschmack und Nachgeschmack von stofflichen Zusammensetzungen, vorzugsweise von Lebensmitteln, Getränken, Genussmitteln, Süßungsmitteln, Tiernahrungsmitteln und Kosmetika, bevorzugt von Lebensmitteln, Getränken, Genussmitteln, Süßungsmitteln, Tiernahrungsmitteln, Kosmetika und Pharmazeutika, die mindestens einen HIS (High Intensity Sweetener) enthalten, bei dem mindestens ein Typ von wasserdispergierbaren Carotinoid-Nanopartikeln als Geschmacksmodulator verwendet wird.

Nicht zuletzt betrifft die vorliegende Erfindung neue Geschmacksmodulatoren, enthaltend mindestens einen Typ von wasserdispergierbaren Carotinoid-Nanopartikeln.

### Stand der Technik

Stoffliche Zusammensetzungen wie Lebensmittel, Getränke, Genussmittel, Süßungsmittel, Tiernahrungsmittel, Kosmetika und Pharmazeutika enthalten häufig Geschmacksstoffe, die grundsätzlich unerwünscht oder in der vorhandenen Intensität zu dominant oder zu niedrig sind. Im Bereich der Süßungsmittel treten häufig neben den süßen Geschmackseindrücken weitere Geschmackseindrücke wie z.B. ein metallischer, chemischer, bitterer oder synthetischer Geschmack oder Nachgeschmack auf, die den Gesamtgeschmackseindruck der zu süßenden Zusammensetzung nachteilig beeinflussen. Im Rahmen der vorliegenden Erfindung wird unter Geschmack der unmittelbar Geschmackseindruck verstanden, der entsteht, während sich die Zusammensetzung im Mund befindet. Unter Nachgeschmack wird die Geschmackswahrnehmung nach dem Abschlucken, insbesondere nach einer Wartezeit von etwa 30 Sekunden, verstanden.

Beispielsweise sind Koffein in Tee oder Kaffee sowie Hopfenextrakte in Bier natürliche Bitterstoffe, welche jedoch in zu hoher Konzentration einen negativen Geschmackseindruck hervorrufen. In speziellen Bittergetränken wie beispielsweise Tonic Water oder Bitter Lemon ist ein charakteristischer Bittergeschmack, hervorgerufen durch den Zusatzstoff Chinin, in besonderem Maße erwünscht.

Fruchtsäfte, insbesondere Orangensaft, leiden unter der Beeinträchtigung des Geschmackes durch z.B. Flavonoidglykoside, welche einen bitteren Geschmack haben.

Zuckerfreie Getränke, die mit Süsstoffen versetzt sind, zeigen ebenfalls negative Geschmacksattribute, u.a. einen bitteren Geschmack oder Nachgeschmack. Das Mischen verschiedener Süßstoffe reduziert die negativen Geschmackseindrücke und optimiert die positiven Attribute. Allerdings gelingt es nicht, den Zuckergeschmack vollständig nachzustellen. Zudem sind einzelne Süßstoffe wie Aspartam (ASP) in bestimmten Fällen unverträglich oder chemisch instabil. Aufgrund der positiven Eigenschaften von ACK wird eine höhere Dosierung dieses Süßstoffes angestrebt. Diese höhere Dosierung ist aber aufgrund des bitteren Geschmacks dieses Süßstoffes in höheren Konzentrationen nur begrenzt möglich. Denn insbesondere die Süßstoffe Saccharin und ACK weisen vor allem in hohen Konzentrationen bittere Geschmacksattribute auf.

Auch viele pharmazeutische Wirkstoffe, insbesondere Ibuprofen haben einen stark bitteren Geschmack, welcher zur Reduktion der Akzeptanz bei der Einnahme des Wirkstoffes führt.

Zur Reduktion des natürlichen Bittergeschmacks beispielsweise von Tee, Kaffee oder Orangensaft werden diese Lebens- und Genussmittel entweder enzymatisch behandelt, um die bitter schmeckenden Substanzen zu zerstören, oder die bittere Substanz wird wie im Falle des Koffeins im Tee bzw. Kaffee durch Entkoffeinierung entfernt.

Eine weitere Möglichkeit der Modifikation des Geschmackseindrucks stellt die Zugabe von Geschmacksmodulatoren zu den gewünschten Lebensmitteln, Getränken, Genussmitteln, Tiernahrungsmitteln, Süßungsmitteln, Kosmetika und Pharmazeutika dar.

Es ist daher wünschenswert, Stoffe zu finden, die unangenehme Geschmackseindrücke unterdrücken oder reduzieren sowie gewünschte Geschmackseindrücke gezielt verstärken können.

Insbesondere im Bereich der Pharmawirkstoffe sind eine ganze Reihe von insbesondere bittermodifizierenden Substanzen bekannt. So wird beispielsweise der bittere Geschmack von Ibuprofen durch Polylysine und Polyarginine (vgl. die internationale Patentanmeldung WO 2003/086293), durch Meglumin-Salz (vgl. das amerikanische Patent US 5,028,625), durch Natriumchlorid oder Natrium-Saccharin (vgl. die internationale Patentanmeldung WO 2003/047550) oder durch Hydroxypropyl-beta-cyclodextrin oder kaubare Methacrylsäurecopolymerisate (vgl. Modifying Bitterness, Mechanism, Ingredients And Applications, Glenn Roy, 1997) maskiert, um die Einnahme durch die Patienten zu erleichtern. Auch Koffein lässt sich durch eine Vielzahl von Geschmacksmodulatoren in seiner Bitterkeit reduzieren wie beispielsweise durch Glutaminsäure, Dicalcium-disalicylat, Stärke, Lactose, Manitol sowie durch Phosphatidsäure und beta-Lactoglobulin (vgl. Glenn Roy, 1997) und weiterhin durch Hydroxybenzoesäureamide insbesondere Hydroxybenzoesäurevanillylamide (vgl. Ley et al., Journal of Agricultural & Food Chemistry, 2006).

Weitere Stoffe, die zur Reduktion eines Bittergeschmacks im Allgemeinen und insbesondere in Pharmazeutika und Lebensmittel eingesetzt wurden, sind Lecithin, Ascorbat und Citrat (vgl. die japanische Patentanmeldung JP 2001226293), Ester von Mono- oder Diglyceriden wie Glycerinmonostearat und Polycarbonsäuren wie Succinsäure (vgl. die europäische Patentanmeldung EP 0 732 064 A1), Hydroxyflavanone (vgl. die europäische Patentanmeldung EP 1 258 200 A1), 2-Phenyl-4-chromanonderivate (vgl. die deutsche Patentanmeldung DE 101 22 898), Natriumsulfathydrat (vgl. die japanische Patentanmeldung JP 02025428). Aus dem amerikanischen Patent US 5,637,618 ist weiterhin die Verwendung von Benzoesäurederivaten zur Reduktion des Bittergeschmacks in Getränken sowie von Süßstoffen und von Kaliumchlorid bekannt. Der Bittergeschmack von Kaliumchlorid wird auch mit Hilfe von 2,4-Dihydroxybenzoesäure, Carrageenan und Thaumatin gehemmt (vgl. Glenn Rog, 1997; das amerikanische Patent US 5,637,618 sowie die japanischen Patentanmeldungen JP 04262758 und JP 07083684).

Die bekannten Geschmacksmodulatoren befriedigen aber nicht in vollem Umfang, insbesondere wenn sie zur Reduktion des Bittergeschmacks von stofflichen Zusammensetzungen, wie z.B. Lebensmittel, Getränke, Genussmittel, Süßungsmittel, Tiernahrungsmittel, Kosmetika und Pharmazeutika, die mindestens einen HIS, insbesondere aber ACK, enthalten, insbesondere aber von HIS enthaltenden Erfrischungsgetränken verwendet werden sollen. So ist ihre die Bitterkeit reduzierende Wirkung häufig nicht ausreichend. Wird aus diesem Grunde die Konzentration der bekannten Geschmacksmodulatoren erhöht, um eine ausreichende Wirkung zu erzielen, kann es zu unerwünschten physikalischen und/oder chemischen Wechselwirkungen mit den übrigen Bestandteilen der jeweiligen Zusammensetzungen und/oder zu einer nachteiligen Beeinflussung, insbesondere zu einer Verschlechterung bis hin zu einer völligen Verfälschung ihres charakteristischen Geschmackseindrucks, kommen.

Wasserdispergierbare Carotinoid-Nanopartikel, Verfahren zu ihrer Herstellung und ihre Verwendung sind an sich bekannt.

So gehen z.B. aus der europäischen Patentanmeldung EP 0 832 569 A2, dem Aufsatz von Dieter Horn und Jens Rieger, »Organische Nanopartikel in wässriger Phase - Theorie, Experiment und Anwendung«, in Angewandte Chemie, 2001, Band 113, Seiten 4460 bis 4492, oder dem Lehrbuch von J. C. Bauernfeind, »Carotinoids as Colorants and Vitamin A Precursors. Technological and Nutritional Applications«, Chapter 2, J. C. Bauernfeind and H. Kläui, »Carotinoids as Food Color«, Seiten 92 bis 95, Academic Press, ISBN 0-12-082850-2, 1981, wasserdispergierbare Carotinoid-Nanopartikel hervor. Vorzugsweise handelt es sich dabei um sphärische oder spheroide Teilchen, die eine Partikelgröße < 1 µm, vorzugsweise bestimmt anhand elektronenmikroskopischer Aufnahmen, aufweisen. Die wasserdispergierbaren Carotinoid-Nanopartikel liegen in Formulierungen oder Suspensionen vor, die noch Zusatzstoffe wie Öle, Schutzkolloide, Stabilisatoren oder Emulgatoren enthalten. Dabei können die Carotinoide kristallin oder amorph sein.

Diese wasserdispergierbaren Carotinoid-Nanopartikel bzw. die carotinoidhaltigen Formulierungen, die sie enthalten, können als Zusatzstoffe für Lebensmittel, beispielsweise Backmischungen oder Puddingpulver, oder als Trockenpulver für die Herstellung von Präparaten zur Nahrungsergänzung mit Vitaminen im Human- und Tierbereich sowie für die Herstellung pharmazeutischer Präparate verwendet werden. Aufgrund ihrer guten Kaltwasserdispergierbarkeit eignen sie sich insbesondere als Lebensmittelfarbstoffe, speziell für Erfrischungsgetränke. Die Verwendung dieser wasserdispergierbaren Carotinoid-Nanopartikel bzw. der carotinoidhaltigen Formulierungen, die sie enthalten, als Geschmacksmodulatoren wird nicht beschrieben.

Carotinoid-Nanopartikel können aber auch in carotinoidhaltigen Formulierungen vorliegen, die O/W-Mikroemulsionen (Öl-in-Wasser-Mikroemulsionen; vgl. Römpp Online 2007, »Mikroemulsionen«) sind. Diese O/W-Mikroemulsionen enthalten Öltröpfchen eines Durchmessers < 1 µm, worin die Carotinoide molekulardispers gelöst sind. Die Verwendung dieser wasserdispergierbaren Carotinoid-Nanopartikel oder der O/W-Mikroemulsionen, die sie enthalten, als Geschmacksmodulatoren ist nicht bekannt.

Carotinoid-Nanopartikel können aber auch durch Mahlen von wässrigen Suspensionen, enthaltend Carotinoide, modifizierte Stärke und einen Zucker wie Saccharose, und anschließender Trocknung hergestellt werden, wie dies beispielsweise in der deutschen Patentanmeldung DE 10 2005 030 952 A1 beschrieben wird. Diese Carotinoid-Nanopartikeln eignen sich als Zusatzstoffe zu Lebensmittelzubereitungen, beispielsweise zur Färbung von Lebensmitteln wie Getränken, als Mittel für die Herstellung pharmazeutischer und kosmetischer Zubereitungen sowie für die Herstellung von Nahrungsergänzungpräparaten, beispielsweise von Multivitaminpräparaten im Human- und Tierbereich. Die Verwendung als Geschmacksmodulatoren wird nicht beschrieben.

Carotinoid-Nanopartikel können aber auch als wässrige Solubilisate in gemischten Micellen einer Micellengröße < 100 nm (vergleiche Römpp Online 2007, »Solubilisation« und »Micellen«) vorliegen. Beispiele solcher wässriger Solubilisate sind aus den europäischen Patentanmeldungen EP 0 800 825 A1 und EP 0 848 913 A2 bekannt. Diese Carotinoid-Nanopartikel oder ihre wässriger Solubilisate werden zu Injektionzwecken zur parenteralen Verabreichung und zum Färben von Lebensmitteln und Pharmazeutika, insbesondere zum Einfärben von Getränken, die optisch klar bleiben sollen, verwendet. Die Verwendung als Geschmacksmodulatoren wird nicht beschrieben.

Die Verwendung von beta-Carotin als Farbstoff in kalorienarmen Erfrischungsgetränken, die Süßstoffe wie ASP und ACK enthalten, ist bekannt. Ein Beispiel für solche Erfrischungsgetränke ist Coca-Cola light Sango® mit dem Geschmack von Blutorangen. Es ist nicht bekannt, in welcher Form beta-Carotin dem Erfrischungsgetränk zugesetzt wird.

Die gemeinsame Verwendung von Azofarbstoffen wie Gelb 6 und Rot 40 zur Einfärbung von Erfrischungsgetränken, die ACK als HIS enthalten, ist ebenfalls bekannt. Ein Beispiel für ein solches Produkt ist Diet Sunkist® Orange Soda. Es ist nicht bekannt, ob die eingesetzten Azofarbstoffe auch eine Reduzierung des bitteren Geschmacks und des bitteren Nachgeschmacks von ACK bewirken. Noch weniger ist bekannt, ob sich durch die gemeinsame Verwendung von Azofarbstoffen und Carotinoiden eine möglicherweise vorhandene geschmacksmodulierende Wirkung verstärken lässt.

Weiter ist auch die Verwendung von Azofarbstoffen wie E 110 und E 129 zusammen mit Beta-Carotin im Lebensmittel wie Backwaren und Süßwaren sowie in Instantgetränkpulvern bekannt, allerdings immer mit einer Kombination an Süßmitteln wie ACK und ASP mit Zucker im Fall der Süßwaren und Instantgetränkepulver bzw. Stärken im Fall der Backwaren.

Somit lassen sich den vorstehend beschriebenen Stand der Technik inklusive den am Markt erhältlichen Produkten keinerlei Anregungen oder Hinweise entnehmen, wie die vorstehend geschilderten Probleme gelöst werden könnten.

### Aufgabe der Erfindung

Demgemäß lag der vorliegenden Erfindung die Aufgabe zu Grunde, Stoffe bereitzustellen, die sich hervorragend als Geschmacksmodulatoren insbesondere zur Reduktion von bitterem Geschmack und bitterem Nachgeschmack in stofflichen Zusammensetzungen, vorzugsweise in Lebensmitteln, Getränken, Genussmitteln, Süßungsmitteln, Tiernahrungsmitteln, Kosmetika und Pharmazeutika, bevorzugt in Lebensmitteln, Getränken, Genussmitteln, Süßungsmitteln, Tiernahrungsmitteln, Kosmetika und Pharmazeutika, die mindestens einen HIS (High Intensity Sweetener), insbesondere ACK, enthalten, verwenden lassen.

Dabei sollen diese Stoffe in ihrer neuartigen Verwendung als Geschmacksmodulatoren keine unerwünschten physikalischen und/oder chemischen Wechselwirkungen mit den übrigen Bestandteilen der jeweiligen stofflichen Zusammensetzungen, insbesondere der Lebensmittel, Getränken, Genussmittel, Süßungsmittel, Tiernahrungsmitteln, Kosmetika und Pharmazeutika, hervorrufen. Außerdem sollen sie deren charakteristischen Geschmackseindruck nicht nachteilig beeinflussen, insbesondere nicht verschlechtern oder völlig verfälschen.

Außerdem sollen diese Stoffe zur neuartigen Verwendung als Geschmacksmodulatoren auf der Basis an sich bekannter, leicht erhältlicher und kostengünstiger Stoffe herstellbar sein.

Außerdem lag der vorliegenden Erfindung die Aufgabe zu Grunde, ein neues Verfahren zur Geschmacksmodulation, insbesondere zur Reduktion von bitterem Geschmack und bitterem Nachgeschmack, von stofflichen Zusammensetzungen, vorzugsweise zur Geschmacksmodulation von Lebensmitteln, Getränken, Genussmitteln, Süßungsmitteln, Tiernahrungsmitteln, Kosmetika und Pharmazeutika, bevorzugt von Lebensmitteln, Getränken, Genussmitteln, Süßungsmitteln, Tiernahrungsmitteln, Kosmetika und Pharmazeutika, die mindestens einen HIS (High Intensity Sweetener), insbesondere ACK, enthalten, zu finden.

Das neue Verfahren zur Geschmacksmodulation soll bewirken, dass durch die eingesetzten Geschmacksmodulatoren keine unerwünschten physikalischen und/oder chemischen Wechselwirkungen mit den übrigen Bestandteilen der jeweiligen Zusammensetzungen, insbesondere der Lebensmittel, Getränke, Genussmittel, Süßungsmittel, Tiernahrungsmitteln, Kosmetika und Pharmazeutika, hervorgerufen werden und der charakteristische Geschmackseindruck nicht nachteilig beeinflusst, insbesondere nicht verschlechtert oder gar völlig verfälscht wird.

### Erfindungsgemäße Lösung

Demgemäß wurde die neue Verwendung von wasserdispergierbaren Carotinoid-Nanopartikeln als Geschmacksmodulatoren in stofflichen Zusammensetzungen gefunden,

Im Folgenden wird diese neue Verwendung von wasserdispergierbaren Carotinoid-Nanopartikeln als »erfindungsgemäße Verwendung« bezeichnet.

Außerdem wurde das neue Verfahren zur Geschmacksmodulation von stofflichen Zusammensetzungen gefunden, bei dem mindestens ein Typ von wasserdispergierbaren Carotinoid-Nanopartikeln den stofflichen Zusammensetzungen zugesetzt wird.

Im Folgenden wird das neue Verfahren zur Geschmacksmodulation von stofflichen Zusammensetzungen als »erfindungsgemäßes Verfahren« bezeichnet.

Nicht zuletzt wurden die neuen Geschmacksmodulatoren für stoffliche Zusammensetzungen gefunden, die
(A) mindestens einen Typ von wasserdispergierbaren Carotinoid-Nanopartikeln und
(B) mindestens eine Azoverbindung, enthaltend mindestens eine Azogruppe,
enthalten.

Im Folgenden werden die neuen Geschmacksmodulatoren für stoffliche Zusammensetzungen als »erfindungsgemäße Geschmacksmodulatoren« bezeichnet.

### Vorteile der Erfindung

Im Hinblick auf den Stand der Technik war es überraschend und für den Fachmann nicht vorhersehbar, dass die Aufgabe, die der vorliegenden Erfindung zu Grunde lag, mithilfe der erfindungsgemäßen Verwendung, des erfindungsgemäßen Verfahrens und der erfindungsgemäßen Geschmacksmodulatoren gelöst werden konnte.

Insbesondere überraschte, dass sich die erfindungsgemäß zu verwendendenwasserdispergierbaren Carotinoid-Nanopartikel, insbesondere aber die erfindungsgemäßen Geschmacksmodulatoren, hervorragend als Geschmacksmodulatoren insbesondere zur Reduktion von bitterem Geschmack und bitterem Nachgeschmack in stofflichen Zusammensetzungen, vorzugsweise in Lebensmitteln, Getränken, Genussmitteln, Süßungsmitteln, Tiernahrungsmitteln, Kosmetika und Pharmazeutika, bevorzugt in Lebensmitteln, Getränken, Genussmitteln, Süßungsmitteln, Tiernahrungsmitteln, Kosmetika und Pharmazeutika, die mindestens einen HIS (High Intensity Sweetener), insbesondere ACK, enthielten, verwenden ließen.

Dabei zeigten die erfindungsgemäß zu verwendenden wasserdispergierbaren Carotinoid-Nanopartikel, insbesondere aber die erfindungsgemäßen Geschmacksmodulatoren, keine unerwünschten physikalischen und/oder chemischen Wechselwirkungen mit den übrigen Bestandteilen der jeweiligen stofflichen Zusammensetzungen, insbesondere der Lebensmittel, Getränke, Genussmittel, Süßungsmittel, Tiernahrungsmitteln, Kosmetika und Pharmazeutika. Außerdem beeinflussten sie deren charakteristischen Geschmackseindruck nicht nachteilig, insbesondere verschlechterten oder verfälschten sie ihn nicht.

Außerdem konnten die erfindungsgemäß zu verwendenden wasserdispergierbaren Carotinoid-Nanopartikel, insbesondere aber die erfindungsgemäßen Geschmacksmodulatoren, auf der Basis an sich bekannter, leicht erhältlicher und kostengünstiger Stoffe in einfacher Weise hergestellt werden.

Des Weiteren bewirkte das erfindungsgemäße Verfahren, dass durch die eingesetzten Geschmacksmodulatoren keine unerwünschten physikalischen und/oder chemischen Wechselwirkungen mit den übrigen Bestandteilen der jeweiligen Zusammensetzungen, insbesondere der Lebensmittel, Getränke, Genussmittel, Süßungsmittel, Tiernahrungsmittel, Kosmetika und Pharmazeutika, hervorgerufen wurden und der charakteristische Geschmackseindruck nicht nachteilig beeinflusst, insbesondere nicht verschlechtert oder gar völlig verfälscht wurde.

Vor allem aber war es überraschend, dass die Geschmacksmodulation einer gegebenen stofflichen Zusammensetzung durch die erfindungsgemäße Verwendung, das erfindungsgemäß Verfahren und die erfindungsgemäßen Geschmacksmodulatoren hervorragend reproduzierbar war, was gerade im Hinblick auf die Herstellung von Massenprodukten wie Lebensmittel, Getränke, Genussmittel, Süßungsmittel, Tiernahrungsmittel, Kosmetika und Pharmazeutika ein ganz besonderer Vorteil ist.

### Ausführliche Beschreibung der Erfindung

Für die erfindungsgemäße Verwendung, das erfindungsgemäß Verfahren und die erfindungsgemäßen Geschmacksmodulatoren sind die erfindungsgemäß zu verwendenden wasserdispergierbaren Carotinoid-Nanopartikel wesentlich.

Die Carotinoid-Nanopartikel können beliebige dreidimensionale Formen, wie z.B. pyramidale, würfelförmige, octaedrische, ikosaedrische, plättchenförmige, nadelförmige, zylindrische, sphärische oder spheroide Formen aufweisen. Vorzugsweise haben sie eine sphärische, oder spheroide Form. Bevorzugt ist die sphärische Form kugelförmig.

Die Partikelgröße der Carotinoid-Nanopartikel liegt unterhalb 1 µm, vorzugsweise zwischen 10 bis 900 nm, bevorzugt zwischen 20 bis 700 nm, besonders bevorzugt zwischen 20 bis 600 nm, ganz besonders bevorzugt zwischen 20 bis 500 nm und insbesondere zwischen 20 bis 300 nm. Vorzugsweise wird die Partikelgröße anhand elektronenmikroskopischer Aufnahmen ermittelt.

Dabei sind die spheroiden Partikel vorzugsweise länglich prolat und weisen dabei bevorzugt eine Länge von 200 bis 300 nm und eine Dicke von 100 bis 150 nm auf. Vorzugsweise liegt die mittlere Partikelgrößeder Carotinoid-Nanopartikel, ermittelt durch quasielastische Lichtstreuung, zwischen 10 bis 900 nm, bevorzugt 20 bis 700 nm, besonders bevorzugt 20 bis 500 nm, insbesondere 20 bis 300 nm.

Der wesentliche Bestandteil der Carotinoid-Nanopartikel ist mindestens ein, insbesondere ein, Carotinoid.»Carotinoide« ist die Sammelbezeichnung für Carotine, eine Gruppe hochungesättigter, aliphatischer und alicyclischer Kohlenwasserstoffe und deren vielfältig abgewandelte Derivate. In der Mehrzahl handelt es sich um Tetraterpene, die sich aus 8 Isopren-Einheiten aufbauen. Die Farbigkeit der Carotinoide (gelb bis rot) beruht auf ihrer Polyen-Struktur mit zahlreichen konjugierten Doppelbindungen. Von dem Grundgerüst mit 40 Kohlenstoffatomen leiten sich nicht nur die durch Hydroxyl-, oder Oxogruppen substituierten Xanthophylle ab, sondern auch Apo-, Nor- oder Seco-Carotinoide, die verkürzte Ketten oder geöffnete Ringe aufweisen, und Retro-Carotinoide, worin die Doppelbindungen verschoben sind. Die Carotinoide können aber auch Carboxylgruppen aufweisen.

Beispiele geeigneter Carotinoide sind alpha-, beta- und gamma-Carotin, Lycopin, beta-Apo-4'-carotinal, beta-Apo-8'-carotinal, beta-Apo-12'-carotinal, beta-Apo-8'-carotinsäure, Zeaxanthin, Astaxanthin, Violaxanthin, Canthaxanthin, Citranaxanthin, Cryptoxanthin, Flavoxanthin, Rhodoxanthin, Rubixanthin, Flucoxanthin, Mutatoxanthin, Lutoxanthin, Auroxanthin, Capsanthin, Lutein, Crocetin, Neurosporen, Echinenon, Adonirubin, Torulen, Torularhodin, Bixin, Peridinin und Peridinol, wobei die Hydroxylgruppen und Carboxylgruppen enthaltenden Carotinoide verestert sein können. Insbesondere wird beta-Carotin (Provitamin A) verwendet.

Die Carotinoide können in den Carotinoid-Nanopartikeln in unterschiedlichen Aggregatzuständen vorliegen.

So können die Carotinoid-Nanopartikel fest sein. Dabei können die Carotinoide in den Carotinoid-Nanopartikeln kristallin und/oder röntgenamorph, vorzugsweise röntgenamorph, vorliegen. »Röntgenamorph« bedeutet, dass der kristalline Anteil unterhalb 10% liegt. Vorzugsweise weisen die Carotinoide dabei einen hohen Anteil an all-trans Konfiguration auf, der bevorzugt bei mindestens 50% und insbesondere bei mindestens 60% liegt. Werden die festen Carotinoid-Nanopartikel in Kombination mit mindestens einem Zusatzstoff verwendet, sind die vorzugsweise in eine Matrix aus diesem Zusatzstoff eingebettet.

Außerdem können die Carotinoide in den Carotinoid-Nanopartikeln auch in flüssigem Zustand vorliegen. Dies insbesondere dann der Fall, wenn die Carotinoide in einem flüssigen, vorzugsweise unpolaren Medium, wie z.B. ein Öl, gelöst oder in der Form von solubilisierten gemischten Micellen vorliegen.

Die Carotinoid-Nanopartikel können mithilfe verschiedener Verfahren hergestellt werden.

Vorzugsweise resultieren bei diesem Verfahren flüssige oder feste Formulierungen, enthaltend Carotioid-Nanopartikel. Bevorzugt handelt sich dabei um die festen Formulierungen (A1) und (A3), insbesondere (A1), und die flüssigen Formulierungen (A2) und (A4), insbesondere (A2).

Die festen Formulierungen (A1) können beispielsweise mithilfe der üblichen und bekannten Fällungsverfahren, wie sie in der europäischen Patentanmeldung EP 0 832 569 A2, dem Aufsatz von Dieter Horn und Jens Rieger, »Organische Nanopartikel in wässriger Phase - Theorie, Experiment und Anwendung«, in Angewandte Chemie, 2001, Band 113, Seiten 4460-4492, oder dem Lehrbuch von J. C. Bauernfeind, »Carotinoids as Colorants and Vitamin A Precursors. Technological and Nutritional Applications«, Chapter 2, J. C. Bauernfeind and H. Kläui, »Carotinoids as Food Color«, Seiten 92 bis 95, Academic Press, ISBN 0-12-082850-2, 1981, beschrieben werden, hergestellt werden. Die dabei anfallenden Suspensionen von Nanopartikeln werden mithilfe geeigneter Verfahren, beispielsweise durch Sprühtrocknung, getrocknet und in der Form von Pulvern (A1) verwendet.Vorzugsweise enthalten die festen Formulierungen (A1) mindestens einen Zusatzstoff. Bevorzugt sind in den festen Formulierungen (A1) die Carotinoid-Nanopartikel in eine Matrix aus mindestens einem Zusatzstoff eingebettet. Vorzugsweise liegt das Carotinoid in den betreffenden Carotinoid-Nanopartikeln amorph vor.

Bei den flüssigen Formulierungen (A2) handelt es sich um O/W-Mikroemulsionen, d.h. Öl-in-Wasser-Mikroemulsionen (vgl. a. Römpp Online 2007, »Mikroemulsionen«). Die Teilchengröße der dispersen Phase bzw. der Durchmesser der Öltröpfchen ist < 1 µm. Vorzugsweise liegt der Durchmesser der Öltröpfchen bei 10 bis 900 nm, bevorzugt 20 bis 700 nm und insbesondere 20 bis 500 nm.

In den flüssigen Formulierungen (A2) sind die Carotinoide in den Öltröpfchen molekulardispers gelöst.

Vorzugsweise enthalten die flüssigen Formulierungen (A2) noch mindestens einen Zusatzstoff, der die O/W-Mikroemulsionen stabilisiert, insbesondere mindestens ein Polyol.

Die festen Formulierungen (A3) sind beispielsweise durch die Mahlung von Carotinoidpartikeln in einer wässriger Suspensionen und anschließendes Trocknen herstellbar. Ein geeignetes Verfahren wird beispielsweise in der deutschen Patentanmeldung DE 10 2005 030 952 A1, Seite 4, Absatz [0032], bis Seite 5, Absatz [0043], beschrieben. Vorzugsweise enthalten die festen Formulierungen (A3) mindestens einen Zusatzstoff. Bevorzugt sind in den Formulierungen (A3) die Carotinoid-Nanopartikel in eine Matrix aus mindestens einem Zusatzstoff eingebettet. Vorzugsweise liegt das Carotinoid in den betreffenden Carotinoid-Nanopartikeln kristallin vor.

Die flüssigen Formulierungen (A4) sind wässrige Solubilisate, worin die Carotinoide in gemischten Micellen einer Micellengröße < 100 nm (vergleiche Römpp Online 2007, »Solubilisation« und »Micellen«) vorliegen.

Vorzugsweise enthalten die flüssigen Formulierungen (A4) mindestens einen Zusatzstoff, insbesondere mindestens einen Zusatzstoff, der die gemischten Micellen stabilisiert, insbesondere mindestens einen Emulgator.

Beispiele geeigneter flüssiger Formulierungen (A4) und Verfahren zu ihrer Herstellung sind aus den europäischen Patentanmeldungen EP 0 800 825 A1, Seite 2, Zeile 52, bis Seite 3, Zeile 36, und EP 0 848 913 A2, Seite 2, Seite 42, bis Seite 3, Zeile 58, bekannt.

Vorzugsweise handelt es sich bei dem mindestens einen Zusatzstoff, der für die Herstellung der flüssigen oder festen Formulierungen (A1) bis (A4), insbesondere der festen Formulierungen (A1) und der flüssigen Formulierungen (A2), verwendet wird, um einen lebensmittelrechtlich und/oder arzneimittelrechtlich zugelassenen Zusatzstoff. Bevorzugt wird der Zusatzstoff aus der Gruppe, bestehend aus Schutzkolloiden, Stabilisatoren gegen den oxidativen Abbau, Emulgatoren, Ölen, Weichmachern und Mitteln gegen das Verbacken ausgewählt.

Beispiele geeigneter Schutzkolloide sind Gelatine, Fischgelatine, Stärke, chemisch oder enzymatisch modifizierte Stärke, Dextrine, Pflanzenproteine, Pektine, Gummi Arabicum, Kasein, Kaseinat, Polyvinylalkohol, Polyvinylpyrrolidon, Methylcellulose, Carboxymethylcellulose und Alginate (vgl. R. A. Morton, Fat Soluble Vitamins, International Encyclopedia of Food and Nutrition, Band 9, Pergamon Press, 1970, Seiten 128 bis 131). Vorzugsweise sind sie in den Formulierungen in einer Menge von, bezogen auf die Formulierungen, 10 bis 80 Gew.-% enthalten.

Beispiele geeigneter Stabilisatoren sind alpha-Tocopherol, tert.-Butylhydroxy-toluol, tert.-Butylhydroxyanisol, Ascorbinsäure oder Ethoxyquin (6-Ethoxy-1,2-dihydroxy-2,2,4-trimethylchinolin).

Beispiele geeigneter Emulgatoren sind Ascorbylpalmitat, Polyglycerin-Fettsäureester, Sorbitan-Fettsäureester, Polypropylenglycol-Fettsäureester und Lecithin. Vorzugsweise werden sie in einer Menge von bis zu 200 Gew.-%, bevorzugt 10 bis 150 Gew.-% und insbesondere 20 bis 80 Gew.-%, jeweils bezogen auf die Carotinoide, verwendet. Beispiele geeigneter Öle sind Sesamöl, Maiskeimöl, Baumwollsaatöl, Sojabohnenöl, Erdnussöl sowie die Ester mittelkettiger pflanzlicher Fettsäuren. Vorzugsweise werden sie in einer Menge von bis zu 500 Gew.-%, bevorzugt 10 bis 300 Gew.-% und insbesondere 20 bis 100 Gew.-%, jeweils bezogen auf die Carotinoide, verwendet.

Ein Beispiel für ein geeignetes Polyol ist Glycerin.

Beispiele geeigneter Weichmacher sind Zucker und Zuckeralkohole, wie Saccharose, Glucose, Lactose, Invertzucker, Sorbit, Mannit und Glycerin. Vorzugsweise sind sie in den Carotinoid-Nanopartikel in einer Menge von, bezogen auf die Formulierungen 20 bis 70 Gew.-% enthalten.

Ein Beispiel für ein geeignetes Mittel gegen das Verbacken ist Tricalciumphosphat.

Der Gehalt der Formulierungen, insbesondere der vorstehend beschriebenen flüssigen oder festen Formulierungen (A1) bis (A4), an Carotinoiden kann breit variieren und daher den Erfordernissen des Einzelfalls hervorragend angepasst werden. Vorzugsweise enthalten die Formulierungen, bezogen auf ihre Gesamtmenge, 0,5 bis 30 Gew.-%, bevorzugt 1 bis 20 Gew.-% und insbesondere 5 bis 15 Gew.-% an Carotinoiden.

In ihrer erfindungsgemäßen Verwendung dienen die vorstehend beschriebenen Carotinoid-Nanopartikel, vorzugsweise die in den vorstehend beschriebenen Formulierungen (A1) bis (A4), insbesondere (A1) und (A2), enthaltenen Carotinoid-Nanopartikel der Geschmacksmodulation von stofflichen Zusammensetzungen. Insbesondere dienen sie der Reduzierung des bitteren Geschmacks und des bitteren Nachgeschmacks der stofflichen Zusammensetzungen.

Die Menge der Carotinoid-Nanopartikel oder ihrer Formulierungen, bevorzugt die Menge ihrer Formulierungen (A1) bis (A4), insbesondere die Menge ihrer Formulierungen (A1) und (A2), kann dabei breit variieren und so hervorragend den Erfordernissen des Einzelfalles angepasst werden.

Vorzugsweise werden sie in einer Menge verwendet, dass in festen stofflichen Zusammensetzungen eine Konzentration an Carotinoiden von 0,1 bis 100 ppm, bevorzugt 1 bis 50 ppm und insbesondere 2 bis 30 ppm, jeweils bezogen auf die Gesamtmenge einer stofflichen Zusammensetzung, herrscht.

Handelt es sich bei den stofflichen Zusammensetzungen um Flüssigkeiten, werden sie in einer Menge verwendet, dass in den flüssigen stofflichen Zusammensetzungen eine Konzentration an Carotinoiden von 0,1 bis 100 mg/l, bevorzugt 1 bis 50 mg/l und insbesondere 2 bis 30 mg/l herrscht.

Vorzugsweise handelt es sich bei den stofflichen Zusammensetzungen um Lebensmittel, Getränke, Genussmittel, Süßungsmittel, Tiernahrungsmittel, Kosmetika und Pharmazeutika. Bei den Getränken handelt es sich vorzugsweise um Erfrischungsgetränke, bevorzugt coffeinhaltige Erfrischungsgetränke, insbesondere Cola-Getränke.

Die stofflichen Zusammensetzungen enthalten vorzugsweise mindestens einen High Intensity Sweetener HIS als Süßungsmittel oder Süßstoff. Unter HIS versteht man Verbindungen synthetischer oder natürlicher Herkunft, die keinen oder im Verhältnis zur Süßkraft vernachlässigbaren physiologischen Brennwert besitzen (non-nutritive sweeteners) und eine um ein Vielfaches höhere Süßkraft als Saccharose aufweisen. Die Süßkraft einer Verbindung ist durch die Verdünnung gegeben, bei der sie ebenso süß wie eine Saccharose-Lösung schmeckt (isosüße Lösung; 0,1 M = 4%), d. h. eine 500fach verdünnte Lösung eines Süßstoffs schmeckt isosüß wie eine Saccharose-Lösung, wenn der Süßstoff eine Süßkraft von 500 hat.

Beispiele geeigneter HIS sind aus Römpp Online 2007, »Süßstoffe« bekannt. Vorzugsweise werden die HIS aus der Gruppe, bestehend aus Acesulfam-Kalium ACK, Aspartam ASP, Saccharin und seinen Salzen, Cyclamat und seinen Salzen, Aspartam-Acesulfam-Salz, Sucralose, Thaumatin, Stevia, Steviosid und Neohesperidin-Dihydrochalkon, bevorzugt ACK, ASP, Saccharin und Sucralose, besonders bevorzugt ACK und ASP, insbesondere ACK, ausgewählt. Besonders bevorzugt wird ACK in Getränken eingesetzt.

Vorzugsweise handelt es sich bei der stofflichen Zusammensetzung um eine zuckerarme Zusammensetzung, die weniger als 10 g, vorzugsweise weniger als 1 g, Zucker pro Liter bzw. pro kg Zusammensetzung enthält, insbesondere um eine zuckerfreie Zusammensetzung. Unter Zucker werden vorliegend insbesondere aber nicht ausschließlich Mono- und Di-Saccharide verstanden.

Vorzugsweise handelt es sich bei der stofflichen Zusammensetzung um eine Kohlenhydratarme Zusammensetzung, die weniger als 1 g Kohlenhydrate pro Liter bzw. pro kg Zusammensetzung enthält, insbesondere um eine Kohlenhydratfreie Zusammensetzung.

Vorteilhafterweise handelt es sich bei der stofflichen Zusammensetzung um eine Zusammensetzung mit weniger als 100 kJ, vorzugsweise weniger als 10 kJ, pro Liter oder kg Zusammensetzung. Vorzugsweise handelt es sich bei der stofflichen Zusammensetzung um eine fettarme Zusammensetzung, die weniger als 1 g Fett pro Liter bzw. pro kg Zusammensetzung enthält, insbesondere um eine fettfreie Zusammensetzung.

Bei den zuckerarmen, kohlenhydratarmen und/oder fettarmen insbesondere zuckerfreien Zusammensetzungen handelt es sich vorzugsweise um eine ACK gesüsste Zusammensetzung, insbesondere um ein ACK gesüsstes Getränk.

Im Rahmen der erfindungsgemäßen Verwendung und des erfindungsgemäßen Verfahrens kann den stofflichen Zusammensetzungen zusätzlich zu den Carotinoid-Nanopartikeln noch mindestens eine den bitteren Geschmack und Nachgeschmack reduzierende Azoverbindung mit mindestens einer Azogruppe zugesetzt werden. Vorzugsweise werden mindestens zwei, insbesondere zwei, Azoverbindungen verwendet.

Bei der Mischung aus mindestens einem Typ von Carotinoid-Nanopartikeln und mindestens einer Azoverbindung handelt es sich um einen erfindungsgemäßen Geschmacksmodulator. Dieser kann den stofflichen Zusammensetzungen als fertige Mischung in der Form einer wässrigen Dispersion oder eines Pulvers zugesetzt werden. Oder es werden den stofflichen Zusammensetzungen die einzelnen Bestandteile des erfindungsgemäßen Geschmacksmodulators gleichzeitig oder nacheinander zugesetzt.

Die Menge der Azoverbindungen kann breit variiert und so hervorragend den Erfordernissen des Einzelfalles angepasst werden. Vorzugsweise werden sie in einer Menge von 0,1 bis 100 ppm, bevorzugt 0,5 bis 50 ppm und insbesondere 1 bis 20 ppm, jeweils bezogen auf die Gesamtmenge einer stofflichen Zusammensetzung, verwendet. Handelt es sich bei der stofflichen Zusammensetzung um eine Flüssigkeit, werden die Carotinoid-Nanopartikel vorzugsweise in einer Konzentration von 0,1 bis 100 mg/l, bevorzugt 1 bis 50 mg/l und insbesondere 2 bis 30 mg/l verwendet.

Dabei kann das Gewichtsverhältnis von Carotinoid-Nanopartikeln zu Azoverbindungen ebenfalls breit variiert und den Erfordernissen des Einzelfalls hervorragend angepasst werden. Vorzugsweise liegt das Gewichtsverhältnis von Carotinoid-Nanopartikeln zu Azoverbindungen bei 10 : 1 bis 1 : 20, bevorzugt bei 5 : 1 bis 1 : 10 und insbesondere bei 4 : 1 bis 1 : 4.

Werden, was erfindungsgemäß besonders vorteilhaft ist, zwei Azoverbindungen verwendet, kann ihr Gewichtsverhältnis breit variiert und den Erfordernissen des Einzelfalls hervorragend angepasst werden. Vorzugsweise liegt das Gewichtsverhältnis bei 10 : 1 bis 1 : 10, bevorzugt 5 : 1 bis 1 : 5 und insbesondere 2 : 1 bis 1 : 2.

Vorzugsweise sind die Azogruppen der Azoverbindungen mit Arylgruppen und/oder Arylgruppen mit Heteroatomen, bevorzugt Arylgruppen, insbesondere Phenylgruppen und/oder Naphthylgruppen, verknüpft. Dabei kann oder können in einer Azoverbindung eine oder mehrere Azogruppen vorhanden sein. Diese können unabhängig voneinander mit Arylgruppen und/oder Arylgruppen mit Heteroatomen, vorzugsweise Arylgruppen, insbesondere insbesondere Phenylgruppen und Naphthylgruppen, verknüpft sein. Vorzugsweise ist mindestens eine Arylgruppe mindestens einfach substituiert. Dabei kann die eine Arylgruppe einer Azogruppe unsubstituiert sein, während die andere mehrfach substituiert ist.

Beispiele geeigneter Substituenten sind Sulfonsäuregruppen, Nitrogruppen, Alkylgruppen, Carboxylgruppen, Hydroxylgruppen, Estergruppen, Ethergruppen, primäre und sekundäre Aminogruppen, Amidgruppen, Nitrilgruppen und Halogenatome, bevorzugt Sulfonsäuregruppen, Hydroxylgruppen und Nitrogruppen, insbesondere Sulfonsäuregruppen und Hydroxylgruppen.

Vorzugsweise werden die Azoverbindungen aus der Gruppe, bestehend aus den nachstehend aufgeführten Verbindungen 1 bis 112 ausgewählt. Die Azoverbindung können ionisch oder nicht-ionisch sein und geladen oder ungeladen vorliegen.

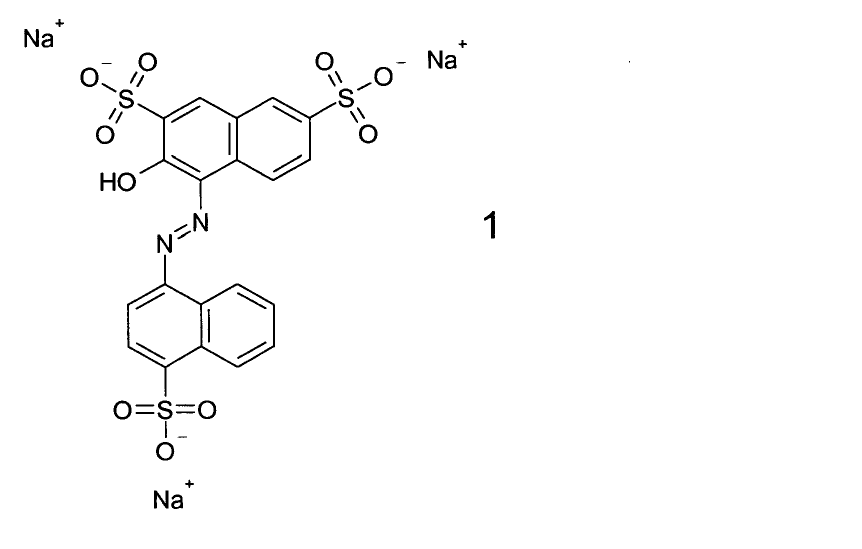

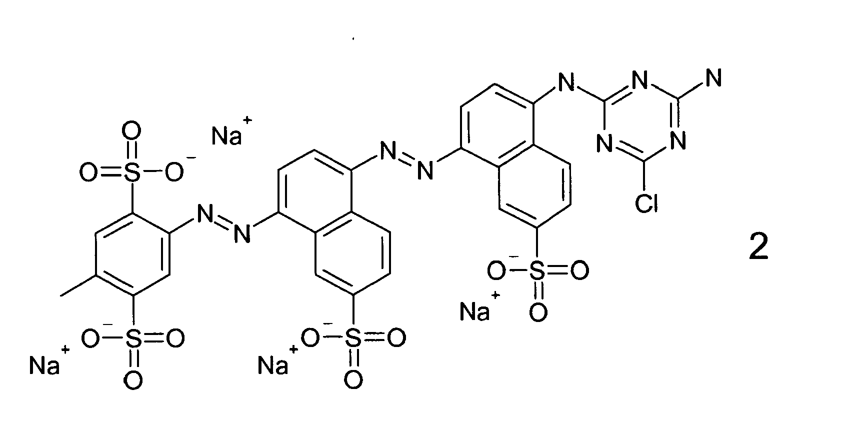

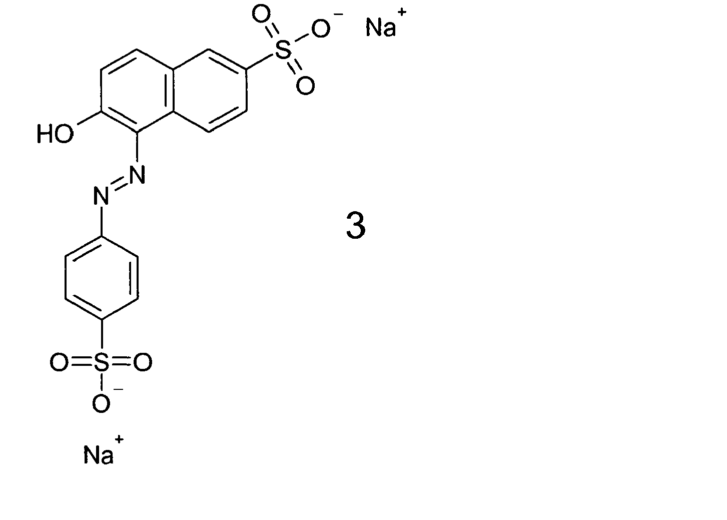

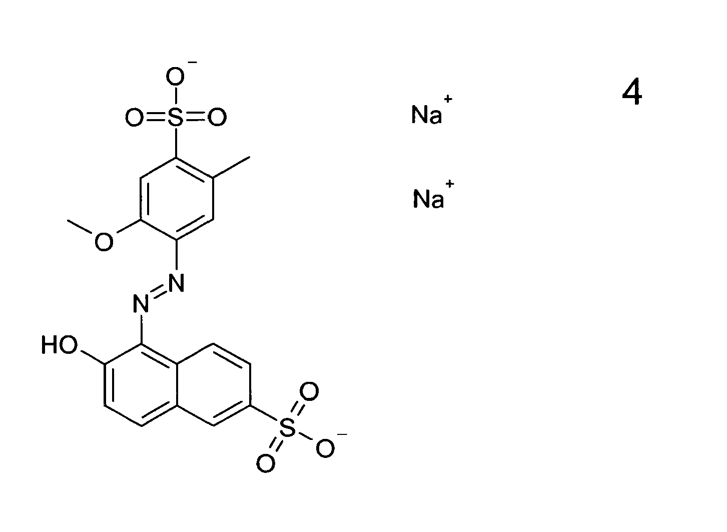

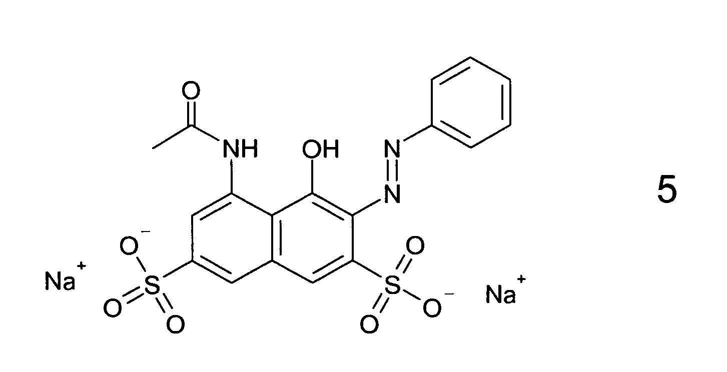

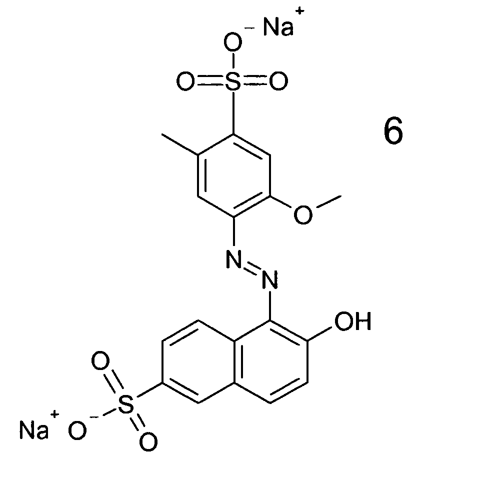

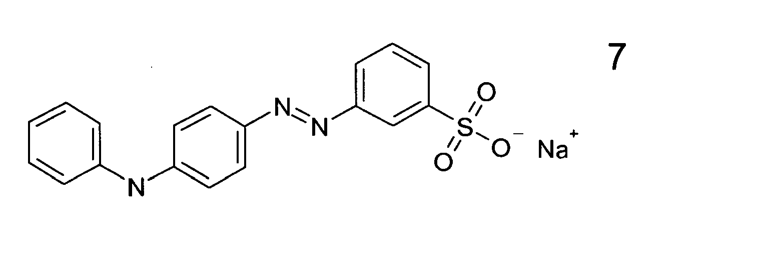

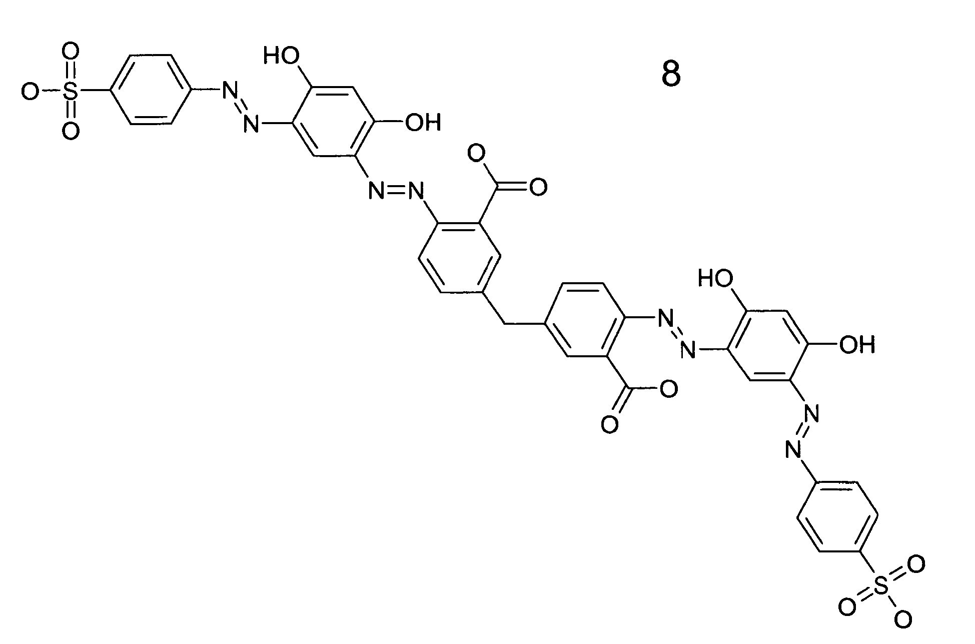

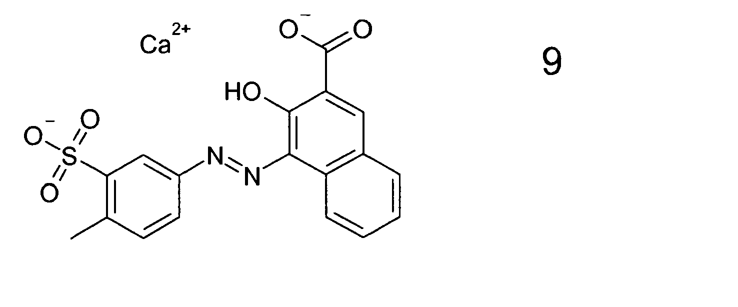

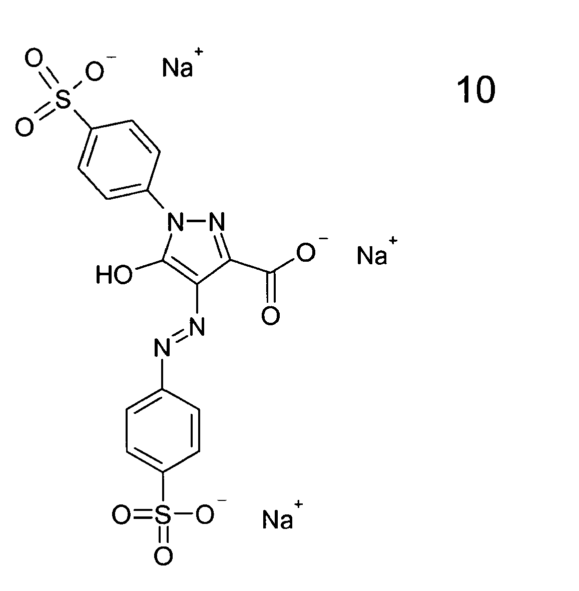

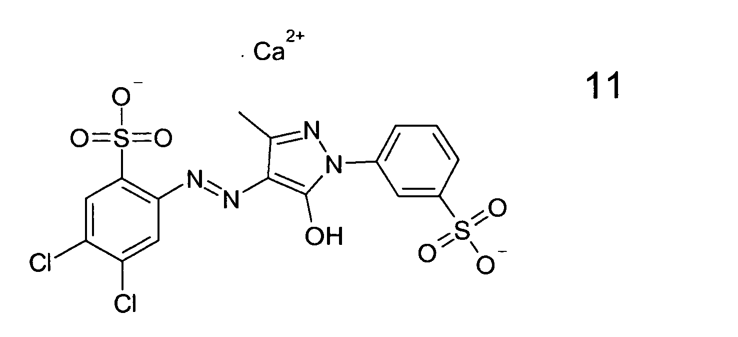

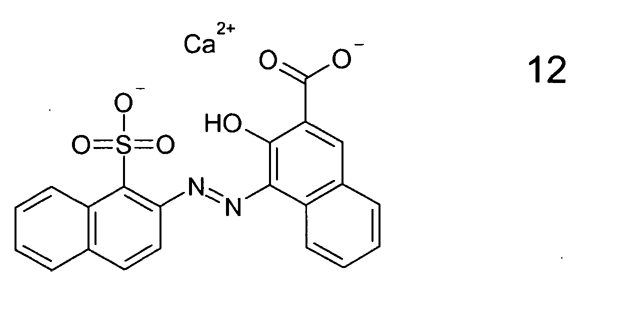

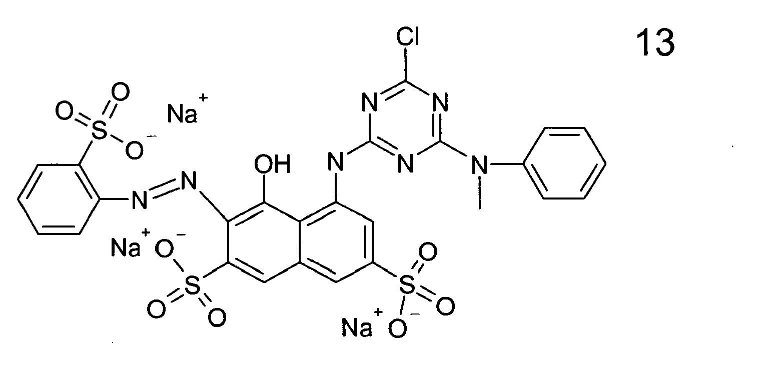

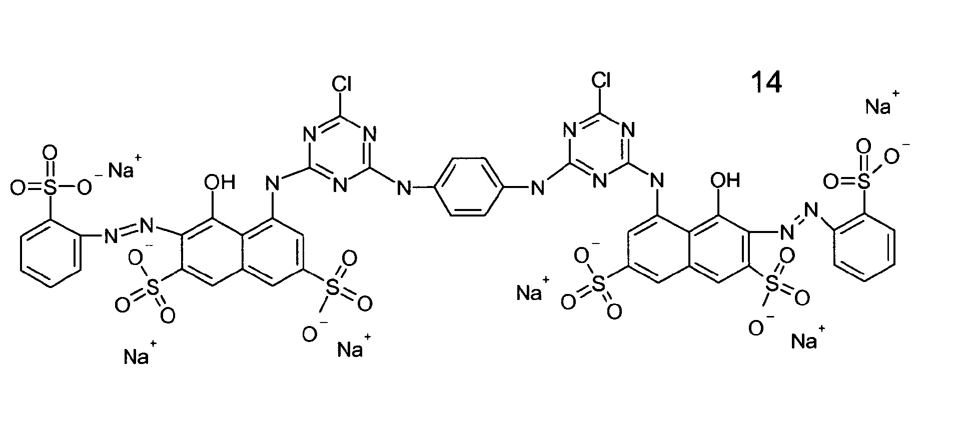

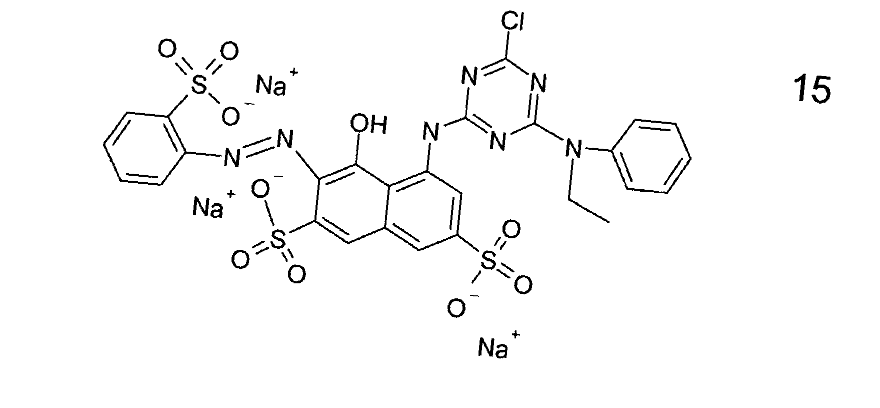

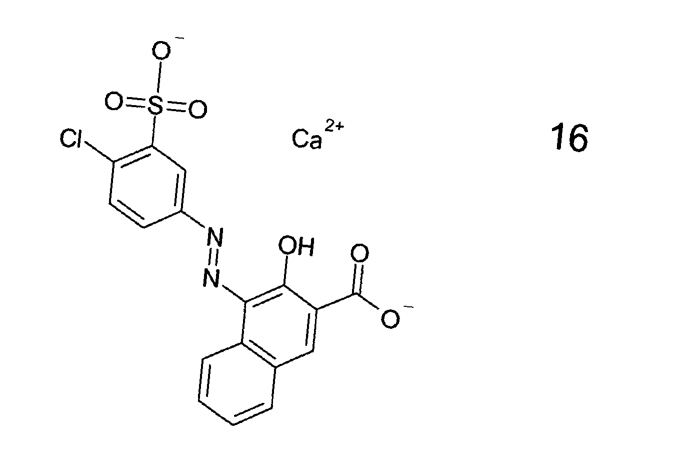

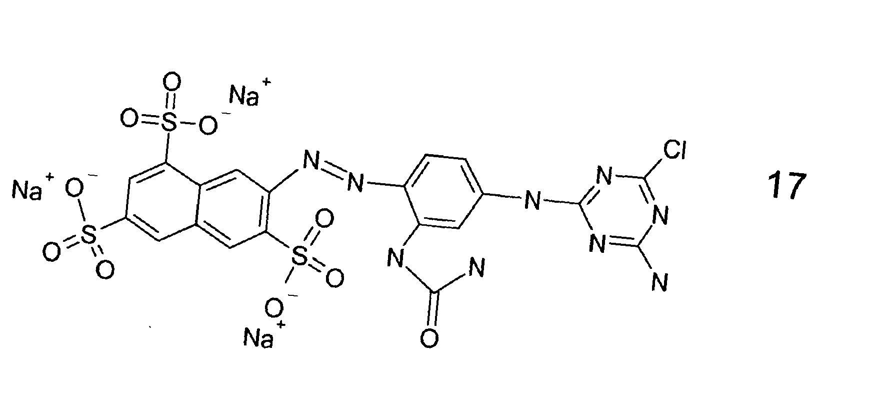

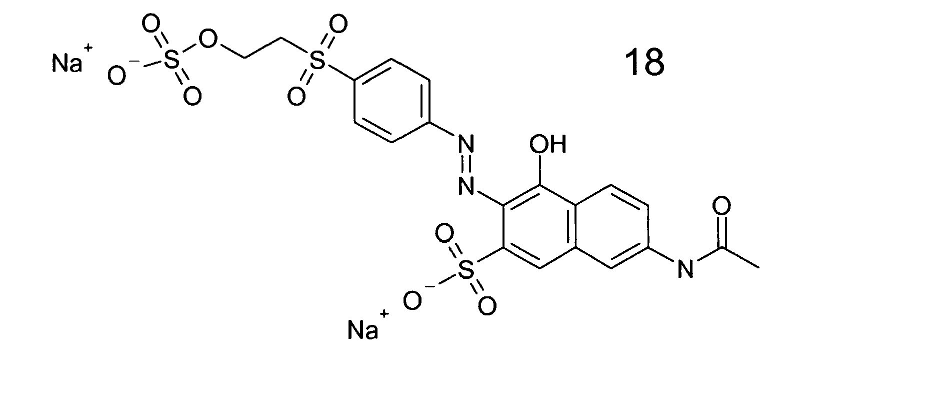

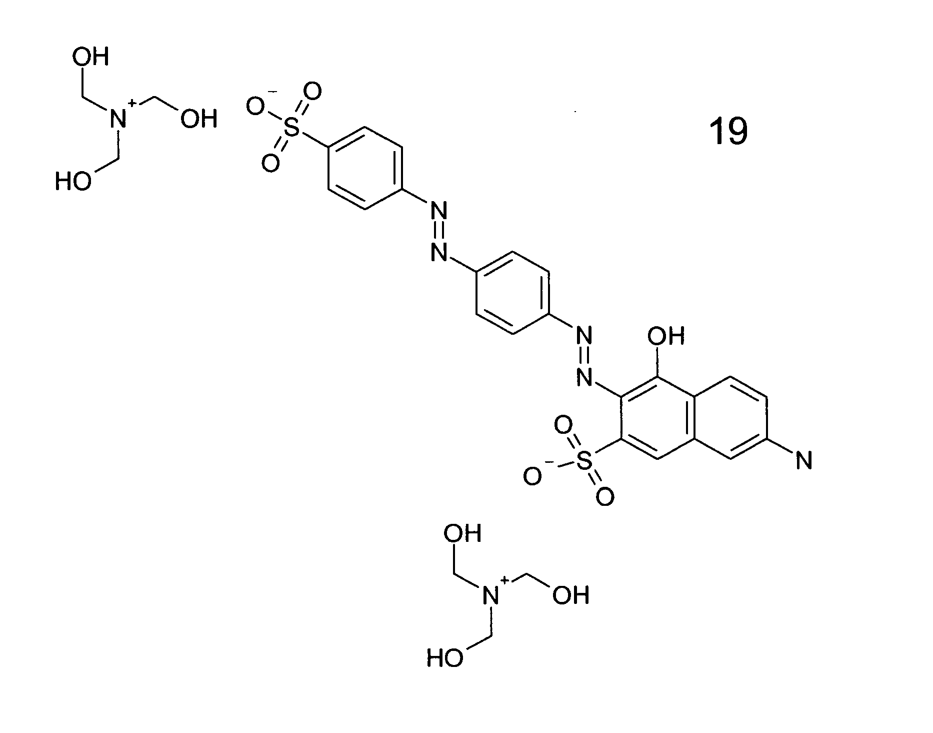

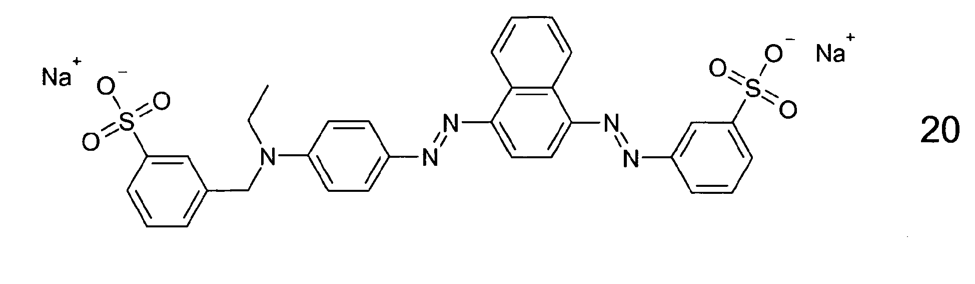

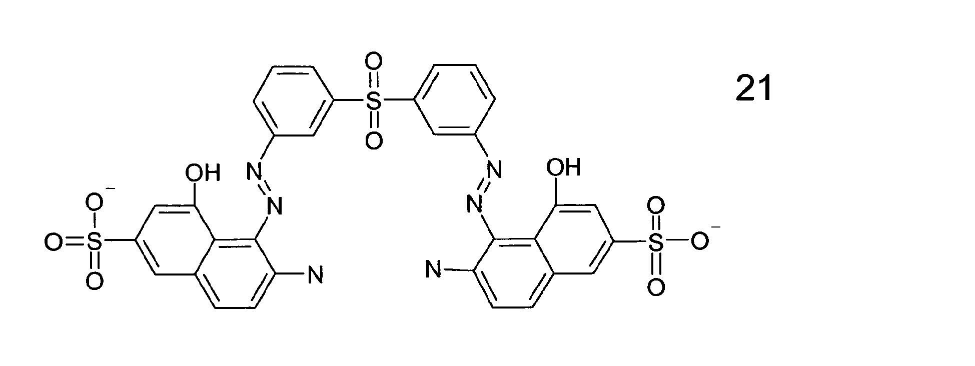

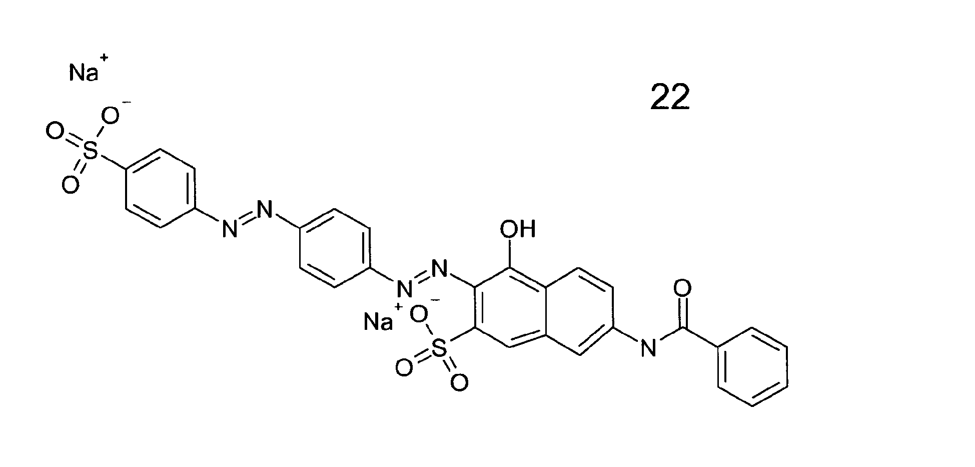

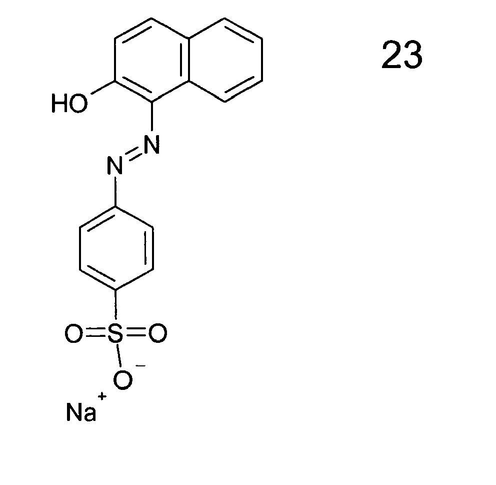

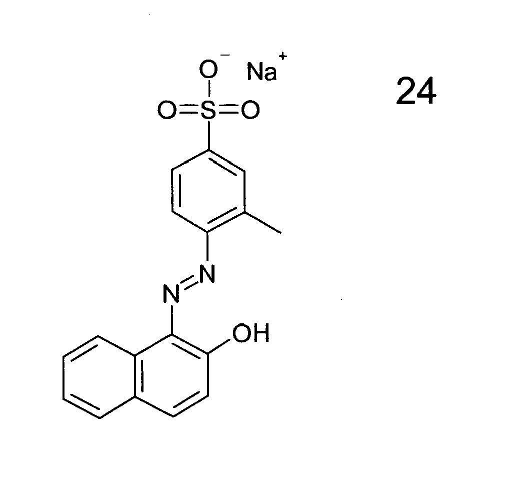

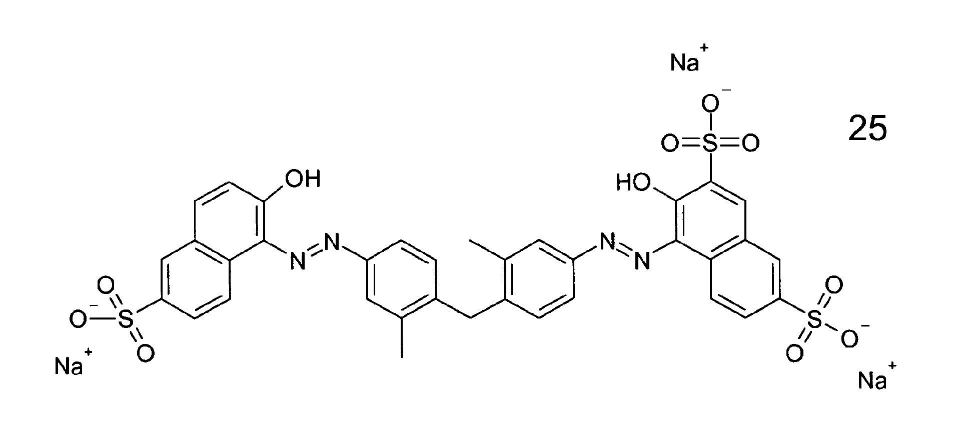

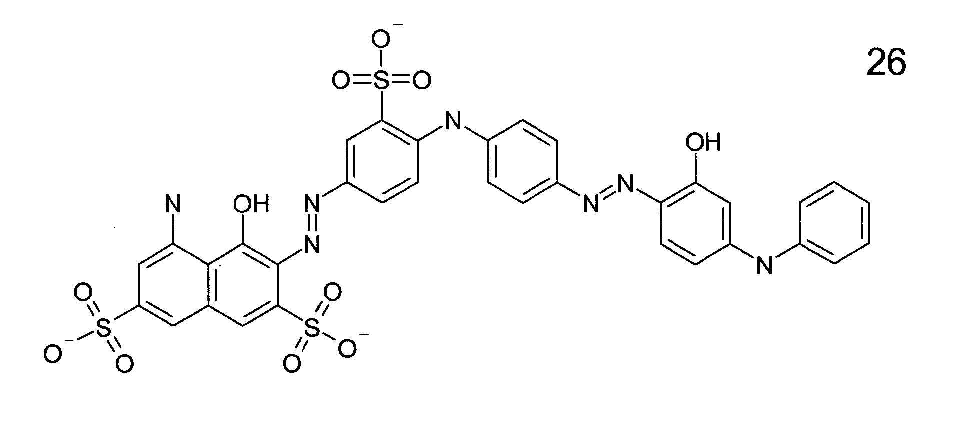

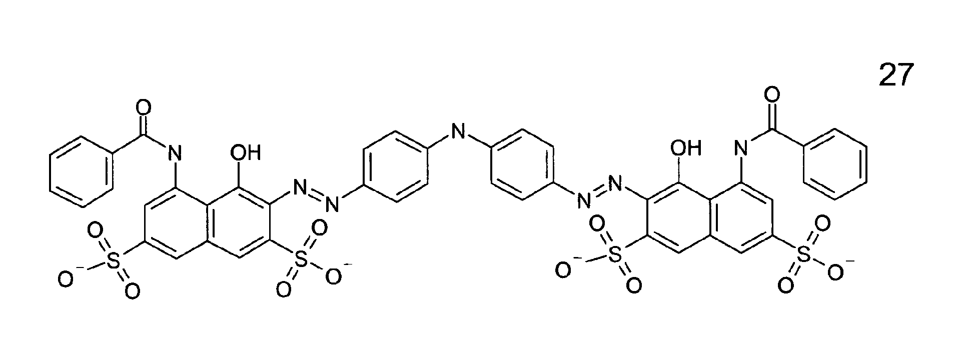

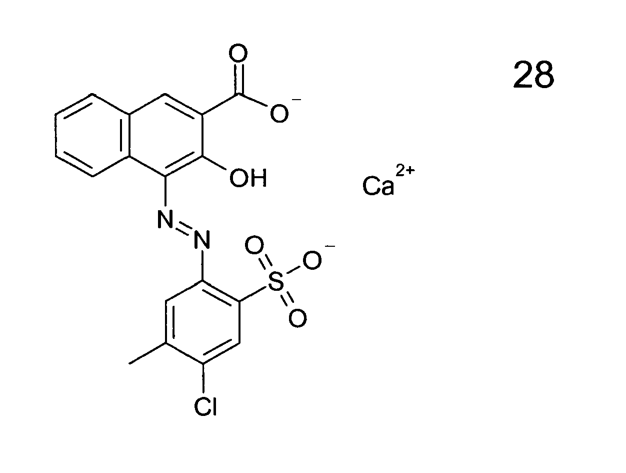

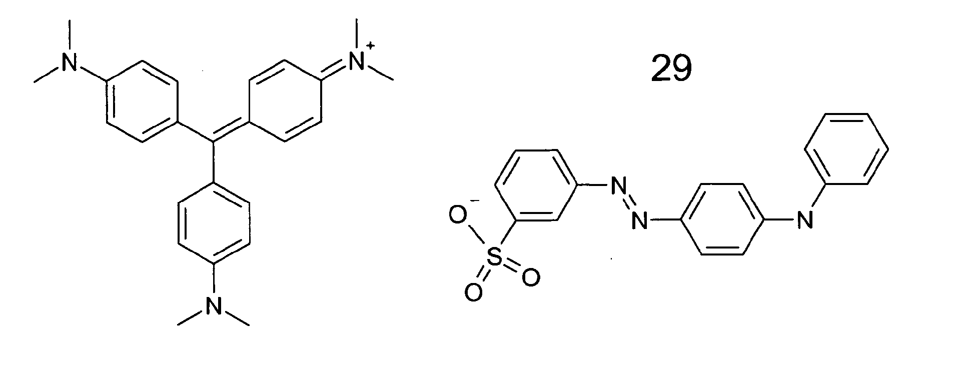

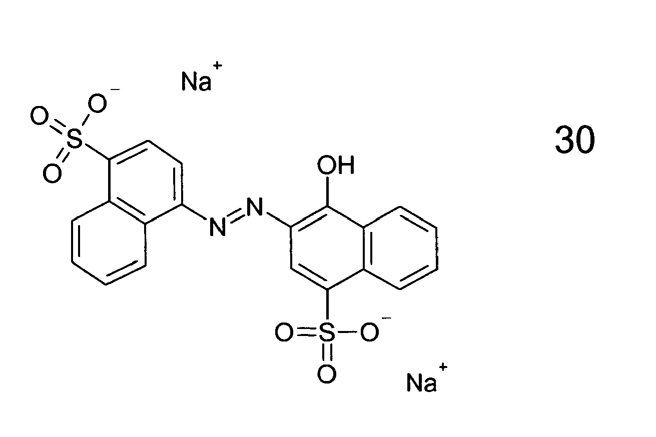

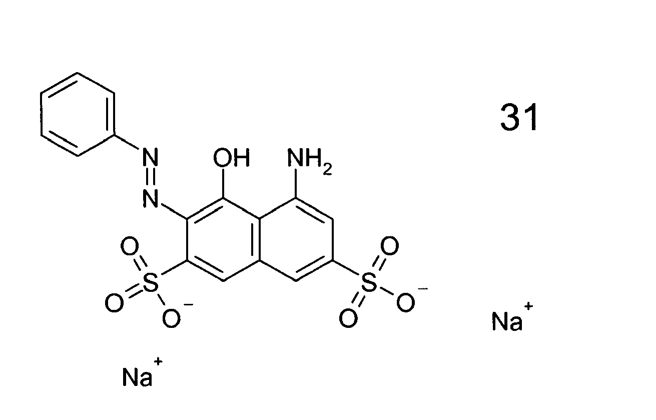

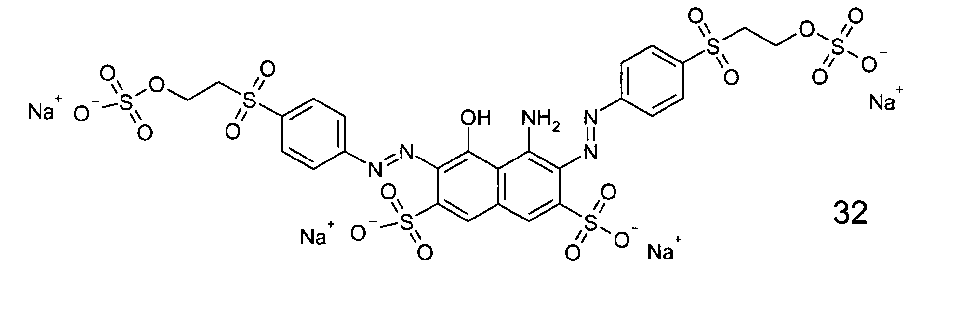

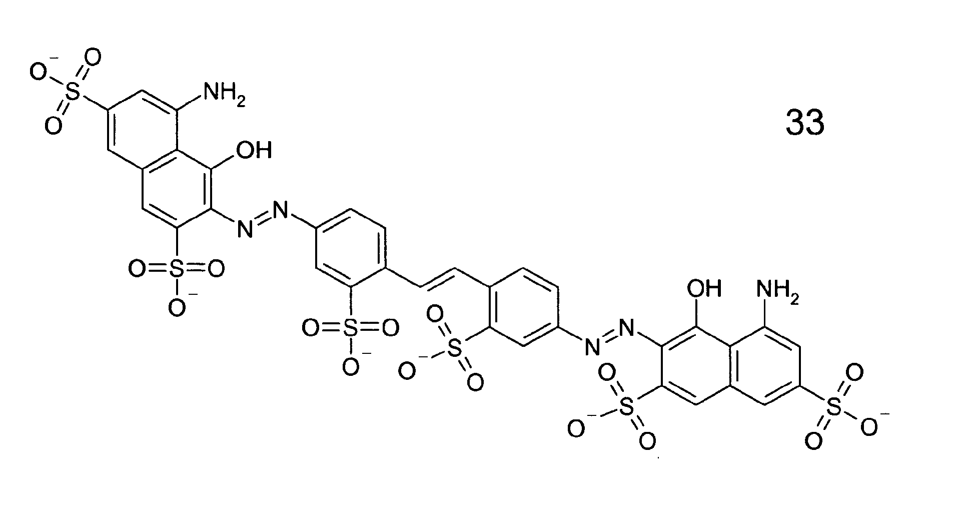

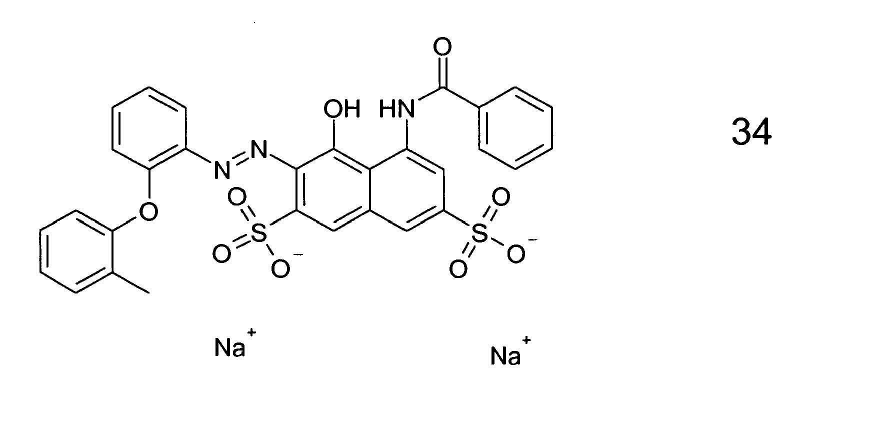

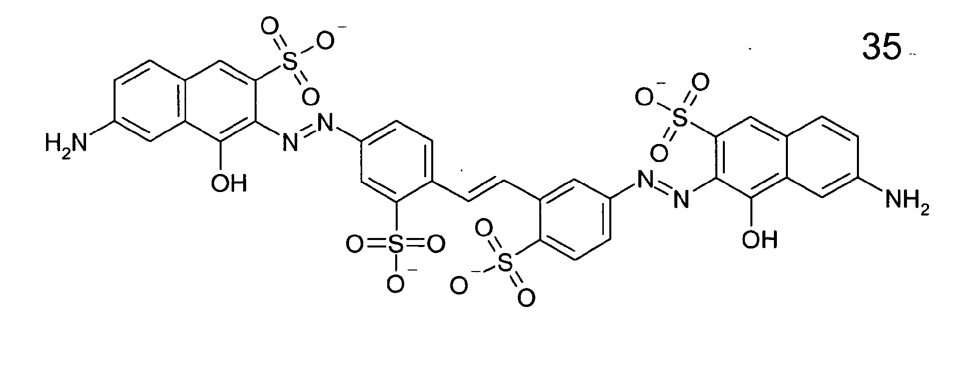

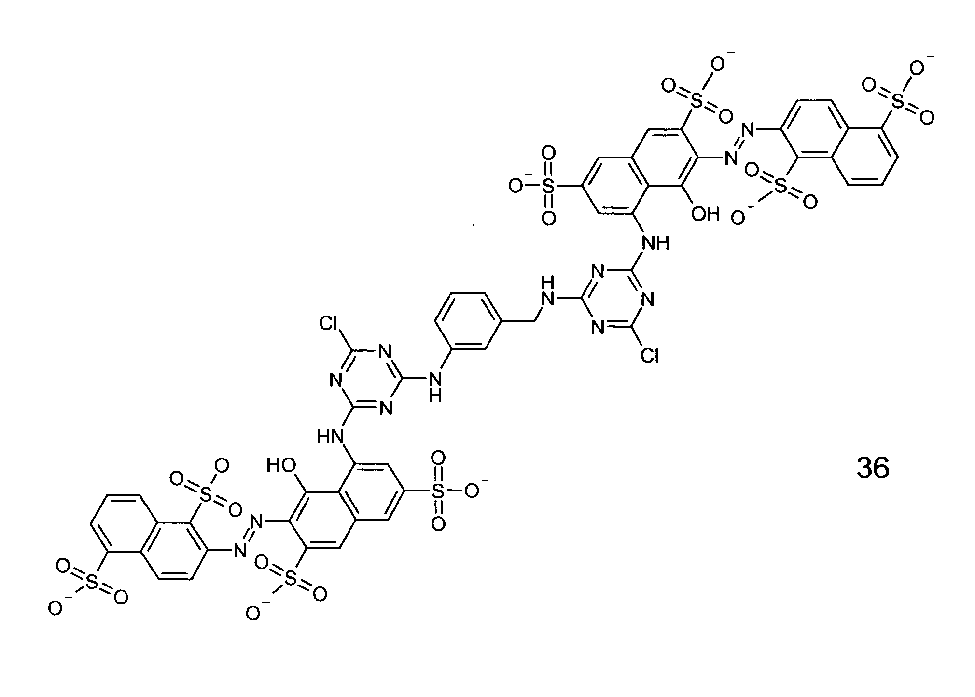

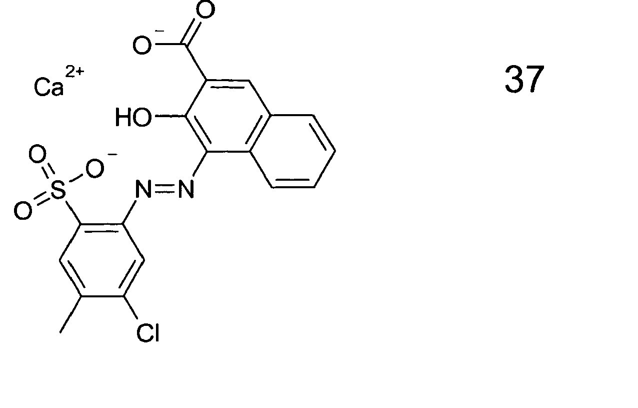

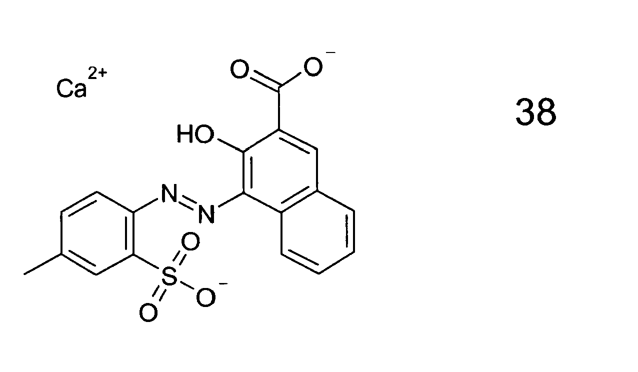

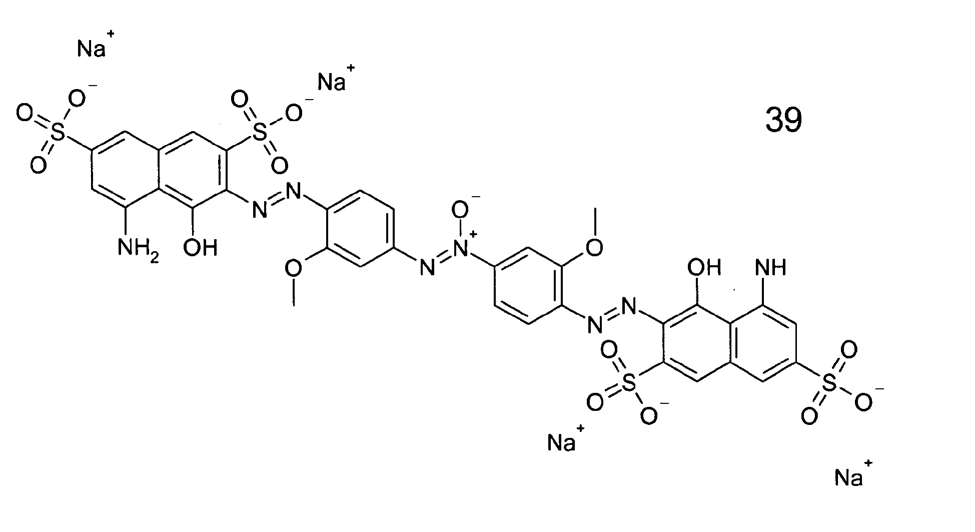

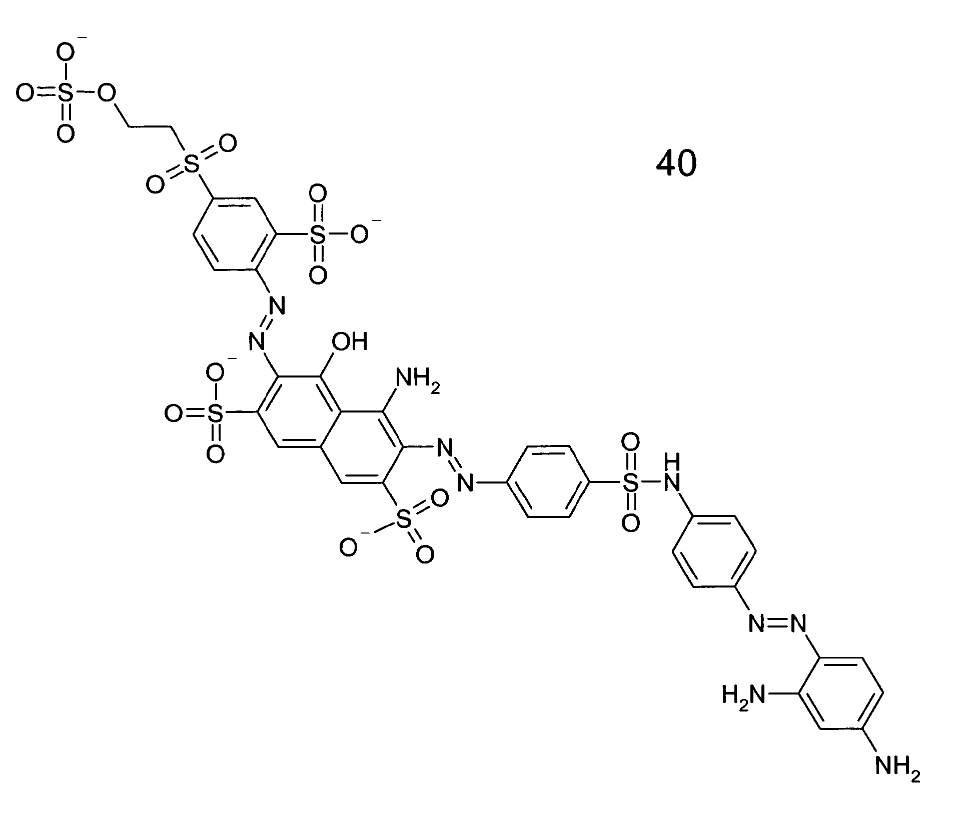

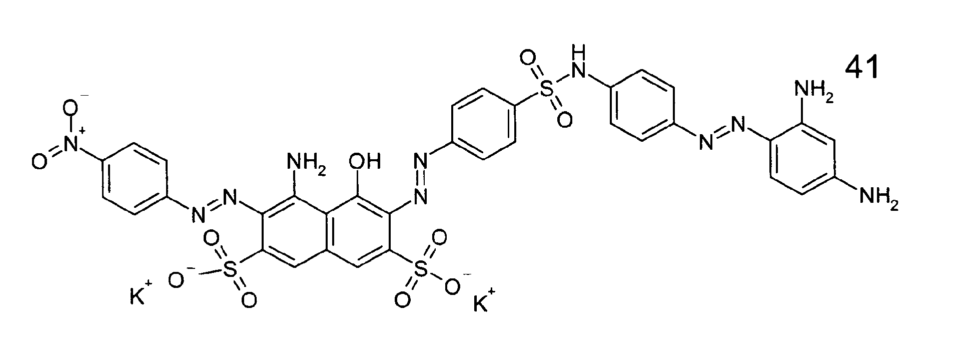

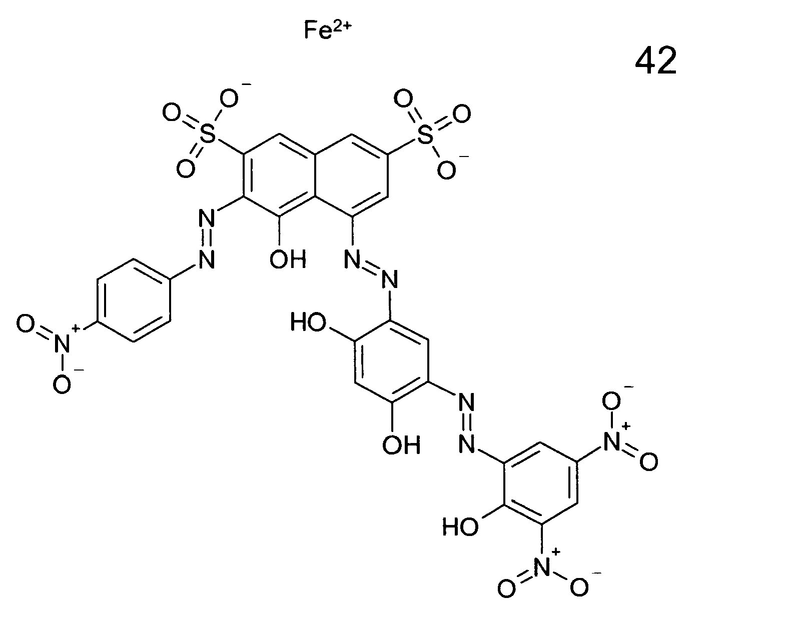

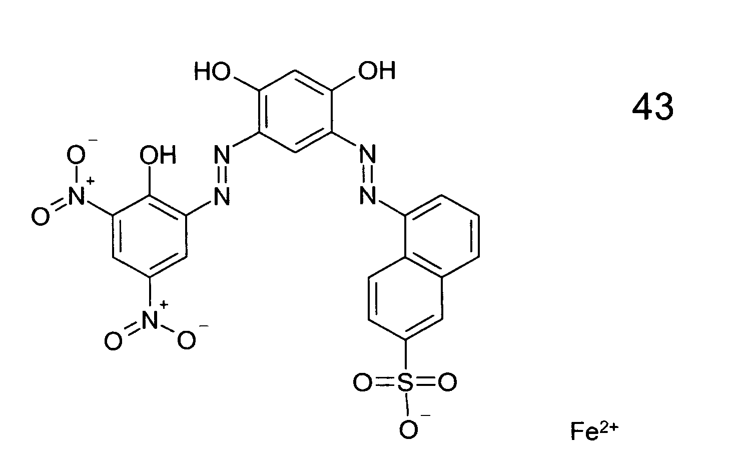

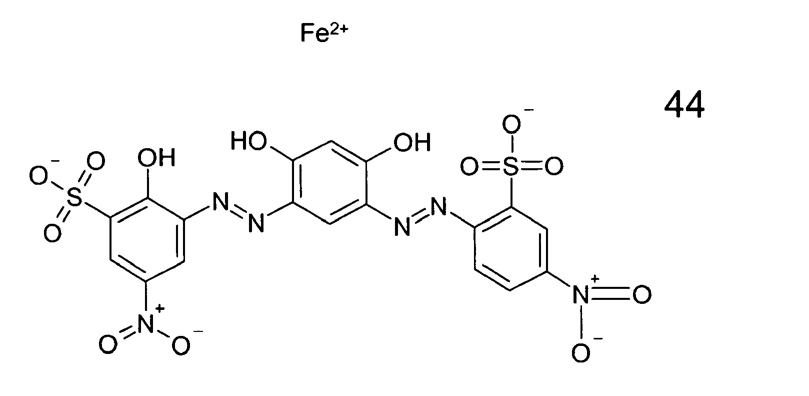

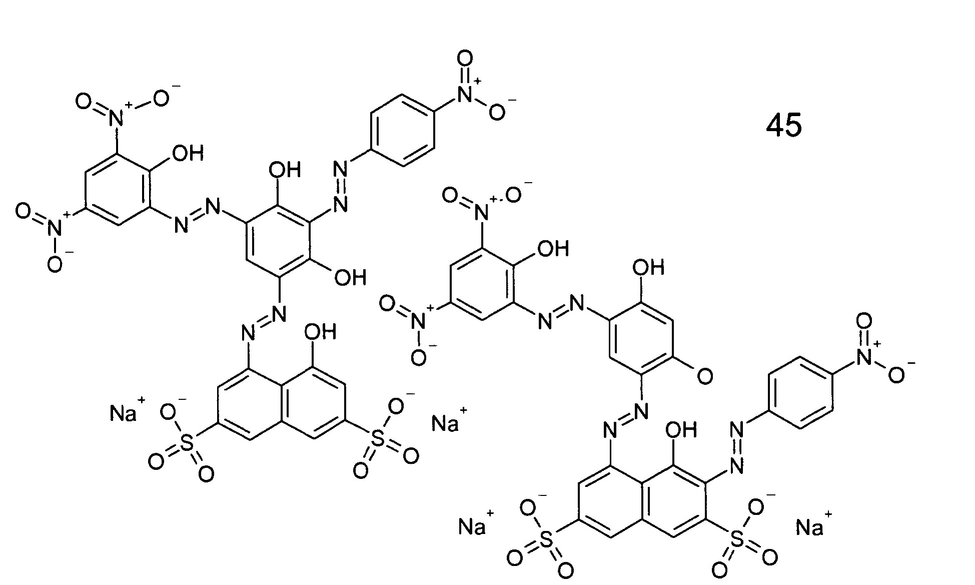

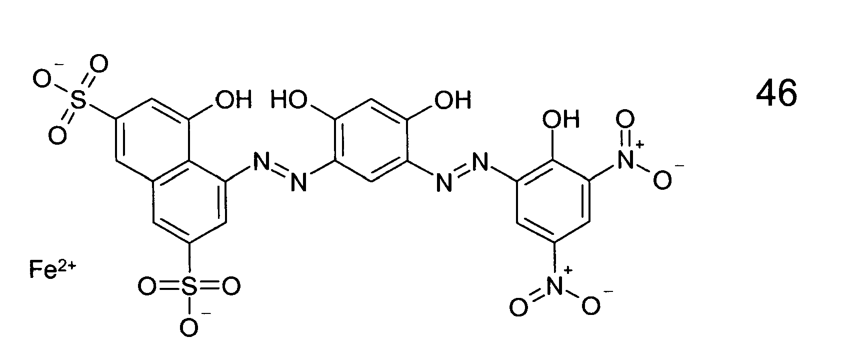

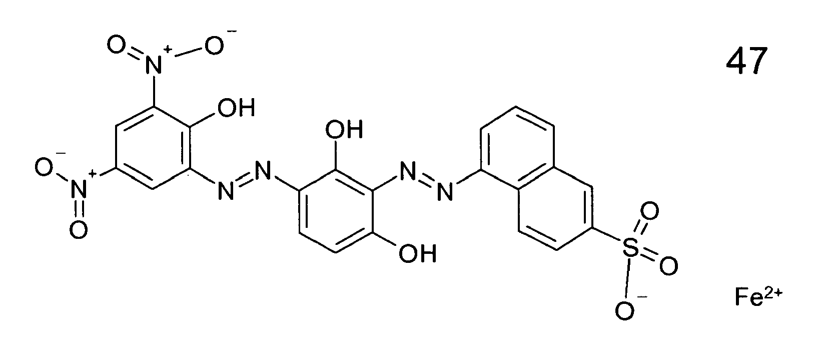

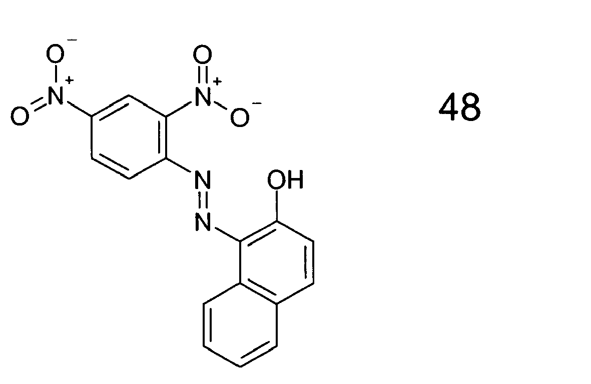

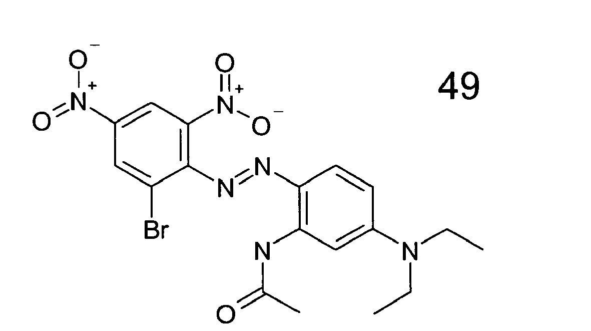

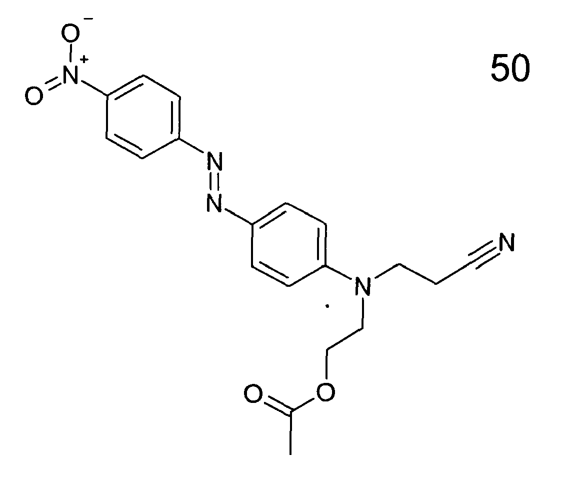

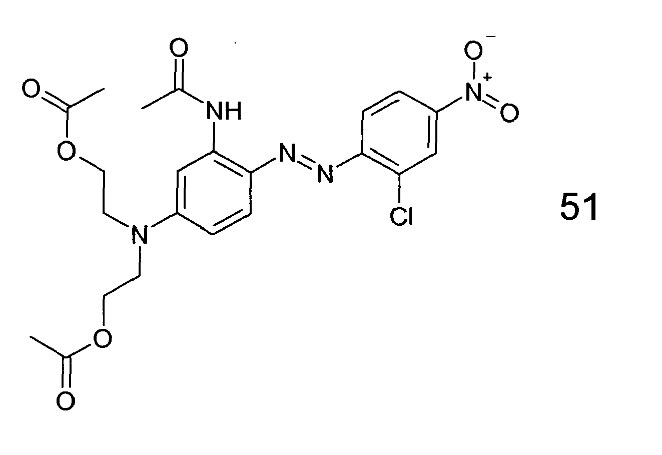

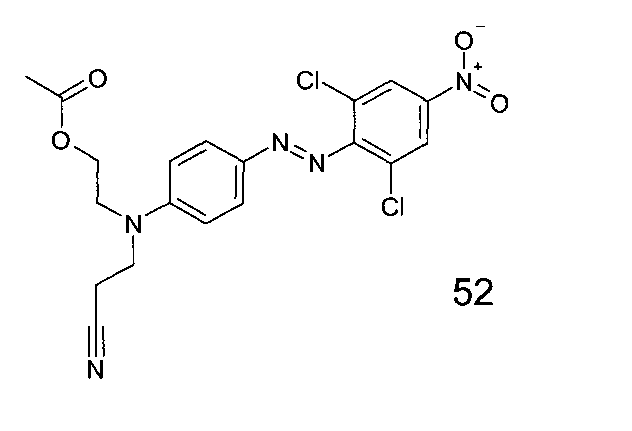

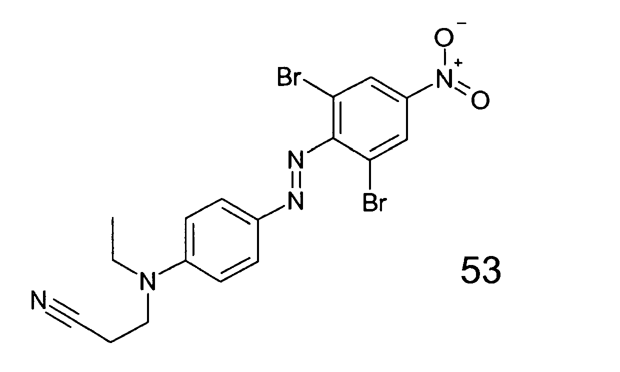

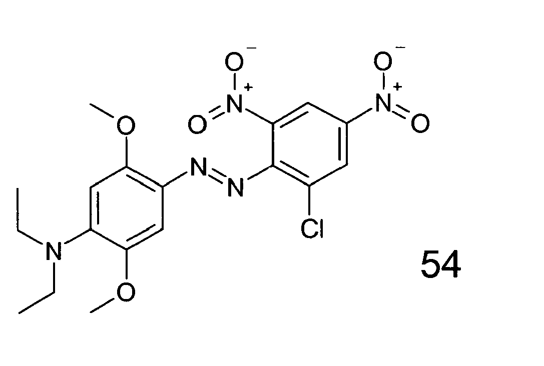

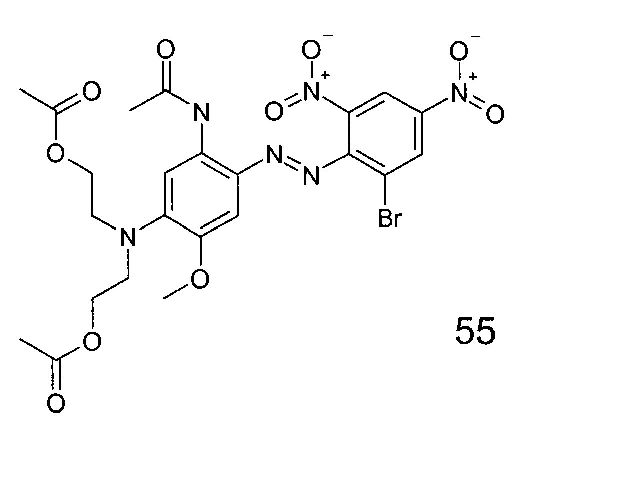

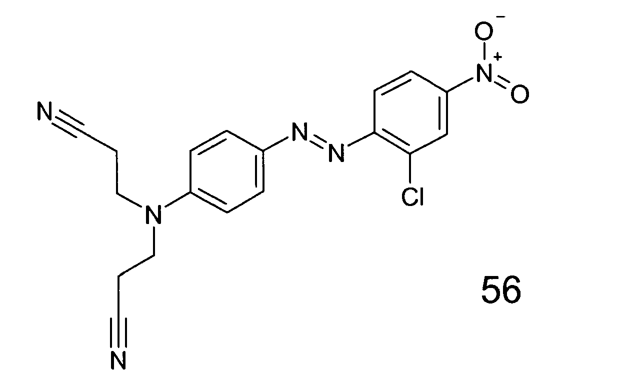

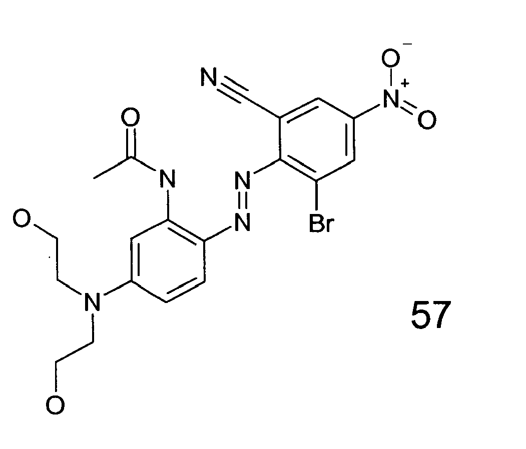

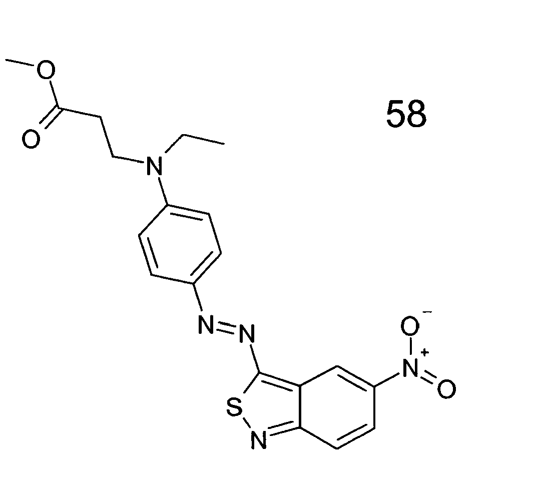

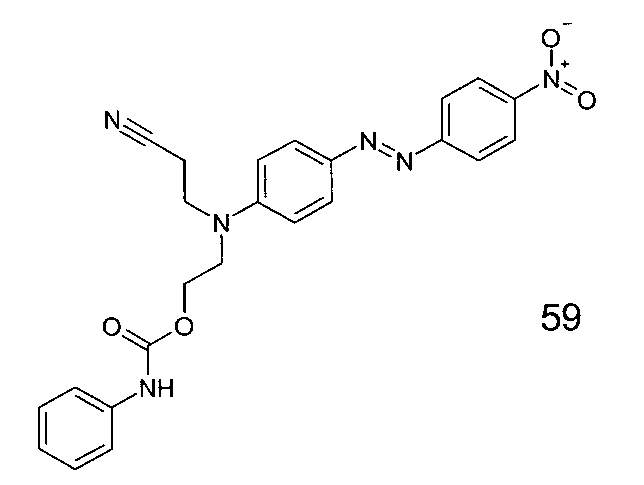

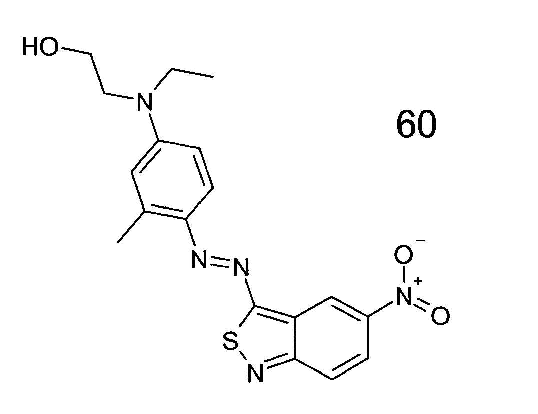

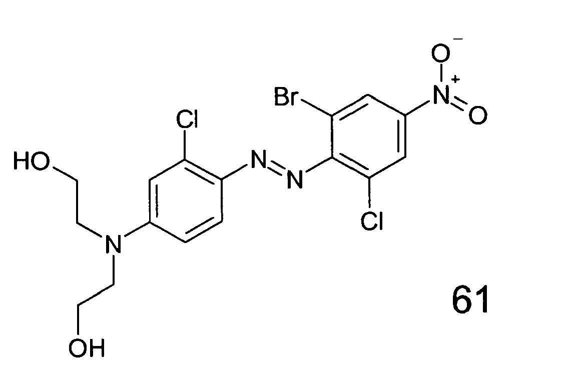

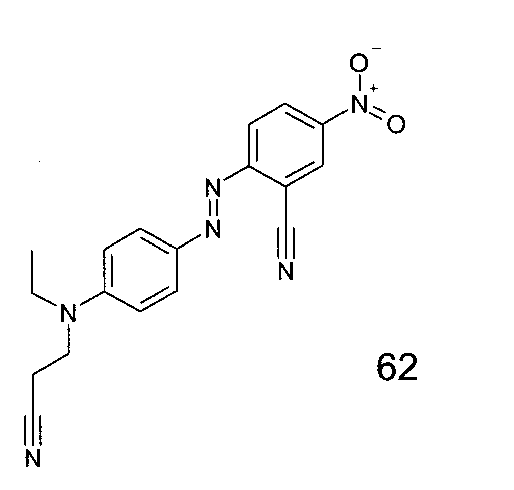

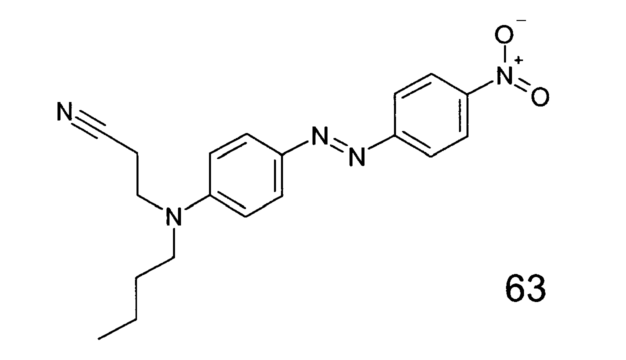

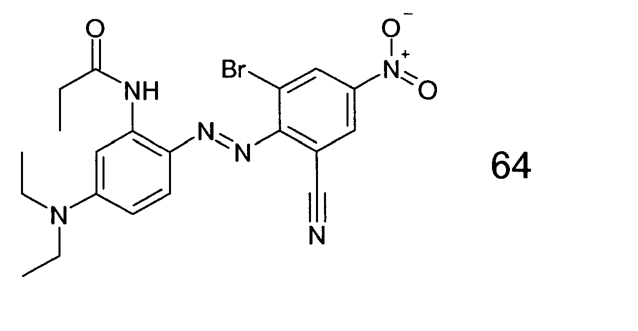

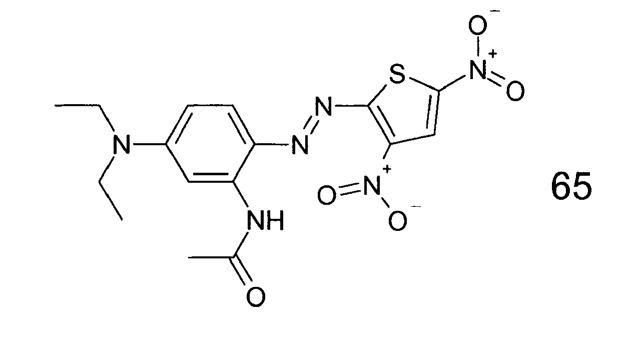

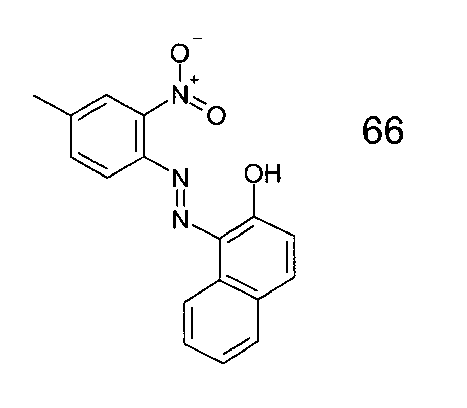

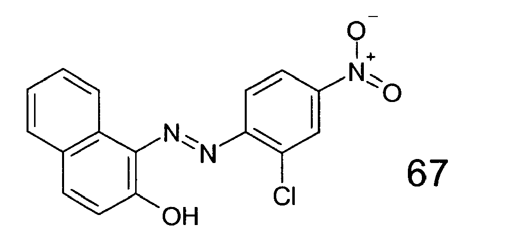

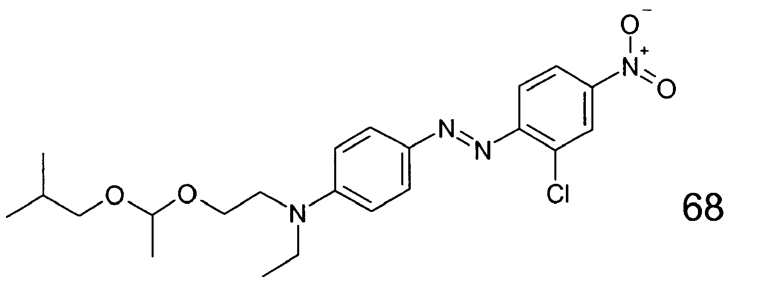

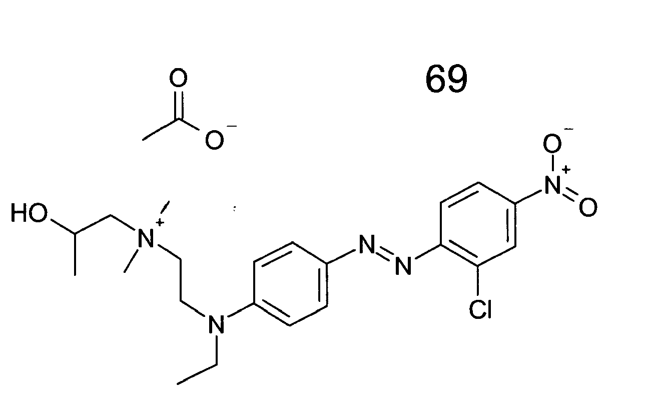

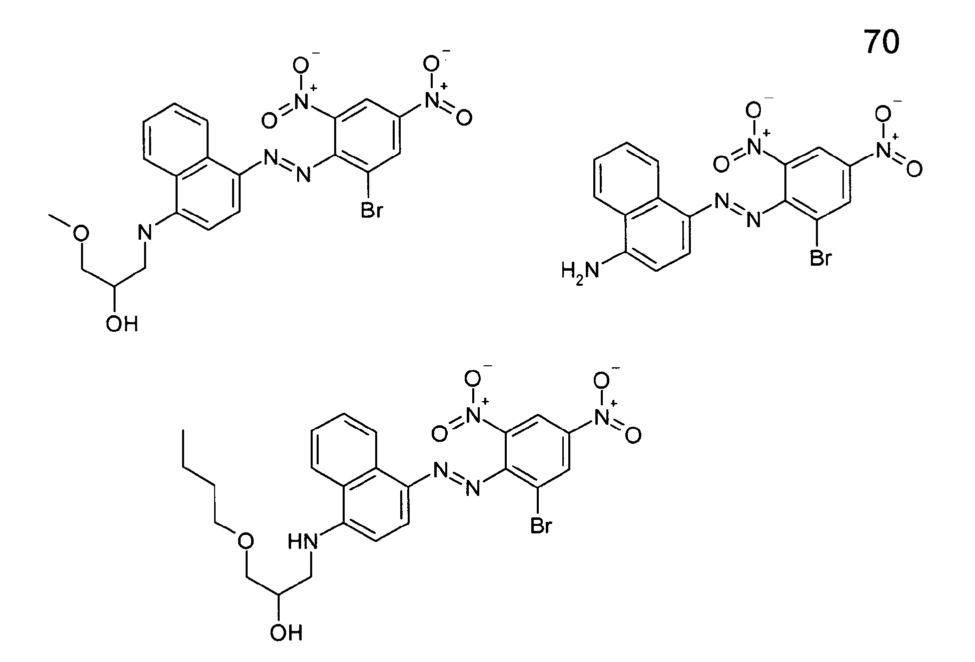

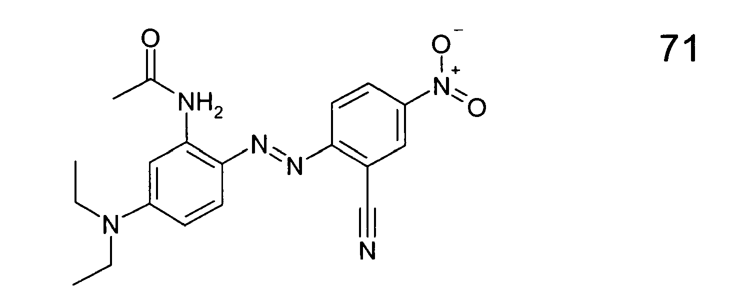

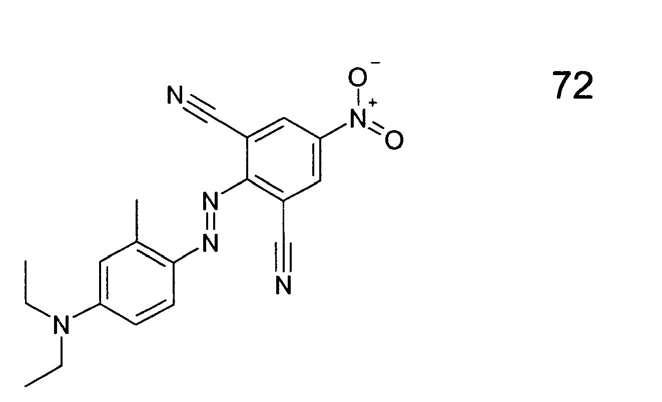

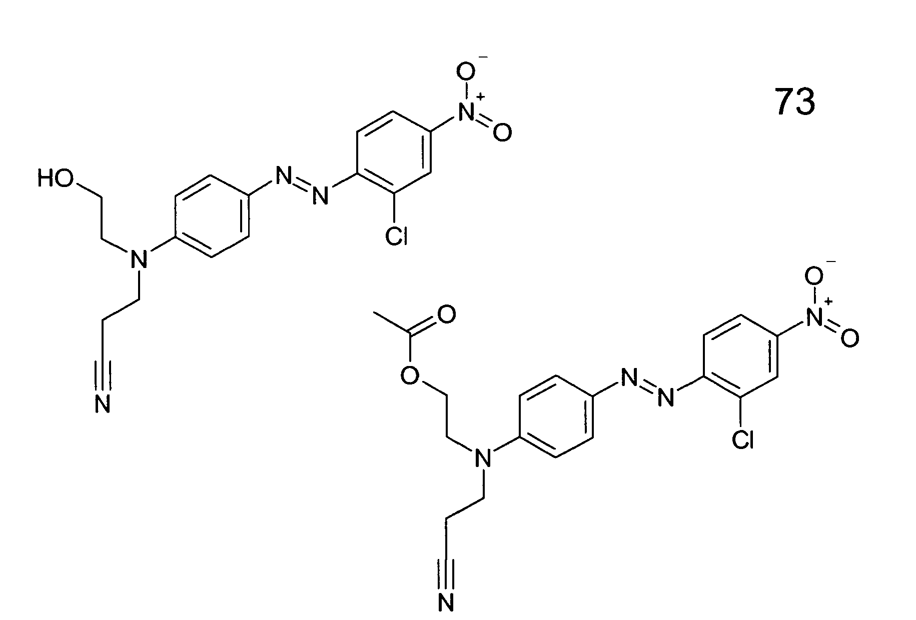

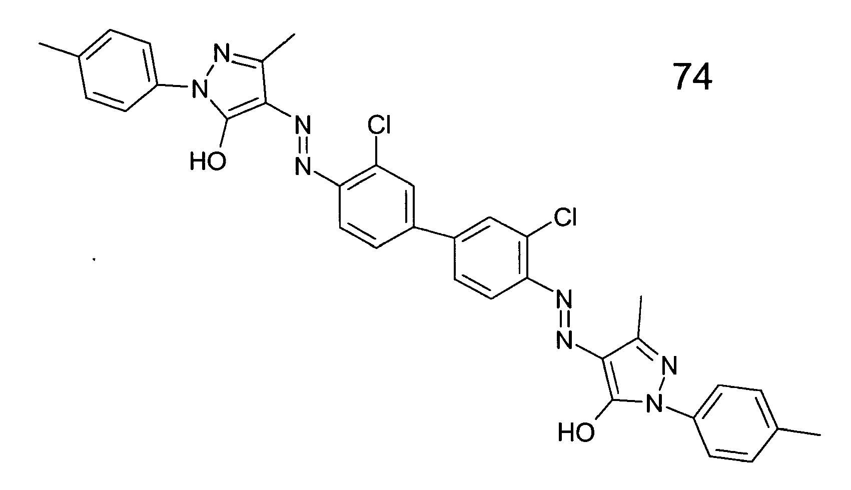

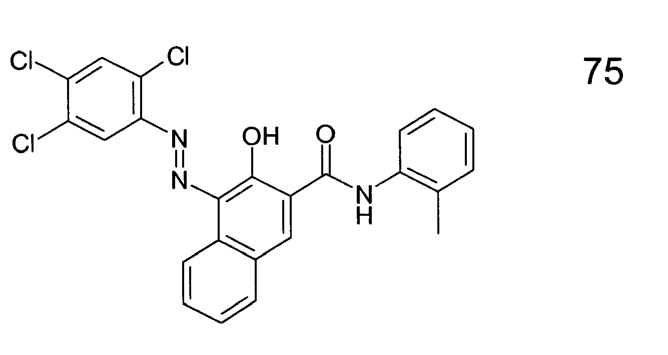

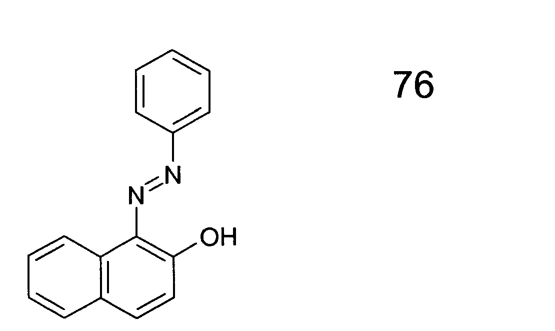

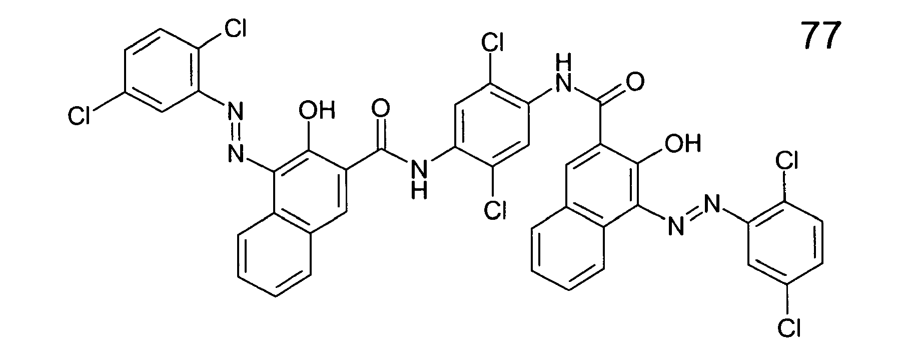

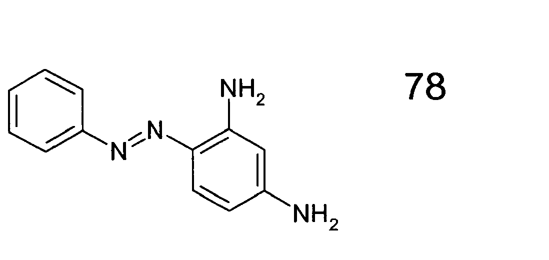

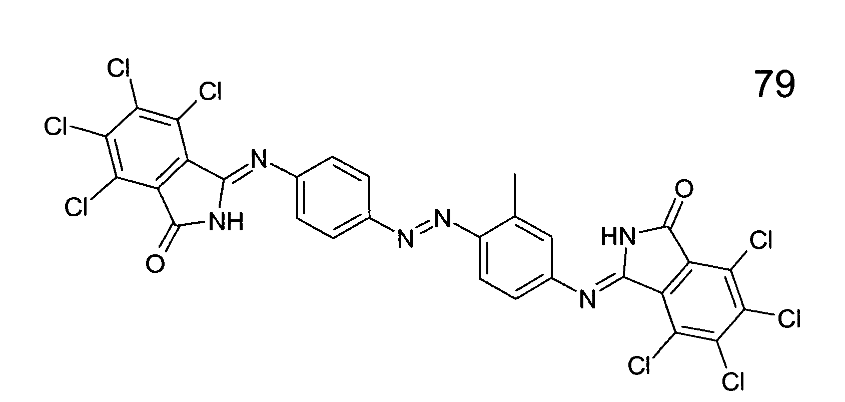

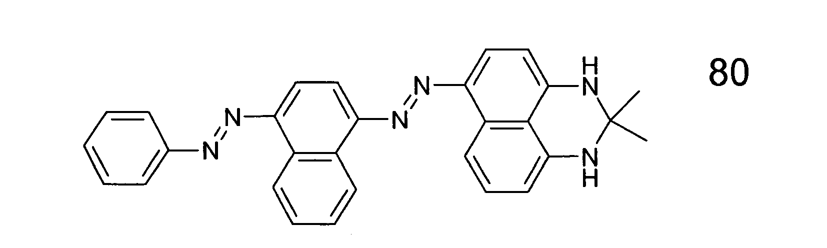

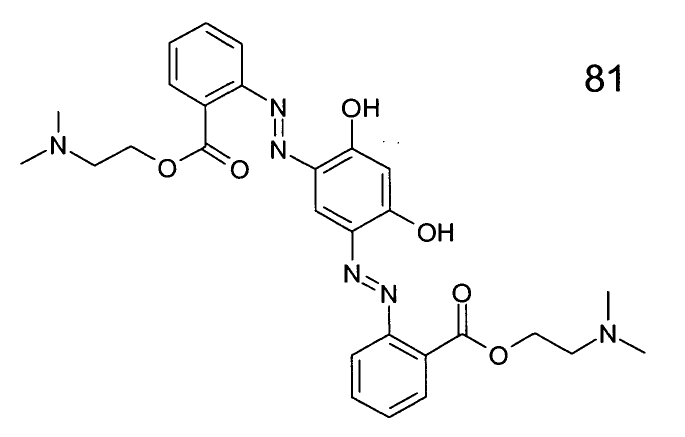

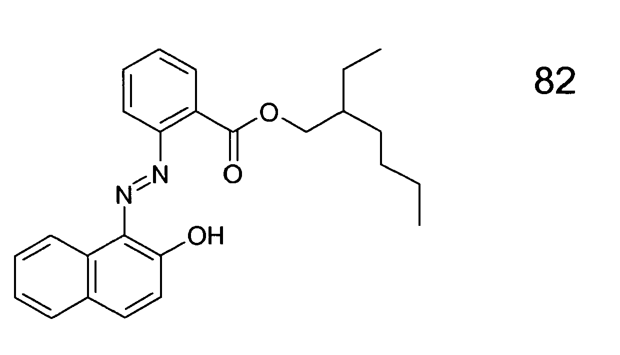

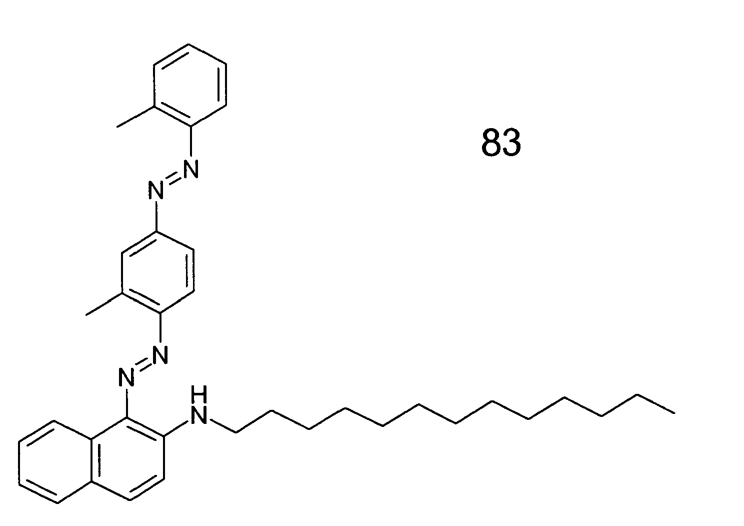

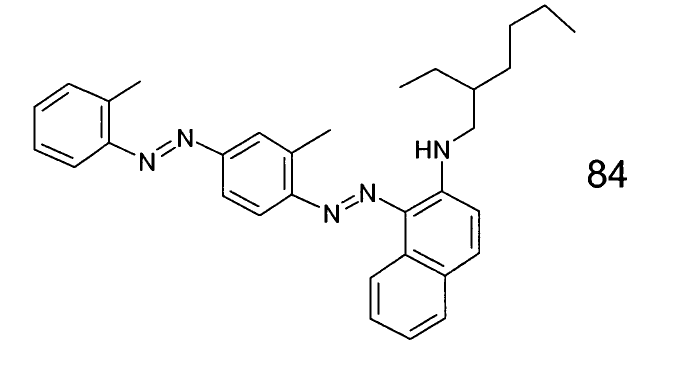

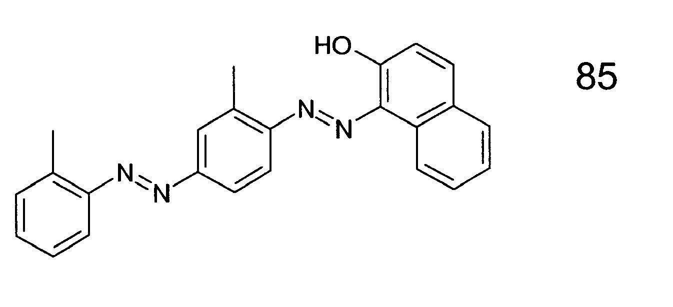

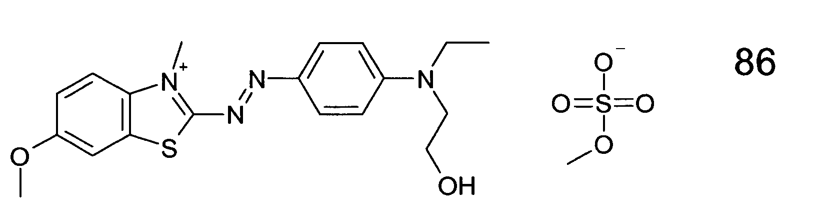

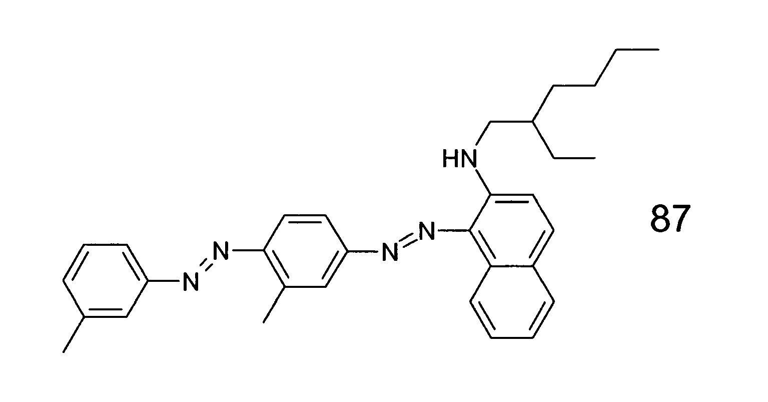

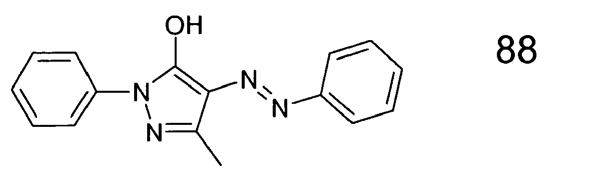

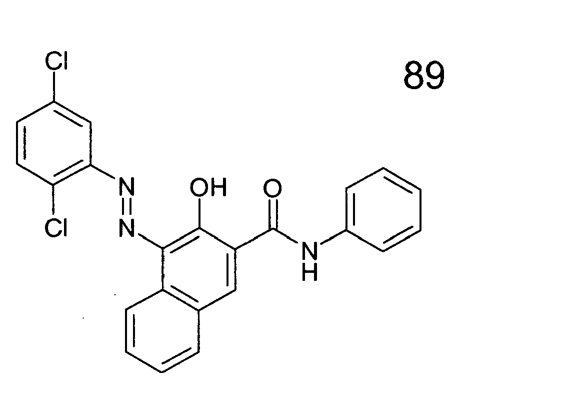

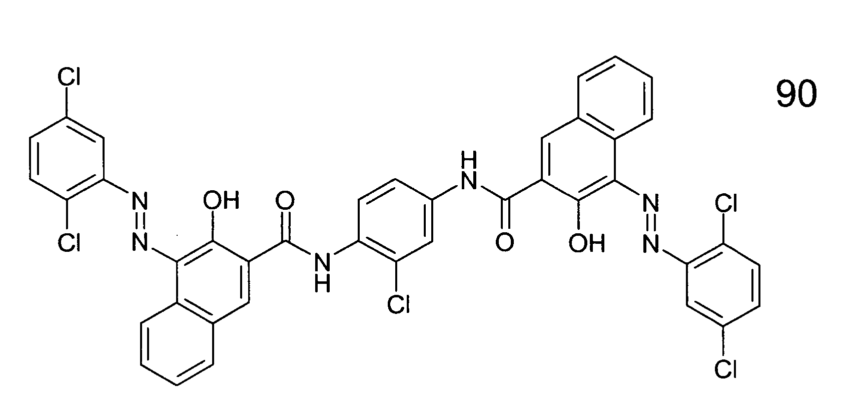

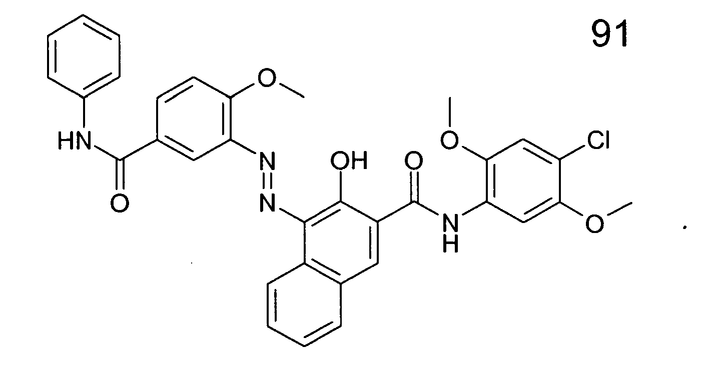

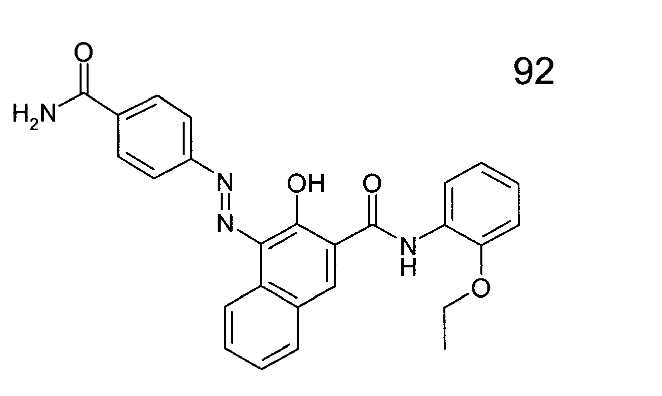

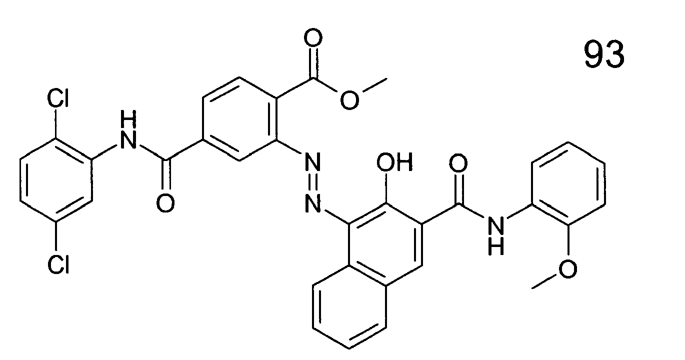

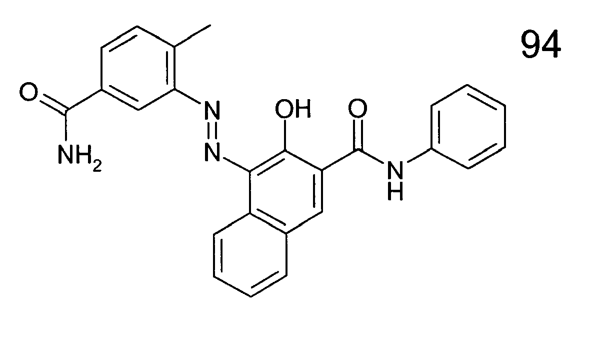

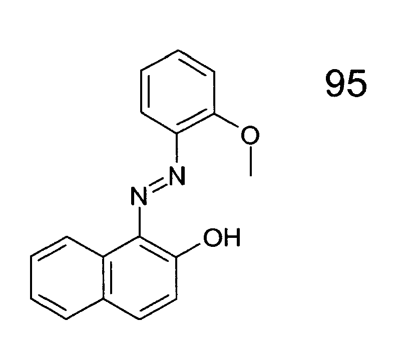

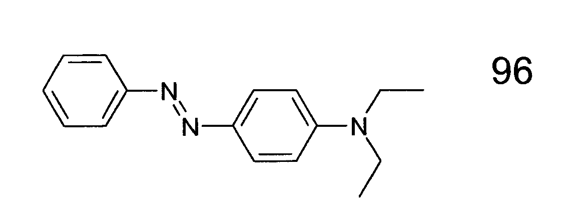

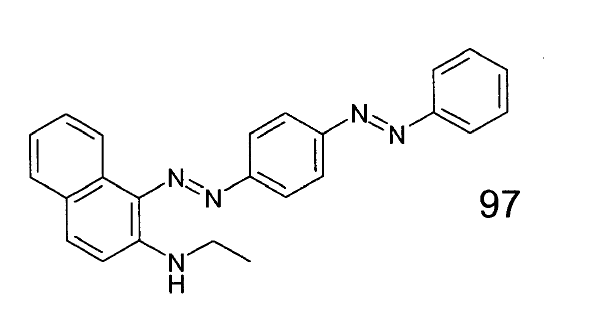

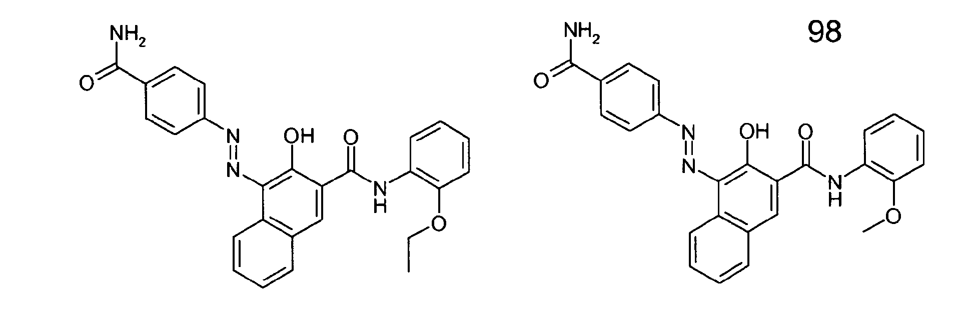

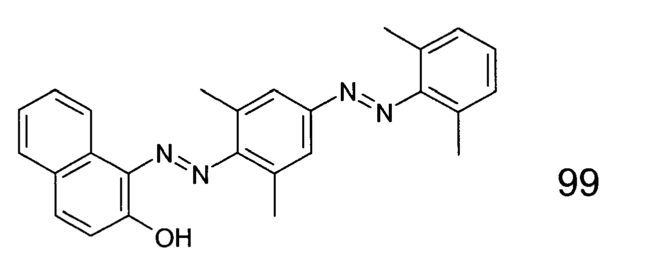

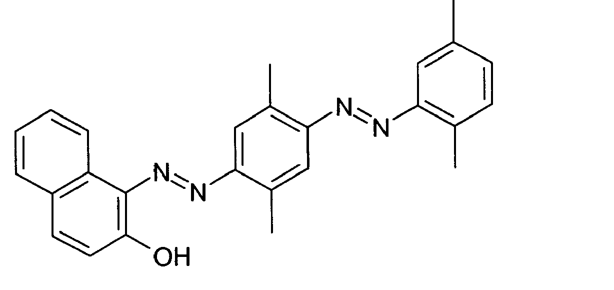

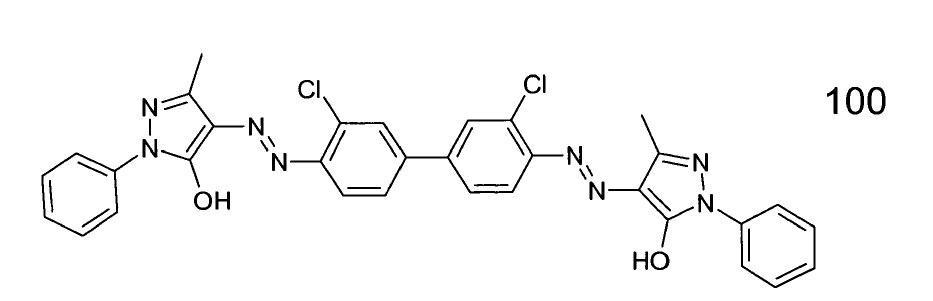

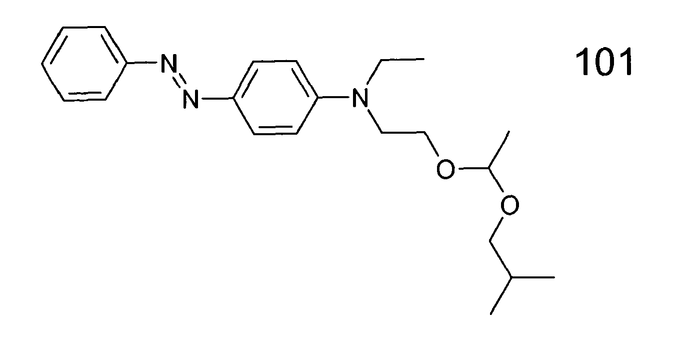

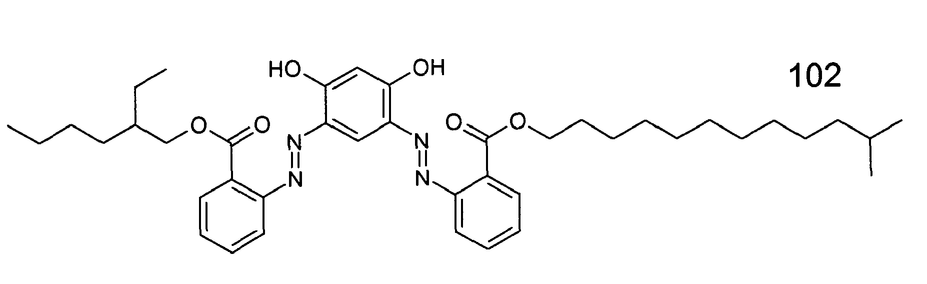

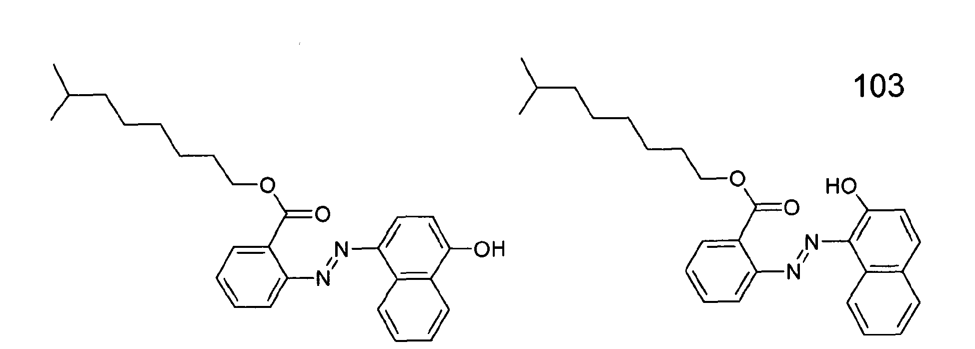

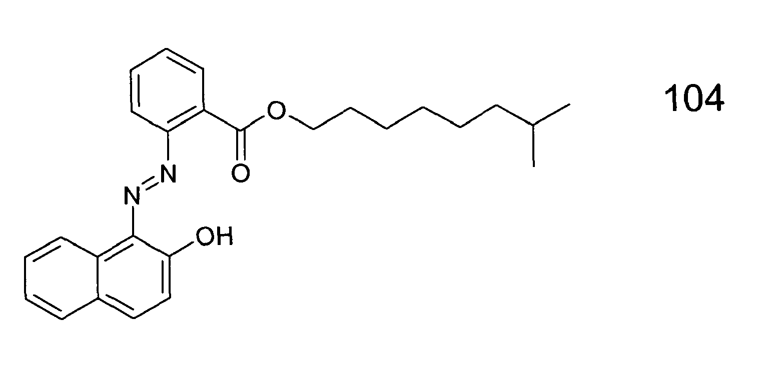

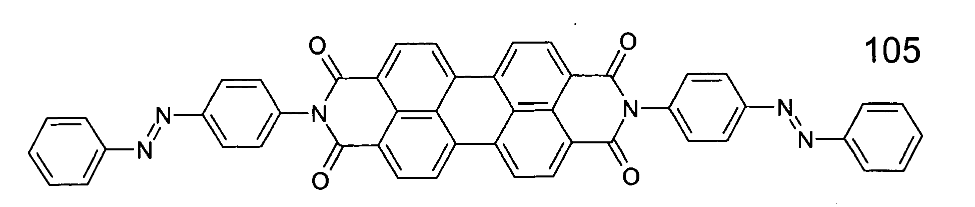

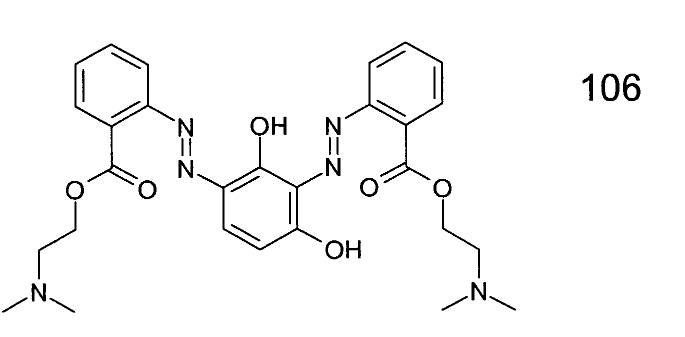

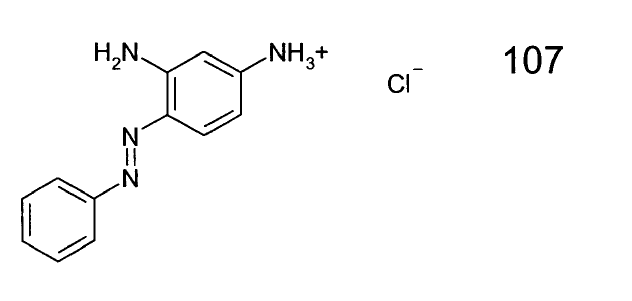

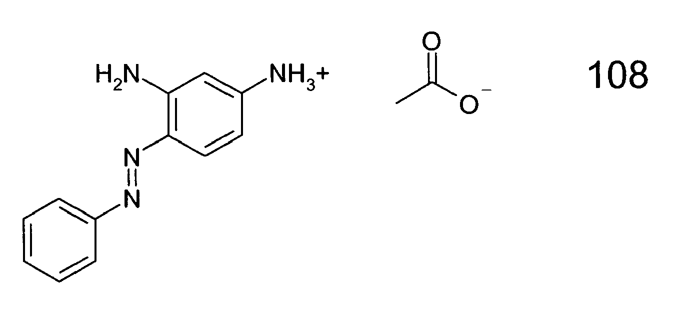

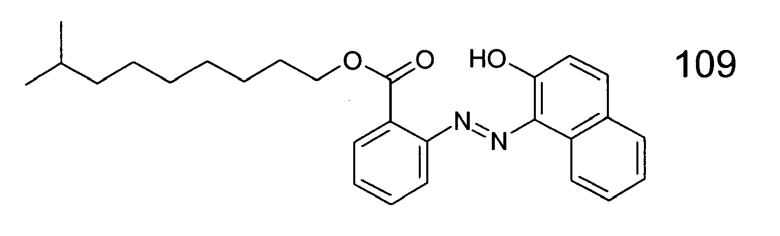

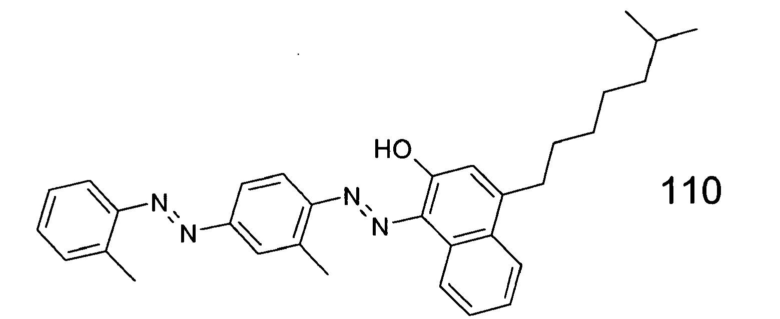

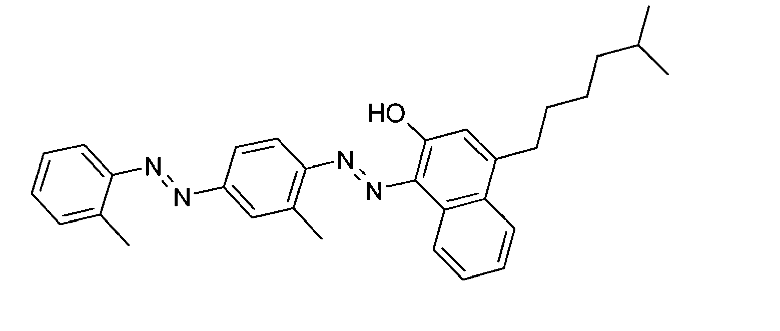

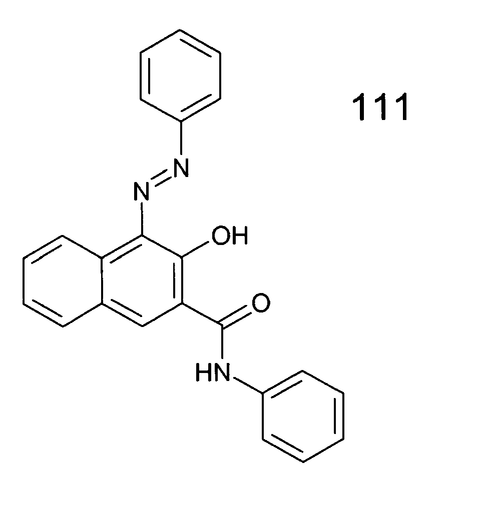

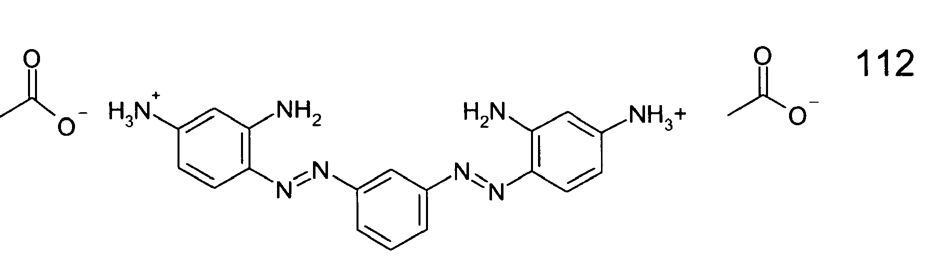

Bevorzugt werden die Azoverbindung aus der Gruppe, bestehend aus den Azoverbindungen 1, 3, 5, 6, 30, 78, 59 und 112, und insbesondere 1 (= E123), 3 (= E110), 5 (= E128) und 6 (= E129):

E 123, Amaranth:

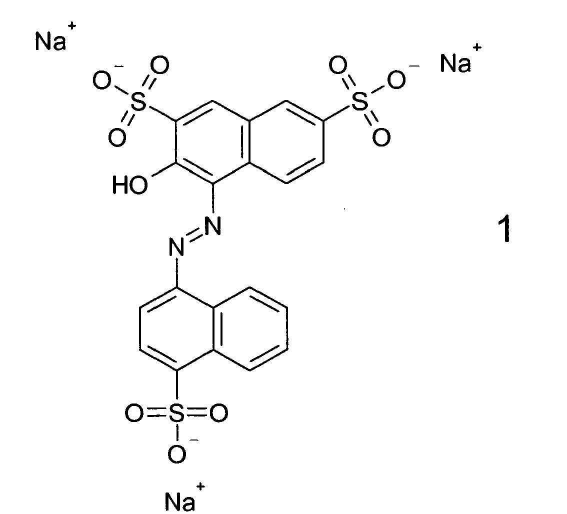

E110, Gelborange S, Sunset Yellow:

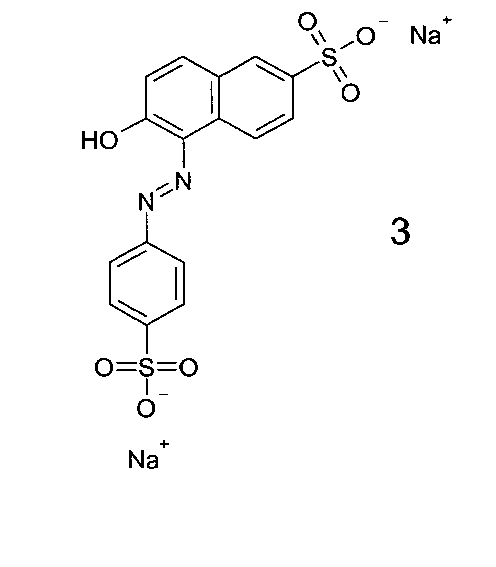

E128, Red 2G, Rot 2G:

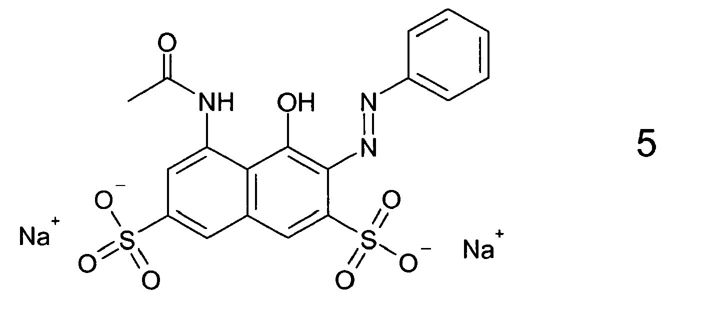

E129, Allura Red, Allurarot AC: 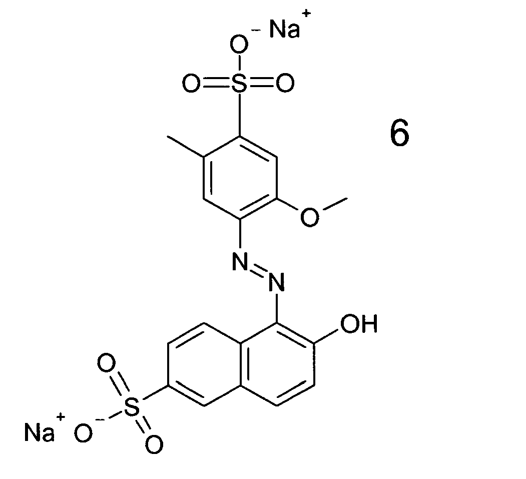 ausgewählt.

Dabei sind die folgenden Kombinationen zweier bevorzugter Azoverbindungen besonders vorteilhaft: E110/E128, E110/E129, E128/E129, E123/E110, E123/E128 und E123/E129, insbesondere E110/E129.

Bevorzugt handelt es sich um ACK gesüsste Getränke, vorzugsweise koffeinhaltige Getränke, die neben den Carotinoid-Partikeln die Azoverbindungen E 110 und E 129 enthalten. Diese können weitere HIS enthalten und sind besonders bevorzugt zuckerfrei und kohlenhydratfrei.

### Beispiele und Vergleichsversuche

### Beispiele 1 bis 18

Die Verwendung von wasserdispergierbaren Carotinoid-Nanopartikeln zur Geschmacksmodulation von Süßstoffen

Für die Beispiele 1 bis 17 wurden die folgenden Stoffe verwendet.

Süßstoffe:
Acesulfam K (ACK) von Fluca Bio Chemika;
Aspartam (ASP) von Fluca Bio Chemika;

Formulierungen (A1) bis (A3):

### Formulierung (A11):

Bestandteile: In Wasser dispergierbare Nanopartikel einer mithilfe elektronenmikroskopischer Aufnahmen bestimmten Partikelgröße von 100 bis 300 nm, enthaltend, bezogen auf die Formulierung (A11), 10 Gew.-% an beta-Carotin-Nanopartikel in Pflanzenöltröpfchen, stabilisiert mit DL-alpha-Tocopherol (E 307) und eingebettet in eine Matrix aus modifizierter Nahrungsmittelstärke (E 1450) und Glucosesirup, sowie Tricalciumphosphat (E 341) als Mittel gegen das Verbacken;

Herstellung: Z.B. mithilfe des von Dieter Horn und Jens Rieger in »Organische Nanopartikel in wässriger Phase - Theorie, Experiment und Anwendung«, in Angewandte Chemie, 2001, Band 113, Seiten 4477, rechte Spalte, letzter Absatz, bis 4478, rechte Spalte, erster Absatz, sowie in dem Abschnitt »4.1.2. In Wasser unlösliche Wirkstoffe«, Seiten 4481 bis 4483, beschriebenen kontinuierlichen Mischkammerverfahrens und anschließender Sprühtrocknung;

### Formulierung (A12):

Bestandteile: In Wasser dispergierbare Nanopartikel einer mithilfe elektronenmikroskopischer Aufnahmen bestimmten Partikelgröße von 100 bis 300 nm, enthaltend, bezogen auf die Formulierung (A12), 10 Gew.-% an beta-Carotin-Nanopartikeln in Pflanzenöltröpfchen, stabilisiert mit DL-alpha-Tocopherol (E 307) und Ascorbylpalmitat (E 304) und eingebettet in eine Matrix aus Fischgelatine (Schutzkolloid) und Glucosesirup, sowie Tricalciumphosphat (E 341) als Mittel gegen das Verbacken;

Herstellung: z.B. mithilfe des von Dieter Horn und Jens Rieger in »Organische Nanopartikel in wässriger Phase - Theorie, Experiment und Anwendung«, in Angewandte Chemie, 2001, Band 113, Seiten 4477, rechte Spalte, letzter Absatz, bis 4478, rechte Spalte, erster Absatz, sowie in dem Abschnitt »4.1.2. In Wasser unlösliche Wirkstoffe«, Seiten 4481 bis 4483, beschriebenen kontinuierlichen Mischkammerverfahrens und anschließender Sprühtrocknung;

### Formulierung (A13):

Bestandteile: In Wasser dispergierbare Nanopartikel einer mithilfe elektronenmikroskopischer Aufnahmen bestimmten mittleren Partikelgröße von 100 bis 300 nm; enthaltend, bezogen auf die Formulierung (A13) , 10 Gew.-% Lycopin-Nanopartikel in Pflanzenöltröpfchen, stabilisiert mit DL-alpha-Tocopherol (E 307) und eingebettet in eine Matrix aus modifizierter Nahrungsmittelstärke (E 1450) und Glucosesirup, sowie Tricalciumphosphat (E 341) als Mittel gegen das Verbacken;

Herstellung: z.B. mithilfe des von Dieter Horn und Jens Rieger in »Organische Nanopartikel in wässriger Phase - Theorie, Experiment und Anwendung«, in Angewandte Chemie, 2001, Band 113, Seiten 4477, rechte Spalte, letzter Absatz, bis 4478, rechte Spalte, erster Absatz, sowie in dem Abschnitt »4.1.2. In Wasser unlösliche Wirkstoffe«, Seiten 4481 bis 4483, beschriebenen kontinuierlichen Mischkammerverfahrens und anschließender Sprühtrocknung;

### Formulierung (A14):

Bestandteile: In Wasser dispergierbare Nanopartikel einer mithilfe elektronenmikroskopischer Aufnahmen ermittelten Partikelgröße von 200 bis 300 nm; enthaltend, bezogen auf die Formulierung (A14), 10 Gew.-% beta-Carotin-Nanopartikel in Maiskeimöltröpfchen, eingebettet in eine Matrix aus modifizierter Nahrungsmittelstärke;

Herstellung z.B. mithilfe des in dem Lehrbuch von J. C. Bauernfeind, »Carotinoids as Colorants and Vitamin A Precursors. Technological and Nutritional Applications«, Chapter 2, J. C. Bauernfeind and H. Kläui, »Carotinoids as Food Color«, Seiten 92 bis 95, Academic Press, ISBN 0-12-082850-2, 1981, beschriebenen Verfahrens und anschließender Sprühtrocknung;

### Formulierung (A2):

O/W-Mikroemulsion, einer mithilfe der quasielastischen Lichtstreuung ermittelten Tröpfchengröße von 200 nm, enthaltend, bezogen auf die Formulierung (A2), 10 Gew.-% beta-Carotin, gelöst in Pflanzenöl und Triglyzeriden von Fettsäuren mittlerer Kettenlänge, stabilisiert mit DL-alpha-Tocopherol (E 307) und Ascorbylpalmitat (E 304) und emulgiert in einem Glycerin/Wasser-Gemisch;

### Formulierung (A3):

Bestandteile: In Wasser dispergierbare Partikel mit Partikelgrößen zwischen 150 und 850 µm; enthaltend, bezogen auf die Formulierung (A3), 10 Gew.-% beta-Carotin-Nanopartikel, modifizierte Stärke, Saccharose, DL-alpha-Tocopherol (E 307), Ascorbinsäure, Natriumascorbat und Tricalciumphosphat (E 341);

Herstellung z.B. durch Mahlung in Suspension und anschließender Sprühtrocknung der resultierenden Partikel.

Azoverbindungen:
Nr. 3: E110, Sunset Yellow der Firma Sigma;
Nr. 6: E129, Allura Red der Firma Sigma;

Cola-Getränk:

Eine so genannte "Null-Cola" ohne Süßstoff wurde wie folgt hergestellt:
- 36 g Cola-Aroma (Cola-Grundstoff der Firma Döhler, Darmstadt, Artikel-Nr. 200380),
- 7,7 g ortho-Phosphorsäure 85% reinst (Karl Roth GmbH + Co KG, Karlsruhe, Artikel-Nr. 9079.1),
- 3,6 g Zitronensäure 99,5% p.a. (Karl Roth GmbH + Co KG, Karlsruhe, Artikel-Nr. 3958.2),
- 2,4 g Natriumbenzoat der Firma Fluka, Sigma-Aldrich, Steinbrunn, und
- 1,2 g Koffein wasserfrei 99% der Firma Fluka, Sigma-Aldrich, Steinbrunn
wurden in 600 ml Leitungswasser gelöst. Von diesem Konzentrat wurden jeweils 50 ml zu einem Liter "Null-Cola" aufgefüllt.

Quantitative sensorische Prüfung - allgemeine Testvorschrift:

Von den Proben 1 bis 18 der Beispiele 1 bis 18 sowie den Kontrollproben 1 bis 6 wurden Konsensprofile in Übereinstimmung mit DIN 10967-2/ISO 11035 erstellt. Dazu wurden 8 trainierte Probanden, die gemäß den DIN/ISO-Vorschriften ausgewählt worden waren, mit den Produkten durch Definition und Trainieren der vorgegebenen Merkmalseigenschaften vertraut gemacht. Anschließend verkosteten die Probanden die Proben 1 bis 17, um den Geschmack, den Nachgeschmack und das Mundgefühl entsprechend den vorgegebenen Merkmalseigenschaften zu bewerten. Die jeweiligen Konsensprofile wurden vom Prüfungsleiter in der Form von Tabellen und Spinnwebdiagrammen zusammengefasst. Im Folgenden werden der Übersichtlichkeit halber lediglich die Tabellen wiedergegeben.

Proben 1 bis 18 und Kontrollproben 1 bis 5 - stoffliche Zusammensetzung:

Die Proben 1 bis 18 und die Kontrollproben 1 bis 5 wiesen die nachstehend beschriebenen stofflichen Zusammensetzungen auf. Die jeweiligen Abkürzungen, die in den nachfolgenden Tabellen verwendet werden, werden in Klammern angegeben.
Kontrollprobe 1:
   Wasser + 500 mg/l ACK (Abkürzung: Wasser/ACK)
Kontrollprobe 2:
   Wasser + 350 mg/l ASP (Abkürzung: Wasser/ASP)
Kontrollprobe 3:
   Wasser + 140 mg/l ACK + 350 mg/l ASP (Abkürzung: Wasser/ACK/ASP)
Kontrollprobe 4:
   Null-Cola + 500 mg/l ACK (Abkürzung: Cola/ACK)
Kontrollprobe 5:
   Null-Cola + 140 mg/l ACK + 350 mg/l ASP (Abkürzung: Cola/ACK/ASP)
Probe 1 - Beispiel 1:
   Wasser + 500 mg/l ACK + Formulierung (A11), entsprechend 1 ppm beta-Carotin (Abkürzung: Wasser/ACK/A11)
Probe 2 - Beispiel 2:
   Wasser + 500 mg/l ACK + Formulierung (A13), entsprechend 1 ppm Lycopin (Abkürzung: Wasser/ACK/A13)
Probe 3 - Beispiel 3:
   Wasser + 500 mg/l ACK + Formulierung (A12), entsprechend 1 ppm beta-Carotin (Abkürzung: Wasser/ACK/A12)
Probe 4 - Beispiel 4:
   Wasser + 500 mg/l ACK + Formulierung (A11), entsprechend 1 ppm beta-Carotin, + 2,3 mg/l Azoverbindung E110 + Azoverbindung 2,5 mg/l E129 (Abkürzung: Wasser/ACK/A11/E110/E129)
Probe 5 - Beispiel 5:
   Wasser + 140 mg/l ACK + 350 mg/l ASP + Formulierung (A11), entsprechend 1 ppm beta- Carotin (Abkürzung: Wasser/ACK/ASP/A11)
Probe 6 - Beispiel 6:
   Wasser + 350 mg/l ASP + Formulierung (A11), entsprechend 1 ppm beta-Carotin (Abkürzung: Wasser/ASP/A11)
Probe 7 - Beispiel 7:
   Wasser + 140 mg/l ACK + 350 mg/l ASP + Formulierung (A12), entsprechend 1 ppm beta-Carotin (Abkürzung: Wasser/ACK/ASP/A12)
Probe 8 - Beispiel 8:
   Null-Cola + 500 mg/l ACK + Formulierung (A11), entsprechend 1 ppm beta-Carotin (Abkürzung: Cola/ACK/A11)
Probe 9 - Beispiel 9:
   Null-Cola + 140 mg/l ACK + 350 mg/l ASP + Formulierung (A11), entsprechend 1 ppm beta-Carotin (Abkürzung: Cola/ACK/ASP/A11)
Probe 10 - Beispiel 10:
   Null-Cola + 140 mg/l ACK + 350 mg/l ASP + Formulierung (A12), entsprechend 1 ppm beta-Carotin (Abkürzung: Cola/ACK/ASP/A12)
Probe 11 - Beispiel 11:
   Null-Cola + 500 mg/l ACK + Formulierung (A11), entsprechend 1 ppm beta-Carotin, + 2,5 mg/l Azoverbindung E129 (Abkürzung: Cola/ACK/A11/E129)
Probe 12 - Beispiel 12:
   Null-Cola + 500 mg/l ACK + Formulierung (A11), entsprechend 1 ppm beta-Carotin, + 2,3 mg/l Azoverbindung E110 (Abkürzung: Cola/ACK/A11/E110)
Probe 13 - Beispiel 13:
   Null-Cola + 500 mg/l ACK + Formulierung (A11), entsprechend 1 ppm beta-Carotin, + 2,3 mg/l Azoverbindung E110 + 2,5 mg/l Azoverbindung E129 (Abkürzung: Cola/ACK/A11/E110)
Probe 14 - Beispiel 14:
   Null-Cola + 140 mg/l ACK + 350 mg/l ASP + Formulierung (A11), entsprechend 1 ppm beta-Carotin, + 2,3 mg/l Azoverbindung E110 (Abkürzung: Cola/ACK/ASP/A11/E110)
Probe 15 - Beispiel 15:
   Null-Cola + 140 mg/l ACK + 350 mg/l ASP + Formulierung (A11), entsprechend 1 ppm beta-Carotin, + 2,3 mg/l Azoverbindung E110 + 2,5 mg/l Azoverbindung E129 (Abkürzung: Cola/ACK/ASP/A11/E110/E129)
Probe 16 - Beispiel 16:
   Wasser + 500 mg/l ACK + Formulierung (A14), entsprechend 1 ppm beta-Carotin (Abkürzung: Wasser/ACK/A14)
Probe 17 - Beispiel 17:
   Wasser + 500 mg/l ACK + Formulierung (A2), entsprechend 1 ppm beta-Carotin (Abkürzung: Wasser/ACK/A2)
Probe 18 - Beispiel 18:
   Wasser + 500 mg/l ACK + Formulierung (A3), entsprechend 1 ppm beta-Carotin (Abkürzung: Wasser/ACK/A3)

Proben 1 bis 7 sowie 16 bis 18 und Kontrollproben 1 bis 3 - Versuchsergebnisse:

Die Ergebnisse der quantitativen sensorischen Prüfung der Proben 1 bis 7 und 16 bis 18 sowie der Kontrollproben 1 bis 3 sind in der Tabelle 1 zusammengestellt. Der Messwert 0 bedeutet, dass die betreffende sensorische Eigenschaft nicht vorhanden war; der Messwert 10 bedeutet die betreffende sensorische Eigenschaft stark vorhanden war.

**Tabelle 1: Quantitative sensorische Prüfung der Proben 1 bis 7 und 16 bis 18 sowie der Kontrollproben 1 bis 3 - Konsensprofile**

| Kontrollprobe/Probe | Geschmack | | Mundgefühl | | | | Nachgeschmack | | | |
|---|---|---|---|---|---|---|---|---|---|---|
| | S^{a)} | Bt^{b)} | C^{c)} | K^{d)} | Bl^{e)} | A^{f)} | S(N)^{g)} | Bt(N)^{h)} | A(N)ⁱ⁾ | C(N)^{j)} |
| Kontrollprobe 1 Wasser/ACK | 6 | 4 | 4 | 0 | 4 | 2 | 3 | 6 | nb^{k)} | 0 |
| | | | | | | | | | | |
| Kontrollprobe 2 Wasser/ASP | 3^{l)} | 1 | 0 | 0 | 1 | 2 | 1^{m)} | 0 | 2 | nb^{k)} |
| | | | | | | | | | | |
| Kontrollprobe 3 Wasser/ACK/ASP | 8 | 0 | 2 | 0 | 2 | 1 | 6 | 0 | 0 | nb^{k)} |
| | | | | | | | | | | |
| Probe 1 Wasser/ACK/A11 | 8 | 2 | 3 | 0 | 1 | 4 | 4 | 0 | nb^{k)} | nb^{k)} |
| | | | | | | | | | | |
| Probe 2 Wasser/ACK/A13 | 7 | 5 | 5 | 1 | 1 | 3 | 3 | 1 | nb^{k)} | nb^{k)} |
| | | | | | | | | | | |
| Probe 3 Wasser/ACK/A12 | 5 | 3 | 4 | 0 | 6 | 4 | 2 | 0 | nb^{k)} | 0 |
| | | | | | | | | | | |
| Probe 4 Wasser/ACK/A12/ E110/E129 | 7 | 0 | 2 | 0 | 1 | 3 | 3 | 0 | nb^{k)} | nb^{k)} |
| | | | | | | | | | | |
| Probe 5 Wasser/ACK/ ASP/A11 | 9 | 0 | 1 | 0 | 2 | 1 | 5 | 0 | 3 | nb^{k)} |
| | | | | | | | | | | |
| Probe 6 Wasser/ASP/A11 | 3 | 0 | 0 | 0 | 2 | 2 | 3 | 0 | 2 | nb^{k)} |
| | | | | | | | | | | |
| Probe 7 Wasser/ACK/ ASP/A12 | 8 | 0 | 3 | 0 | 2 | 1 | 6 | 0 | 0 | nb^{k)} |
| | | | | | | | | | | |
| Probe 16 Wasser/ACK/A14 | 4 | 1 | 4 | 0 | 2 | 3 | 2 | 1 | nb^{k)} | nb^{k)} |
| Probe 17 Wasser/ACK/A2 | 6 | 4 | 4 | 0 | 1 | 2 | 3 | 1 | nb^{k)} | nb^{k)} |
| Probe 18 Wasser/ACK/A3 | 5 | 4 | 3 | 0 | 1 | 2 | 3 | 5 | nb^{k)} | nb^{k)} |

| | | | | | | | | | | |
|---|---|---|---|---|---|---|---|---|---|---|
| a) S = süß; b) Bt = bitter; c) C = chemisch; d) K = kratzend; e) BI = belegend; f) A = austrocknend; g) S(N) = süßer Nachgeschmack; h) Bt(N) = bitterer Nachgeschmack; i) A(N) = im Nachgeschmack austrocknend; j) C(N) = chemischer Nachgeschmack; k) nb = nicht bestimmt; l) erst wässriger, dann süßer Geschmack; m) Nachgeschmack klingt schnell ab; | | | | | | | | | | |

Aus den Ergebnissen der Tabelle 1 ergaben sich für die Proben 1 bis 7 im Einzelnen die folgenden geschmacksmodulierenden Wirkungen:

### Probe 1:

Die Formulierung (A11) reduzierte den bitteren Geschmack und reduzierten völlig den bitteren Nachgeschmack von ACK in Wasser.

### Probe 2:

Die Formulierung (A13) reduzierte den bitteren Nachgeschmack von ACK in Wasser.

### Probe 3:

Die Formulierung (A12) reduzierte den bitteren Nachgeschmack von ACK in Wasser völlig; der bittere Geschmack wurde leicht reduziert. Allerdings wurde die Probe 3 als etwas stärker belegend und etwas weniger süß als die Probe 1 empfunden.

### Probe 4:

Die Dreierkombination Formulierung (A11)/E110/E129 wies synergistische Effekte bei der Modulation des bitteren Geschmacks und Nachgeschmacks auf. Sowohl der bittere Geschmack als auch der bittere Nachgeschmack wurden völlig reduziert.

### Probe 5:

Die Formulierung (A11) beeinflusste den Geschmack und den Nachgeschmack von ACK/ASP in Wasser nur in sehr geringem Umfang.

### Probe 6:

Die Formulierung (A11) bewirkte kaum Geschmacksänderungen von ASP in Wasser. Allerdings wurde der geringfügige bittere Geschmack von ASP in Wasser nicht mehr wahrgenommen.

### Probe 7:

Die Formulierung (A12) beeinflusste den Geschmack und den Nachgeschmack von ACK/ASP in Wasser nur in sehr geringem Umfang.

### Probe 16:

Die Formulierung (A14) reduzierte den bitteren Geschmack und reduzierten völlig den bitteren Nachgeschmack von ACK in Wasser. Außerdem war die Probe 16 weniger belegend als die Kontrollprobe 1.

### Probe 17:

Die Formulierung (A2) reduzierte den bitteren Geschmack und reduzierten völlig den bitteren Nachgeschmack von ACK in Wasser. Außerdem war die Probe 17 deutlich weniger belegend als die Kontrollprobe 1.

### Probe 18:

Die Formulierung (A3) reduzierte etwas den bitteren Nachgeschmack von ACK in Wasser. Außerdem schmeckte die Probe 18 deutlich weniger chemisch und war deutlich weniger belegend als die Kontrollprobe 1.

Insgesamt konnten mithilfe der Formulierungen (A11) bis (A14) sowie (A2) und (A3) insbesondere in Kombination mit den Azoverbindungen E110 und E129 der bittere Geschmack und der bittere Nachgeschmack von ACK in Wasser signifikant reduziert werden.

Proben 8 bis 15 und Kontrollproben 4 und 5 - Versuchsergebnisse:

Die Ergebnisse der quantitativen sensorischen Prüfungen der Proben 8 bis 15 und der Kontrollproben 4 und 5 sind in der Tabelle 2 zusammengestellt. Der Messwert 0 bedeutet, dass die betreffende sensorische Eigenschaft nicht vorhanden war; der Messwert 10 bedeutet die betreffende sensorische Eigenschaft stark vorhanden war.

**Tabelle 2: Quantitative sensorische Prüfung der Proben 8 bis 15 und der Kontrollproben 4 und 5 - Konsensprofile**

| Kontrollprobe/ Probe | Geschmack | | | | | | Mundgefühl | | | | Nachgeschmack | | |
|---|---|---|---|---|---|---|---|---|---|---|---|---|---|
| | BS^{a)} | Sä^{b)} | Bt^{c)} | KC^{d)} | M^{e)} | K(N)^{m)} | Co^{f)} | S^{g)} | A^{h)} | BIⁱ⁾ | Au^{j)} | S(N)^{k)} | Bt(N)^{l)} |
| Kontrollprobe 4 Cola/ACK | 1 | 6 | 4 | 4 | 2 | 4 | 4 | 2 | 4 | 3 | 3 | 1 | 0 |
| | | | | | | | | | | | | | |
| Kontrollprobe 5 Cola/ACK/ASP | 7 | 4 | 1ⁿ⁾ | 4 | 2 | 5 | 6 | 0 | 1 | 2 | 6 | 0 | 2 |
| | | | | | | | | | | | | | |
| Probe 8 Cola/ACK/A11 | 1 | 6 | 1 | 4 | 3 | 3 | 3 | 1 | 3 | 3 | 3 | 1 | 2 |
| | | | | | | | | | | | | | |
| Probe 9 Cola/ACK/ ASP/A11 | 8 | 3 | 0 | 4 | 2 | 4 | 9 | 1 | 1 | 3 | 5 | 0 | 2 |
| | | | | | | | | | | | | | |
| Probe 10 Cola/ACK/ ASP/A12 | 6 | 5 | 0 | 4 | 1 | 4 | 5 | 0 | 1 | 1 | 5 | 0 | 1 |
| | | | | | | | | | | | | | |
| Probe 11 Cola/ACK/A11/ E129 | 0 | 3ⁿ⁾ | 0 | 5 | 4 | 2 | 2 | 0 | 1 | 1 | 2 | 0 | 0 |
| | | | | | | | | | | | | | |
| Probe 12 Cola/ACK/A11/ E110 | 2 | 5 | 0 | 4 | 2 | 3 | 4 | 0 | 1 | 3 | 3 | 0 | 0 |
| | | | | | | | | | | | | | |
| Probe 13 Cola/ACK/A11/ E129/E110 | 3 | 4 | 0 | 3 | 1 | 5 | 5 | 0 | 1 | 2 | 4 | 0 | 0 |
| | | | | | | | | | | | | | |
| Probe 14 Cola/ACK/ASP/ A11/ E110 | 5 | 3 | 0 | 2 | 0 | 5 | 5 | 0 | 1 | 1 | 5 | 0 | 0 |
| Probe 15 Cola/ACK/ASP/ A11/E129/E1107 | | 3 | 0 | 2 | 0 | 5 | 6 | 0 | 1 | 1 | 6 | 0 | 0 |

| | | | | | | | | | | | | | |
|---|---|---|---|---|---|---|---|---|---|---|---|---|---|
| a) BS = Beginn der Süße; b) Sä = sauer; c) Bt = bitter; d) KC = künstlich/chemisch; e) M = metallisch; f) Co = Cola; g) S = süß; h) A = adstringierend; i) BI = belegend; j) Au = austrocknend; k) S(N) = süßer Nachgeschmack; l) Bt(N) = bitterer Nachgeschmack; m) K(N) = kratzender Nachgeschmack n) nur beim Abschlucken für etwa 10 Sekunden; | | | | | | | | | | | | | |

Aus den Ergebnissen der Tabelle 2 ergaben sich für die Proben 8 bis 15 im Einzelnen die folgenden geschmacksmodulierenden Wirkungen.

### Probe 8:

Die Formulierung (A11) hatte einen positiven Effekt bei der Modulation des bitteren Geschmacks. Allerdings nimmt das Colaaroma leicht ab und die Probe hat einen kratzenden Nachgeschmack.

### Probe 9:

Die Formulierung (A11) beeinflusste den Geschmack von ACK/ASP in Cola nur wenig. Die Probe 9 war etwas weniger sauer und nicht bitter. Sie wies etwas weniger Colaaroma aber etwas mehr Süße auf.

### Probe 10:

Die Formulierung (A12) beeinflusste den Geschmack von ACK/ASP in Cola nur wenig. Es war allerdings kein bitterer Geschmack mehr vorhanden.

### Probe 11:

Die Formulierung (A11) und E129 reduzierten den bitteren Geschmack und den bitteren Nachgeschmack von ACK in Cola völlig. Der süße Geschmack und der süße Nachgeschmack wurden etwas reduziert.

### Probe 12:

Die Formulierung (A11) und E110 reduzierten den bitteren Geschmack und den bitteren Nachgeschmack von ACK in Cola völlig. Die Probe 12 erwies sich außerdem als nicht adstringierend. Das Colaaroma wurde nur etwas reduziert.

### Probe 13:

Die Formulierung (A11), E110 und E129 reduzierten den bitteren Geschmack und Nachgeschmack von ACK in Cola völlig. Die anderen negativen Geschmacksattribute (sauer, künstlich/chemisch, metallisch, adstringierend, belegend, austrocknend) wurden reduziert, wogegen die positiven Geschmacksattribute (Süße, Cola) verbessert wurden.

### Probe 14:

Die Formulierung (A11) und E110 reduzierten etwas den sauren, bitteren, künstlich/chemischen und metallischen Geschmack sowie den kratzenden Nachgeschmack von ACK/ASP in Cola. Die Süße war nicht mehr so intensiv ausgeprägt wie in der Kontrollprobe 6.

### Probe 15:

Die Formulierung (A11), E110 und E129 reduzierten etwas den sauren, bitteren, künstlich/chemischen und metallischen Geschmack sowie den kratzenden Nachgeschmack von ACK/ASP in Cola. Außerdem war die Probe 15 weniger austrocknend als die Kontrollprobe 6. Der bittere Nachgeschmack wurde nicht reduziert. Ansonsten wurden die positiven Geschmacksattribute (Süße, Cola) nicht beeinflusst.

### Vergleichsversuche V1 und V2

Die Verwendung von carotinoidfreien Placebos V2 und V1 zur Geschmacksmodulation von Süßstoffen

### Beispiel 1 wurde wiederholt, nur dass anstelle der Formulierung (A11)

- bei Vergleichsversuch V1 ein carotinoidfreier Placebo V1, der aus modifizierter Stärke, Tocopherol, Pflanzenöl und Glucose nach dem von Dieter Horn und Jens Rieger in »Organische Nanopartikel in wässriger Phase - Theorie, Experiment und Anwendung«, in Angewandte Chemie, 2001, Band 113, Seiten 4477, rechte Spalte, letzter Absatz, bis 4478, rechte Spalte, erster Absatz, sowie in dem Abschnitt »4.1.2. In Wasser unlösliche Wirkstoffe«, Seiten 4481 bis 4483, beschriebenen kontinuierlichen Mischkammerverfahren und Sprühtrocknen hergestellt worden war (Abkürzung: Wasser/ACK/V1), und
- bei Vergleichsversuch V2 ein carotinoidfreier Placebo V2, der durch Vermischen von modifizierter Stärke, Tocopherol, Pflanzenöl und Glucose und Sprühtrocknen des resultierenden Gemischs hergestellt worden war (Abkürzung: Wasser/ACK/V2),

### verwendet wurden.

Die Ergebnisse der quantitativen sensorischen Prüfungen der Proben V1 und V2 der Vergleichsversuche V1 und V2 werden in der Tabelle 3 den an der Kontrollprobe 1 gewonnenen Ergebnissen gegenübergestellt. Die Messwerte haben die vorstehend bei den Tabellen 1 und 2 angegebene Bedeutung.

**Tabelle 3: Quantitative sensorische Prüfung der Proben V1 und V2 und der Kontrollprobe 1 - Konsensprofile**

| Kontrollprobe/ | Geschmack | | Mundgefühl | | | | Nachgeschmack | |
|---|---|---|---|---|---|---|---|---|
| Probe | S^{a)} | Bt^{b)} | C^{c)} | K^{d)} | Bl^{e)} | A^{f)} | S(N)^{g)} | Bt(N)^{h)} |
| Kontrollprobe 1 Wasser/ACK | 6 | 4 | 4 | 0 | 4 | 2 | 3 | 6 |
| | | | | | | | | |
| Probe V1 Wasser/ACK/V1 | 8 | 6 | 4 | 0 | 2 | 2 | 5 | 2 |
| | | | | | | | | |
| Probe V3 Wasser/ACK/V2 | 7 | 5 | 4 | 0 | 4 | 3,5 | 4 | 1 |

| | | | | | | | | |
|---|---|---|---|---|---|---|---|---|
| a) S = süß; b) Bt = bitter; c) C = chemisch; d) K = kratzend; e) BI = belegend; f) A = austrocknend; g) S(N) = süßer Nachgeschmack; h) Bt(N) = bitterer Nachgeschmack; | | | | | | | | |

Die Vergleichsversuche V1 und V2 zeigten, dass die von Beta-Carotin freien Placebos V1 und V2 den bitteren Geschmack von ACK in Wasser eher erhöhten, wogegen sie den bitteren Nachgeschmack reduzieren konnten.

## Patentansprüche

1. Verwendung von wasserdispergierbaren Carotinoid-Nanopartikeln als Geschmacksmodulatoren in stofflichen Zusammensetzungen.

2. Verwendung nach Anspruch 1, **dadurch gekennzeichnet, dass**, die Carotinoid-Nanopartikel eine Partikelgröße < 1 µm haben.

3. Verwendung nach Anspruch 2, **dadurch gekennzeichnet, dass** die Carotinoid-Nanopartikel flüssig oder fest sind.

4. Verwendung nach einem der Ansprüche 1 bis 3, **dadurch gekennzeichnet, dass** die Carotinoide in den festen Carotinoid-Nanopartikeln röntgenamorph sind.

5. Verwendung nach einem der Ansprüche 1 bis 4, **dadurch gekennzeichnet, dass** die Carotinoide in den flüssigen Carotinoid-Nanopartikeln in einem flüssigen Medium gelöst oder in der Form von solubilisierten gemischten Micellen vorliegen.

6. Verwendung nach einem der Ansprüche 1 bis 5, **dadurch gekennzeichnet, dass** die Carotinoid-Nanopartikel in flüssigen oder festen Formulierungen vorliegen, die mindestens einen lebensmittelrechtlich und/oder arzneimittelrechtlich zugelassenen Zusatzstoff enthalten.

7. Verwendung nach Anspruch 6, **dadurch gekennzeichnet, dass** der Zusatzstoff aus der Gruppe, bestehend aus Schutzkolloiden, Stabilisatoren gegen den oxidativen Abbau, Emulgatoren, Ölen, Weichmachern, Mitteln gegen das Verbacken und Polyolen, ausgewählt wird.

8. Verwendung nach einem der Ansprüche 1 bis 7, **dadurch gekennzeichnet, dass** die Carotinoid-Nanopartikel der festen Formulierungen in eine Matrix aus mindestens einem Zusatzstoff eingebettet sind.

9. Verwendung nach einem der Ansprüche 6 bis 8, **dadurch gekennzeichnet, dass** die Formulierungen der Carotinoid-Nanopartikel, bezogen auf ihre jeweilige Gesamtmenge, 0,5 bis 30 Gew.-% an Carotinoid oder Carotinoiden enthalten.

10. Verwendung nach einem der Ansprüche 6 bis 9, **dadurch gekennzeichnet, dass** die Formulierungen der Carotinoid-Nanopartikel, bezogen auf ihre jeweilige Gesamtmenge, 10 bis 80 Gew.-% an Schutzkolloid oder Schutzkolloiden enthalten.

11. Verwendung nach einem der Ansprüche 6 bis 10, **dadurch gekennzeichnet, dass** die Formulierungen der Carotinoid-Nanopartikel, bezogen auf ihre jeweilige Gesamtmenge, 20 bis 70 Gew.-% an Weichmacher enthalten.

12. Verwendung nach einem der Ansprüche 1 bis 11, **dadurch gekennzeichnet, dass** die wasserdispergierbaren Carotinoid-Nanopartikel der Reduzierung des bitteren Geschmacks und des bitteren Nachgeschmacks der stofflichen Zusammensetzungen dienen.

13. Verwendung nach einem der Ansprüche 1 bis 12, **dadurch gekennzeichnet, dass** es sich bei der stofflichen Zusammensetzungen um Lebensmittel, Getränke Genussmittel, Süßungsmittel, Tiernahrungsmittel, Kosmetika und Pharmazeutika handelt.

14. Verwendung nach einem der Ansprüche 1 bis 13, **dadurch gekennzeichnet, dass** es sich bei der stofflichen Zusammensetzung um eine zuckerarme Zusammensetzung mit weniger als 10 g Zucker pro Liter bzw. kg Zusammensetzung, insbesondere um eine zuckerfreie, handelt.

15. Verwendung nach einem der Ansprüche 1 bis 14, **dadurch gekennzeichnet, dass** die stofflichen Zusammensetzungen mindestens einen High Intensity Sweetener HIS als Süßungsmittel enthalten.

16. Verwendung nach einem der Ansprüche 1 bis 15, **dadurch gekennzeichnet, dass** die Carotinoid-Nanopartikel oder ihre Formulierungen in festen stofflichen Zusammensetzungen in einer Menge vorhanden sind, dass eine Konzentration an Carotinoiden von 0,1 bis 100 ppm resultiert.

17. Verwendung nach einem der Ansprüche 1 bis 15, **dadurch gekennzeichnet, dass** die Carotinoid-Nanopartikeln oder ihre Formulierungen in flüssigen stofflichen Zusammensetzungen in einer Menge vorhanden sind, dass eine Konzentration an Carotinoiden von 0,1 bis 100 mg/l resultiert.

18. Verwendung nach einem der Ansprüche 1 bis 17, **dadurch gekennzeichnet, dass** die stofflichen Zusammensetzungen mindestens eine den bitteren Geschmack und Nachgeschmack reduzierende Azoverbindung mit mindestens einer Azogruppe enthalten.

19. Verwendung nach Anspruch 18, **dadurch gekennzeichnet, dass** die stofflichen Zusammensetzungen, bezogen auf ihre jeweilige Gesamtmenge, 0,1 bis 100 ppm an Azoverbindung oder Azoverbindungen enthalten.

20. Verwendung nach Anspruch 18 oder 19, **dadurch gekennzeichnet, dass** die Azoverbindungen aus der Gruppe, bestehend aus E110, E123, E128 und E129 ausgewählt werden:
E123: 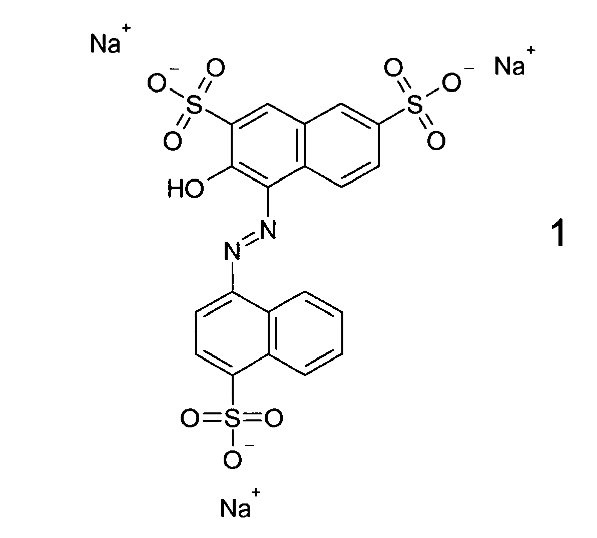
E110: 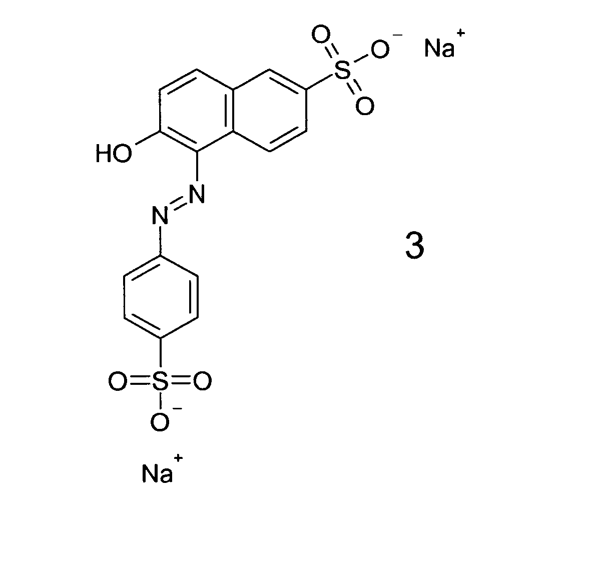
E128: 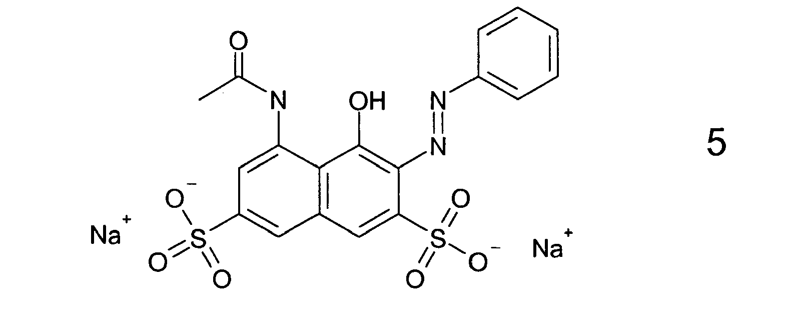
E129:

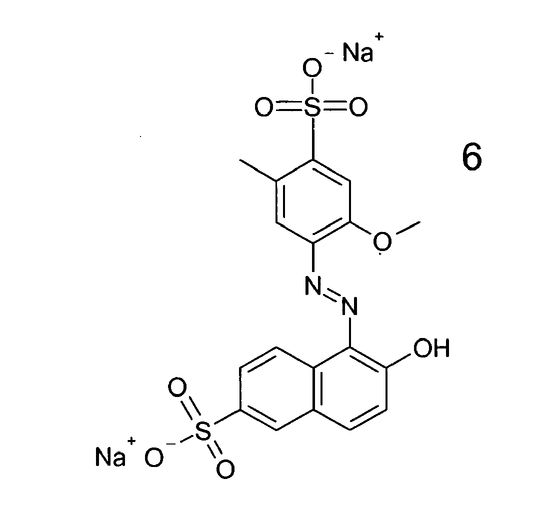

21. Verwendung nach einem der Ansprüche 15 bis 20, **dadurch gekennzeichnet, dass** der oder die HIS aus der Gruppe, bestehend aus Acesulfam-Kalium, Aspartam, Saccharin und seinen Salzen, Cyclamat und seinen Salzen, Aspartam-Acesulfam-Salz, Sucralose, Thaumatin, Stevia, Steviosid und Neohesperidin-Dihydrochalkon, ausgewählt wird oder werden.

22. Verfahren zur Geschmacksmodulation von stofflichen Zusammensetzungen, **dadurch gekennzeichnet, dass** man mindestens einen Typ von wasserdispergierbaren Carotinoid-Nanopartikeln den stofflichen Zusammensetzungen zusetzt.

23. Verfahren nach Anspruch 22, **dadurch gekennzeichnet, dass** die wasserdispergierbaren Carotinoid-Nanopartikel in einer Menge zugesetzt werden, dass, in festen stofflichen Zusammensetzungen eine Konzentration an Carotinoiden von 0,1 bis 100 ppm und in flüssigen stofflichen Zusammensetzungen eine Konzentration an Carotinoiden von 0,1 bis 100 mg/l resultiert.

24. Verfahren nach Anspruch 22 oder 23, **dadurch gekennzeichnet, dass** die stofflichen Zusammensetzungen Lebensmittel, Getränke, Genussmittel, Süßungsmittel, Tiernahrungsmittel, Kosmetika und Pharmazeutika sind.

25. Verfahren nach Anspruch 22 bis 24, **dadurch gekennzeichnet, dass** es sich bei den stofflichen Zusammensetzungen um zuckerarme Zusammensetzungen mit weniger als 10 g Zucker pro Liter bzw. kg, insbesonders um zuckerfreie, Zusammensetzung handelt.

26. Verfahren nach einem der Ansprüche 22 bis 25, **dadurch gekennzeichnet, dass** die stofflichen Zusammensetzungen mindestens einen High Intensity Sweetener HIS als Süßungsmittel enthalten.

27. Verfahren nach einem der Ansprüche 22 bis 26, **dadurch gekennzeichnet, dass** den stofflichen Zusammensetzungen noch mindestens eine den bitteren Geschmack und Nachgeschmack reduzierende Azoverbindung mit mindestens einer Azogruppe zugesetzt wird.

28. Verfahren nach Anspruch 27, **dadurch gekennzeichnet, dass** den stofflichen Zusammensetzungen, bezogen auf ihre jeweilige Gesamtmenge, 0,1 bis 100 ppm an Azoverbindung oder Azoverbindungen zugesetzt werden.

29. Verfahren nach Anspruch 27 oder 28, **dadurch gekennzeichnet, dass** die Azoverbindungen aus der Gruppe, bestehend aus E110, E123, E128 und E129 ausgewählt werden:
E123: 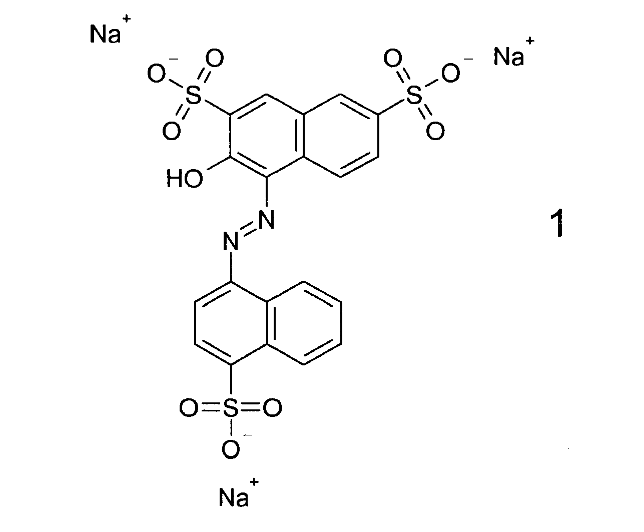
E110: 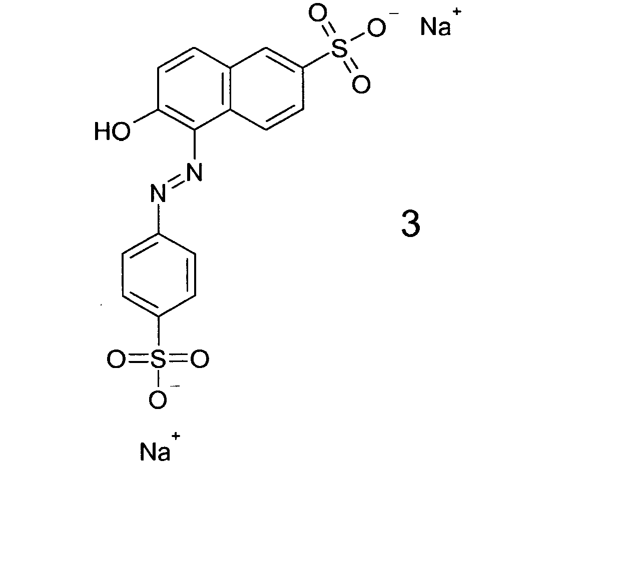
E128: 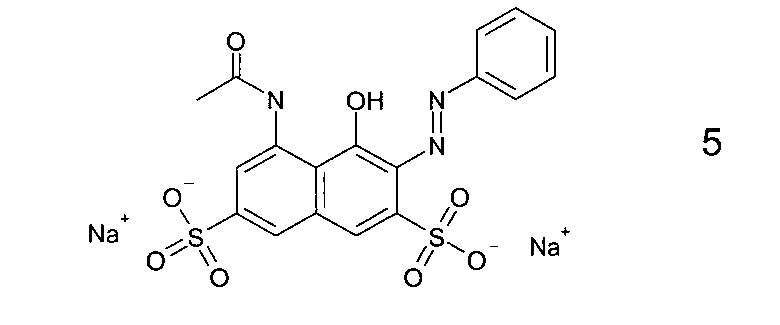
E 129:

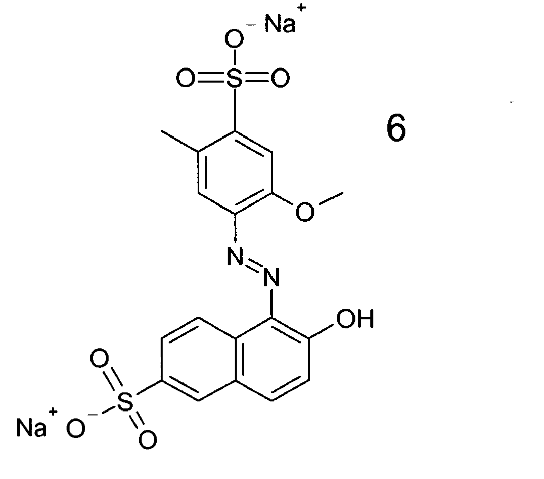

30. Verfahren nach einem der Ansprüche 26 bis 29, **dadurch gekennzeichnet, dass** der oder die HIS aus der Gruppe, bestehend aus Acesulfam-Kalium, Aspartam, Saccharin und seinen Salzen, Cyclamat und seinen Salzen, Aspartam-Acesulfam-Salz, Sucralose, Thaumatin und Neohesperidin-Dihydrochalkon, ausgewählt wird oder werden.

31. Verfahren nach einem der Ansprüche 22 bis 30, **dadurch gekennzeichnet, dass** bei der Geschmacksmodulation der bittere Geschmack und der bittere Nachgeschmack der stofflichen Zusammensetzungen reduziert werden.

32. Geschmacksmodulatoren für stoffliche Zusammensetzungen, enthaltend
(A) mindestens einen Typ von wasserdispergierbaren Carotinoid-Nanopartikeln
(B) mindestens eine Azoverbindung, enthaltend mindestens eine Azogruppe.

33. Geschmacksmodulatoren nach Anspruch 32, **dadurch gekennzeichnet, dass** das Gewichtsverhältnis von (A) zu (B) = 10 : 1 bis 1 : 20.

34. Geschmacksmodulatoren nach Anspruch 32 oder 33, **dadurch gekennzeichnet, dass**, die Carotinoid-Nanopartikel eine Partikelgröße < 1 µm haben.

35. Geschmacksmodulatoren nach einem der Ansprüche 32 bis 34, **dadurch gekennzeichnet, dass** die Carotinoid-Nanopartikel flüssig oder fest sind.

36. Geschmacksmodulatoren nach einem der Ansprüche 32 bis 35, **dadurch gekennzeichnet, dass** die Carotinoide in den festen Carotinoid-Nanopartikeln röntgenamorph sind.

37. Geschmacksmodulatoren nach einem der Ansprüche 32 bis 36, **dadurch gekennzeichnet, dass** die Carotinoide in den flüssigen Carotinoid-Nanopartikeln in einem flüssigen Medium gelöst oder in der Form von solubilisierten gemischten Micellen vorliegen.

38. Geschmacksmodulatoren nach einem der Ansprüche 32 bis 37, **dadurch gekennzeichnet, dass** die Carotinoid-Nanopartikel in flüssigen oder festen Formulierungen vorliegen, die mindestens einen lebensmittelrechtlich und/oder arzneimittelrechtlich zugelassenen Zusatzstoff enthalten.

39. Geschmacksmodulatoren nach Anspruch 38, **dadurch gekennzeichnet, dass** der Zusatzstoff aus der Gruppe, bestehend aus Schutzkolloiden, Stabilisatoren gegen den oxidativen Abbau, Emulgatoren, Ölen, Weichmachern, Mitteln gegen das Verbacken und Polyolen, ausgewählt ist.

40. Geschmacksmodulatoren nach Anspruch 38 oder 39, **dadurch gekennzeichnet, dass** die Carotinoid-Nanopartikel der festen Formulierungen in eine Matrix aus mindestens einem Zusatzstoff eingebettet sind.

41. Geschmacksmodulatoren nach einem der Ansprüche 38 bis 40, **dadurch gekennzeichnet, dass** die Formulierungen der Carotinoid-Nanopartikel, bezogen auf ihre jeweilige Gesamtmenge, 0,5 bis 30 Gew.-% an Carotinoid oder Carotinoiden enthalten.

42. Geschmacksmodulatoren nach einem der Ansprüche 38 bis 41, **dadurch gekennzeichnet, dass** die Formulierungen der Carotinoid-Nanopartikel, bezogen auf ihre jeweilige Gesamtmenge, 10 bis 80 Gew.-% an Schutzkolloid oder Schutzkolloiden enthalten.

43. Geschmacksmodulatoren nach einem der Ansprüche 38 bis 42, **dadurch gekennzeichnet, dass** die Formulierungen der Carotinoid-Nanopartikel, bezogen auf ihre jeweilige Gesamtmenge, 20 bis 70 Gew.-% an Weichmacher oder an Weichmachern enthalten.

44. Geschmacksmodulatoren nach einem der Ansprüche 32 bis 43, **dadurch gekennzeichnet, dass** die Azogruppen der Azoverbindungen (B) mit Arylgruppen verknüpft sind.

45. Geschmacksmodulatoren nach Anspruch 44, **dadurch gekennzeichnet, dass** die Arylgruppen Phenylgruppen und/oder Naphthylgruppen sind.

46. Geschmacksmodulatoren nach Anspruch 44 oder 45, **dadurch gekennzeichnet, dass** mindestens eine Arylgruppe mit mindestens einer Sulfonsäuregruppe und/oder mindestens einer Hydroxylgruppe substituiert ist.

47. Geschmacksmodulatoren nach einem der Ansprüche 44 bis 46, **dadurch gekennzeichnet, dass** die Azoverbindungen (B) aus der Gruppe, bestehend aus E110, E123, E128 und E129 ausgewählt sind:
E123: 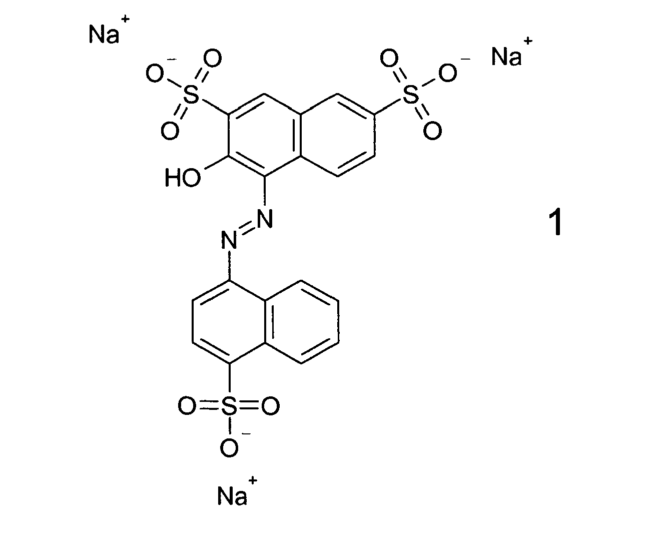
E110: 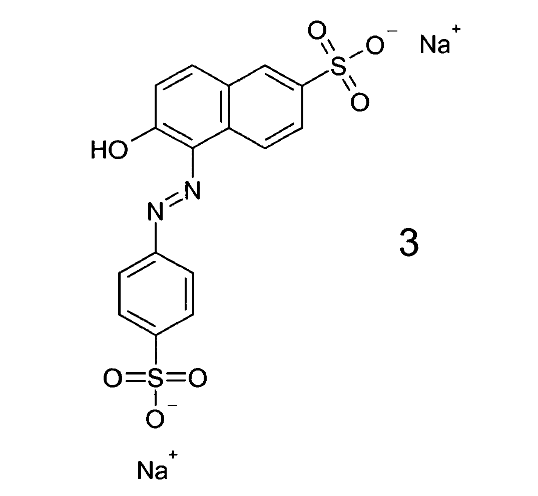
E128: 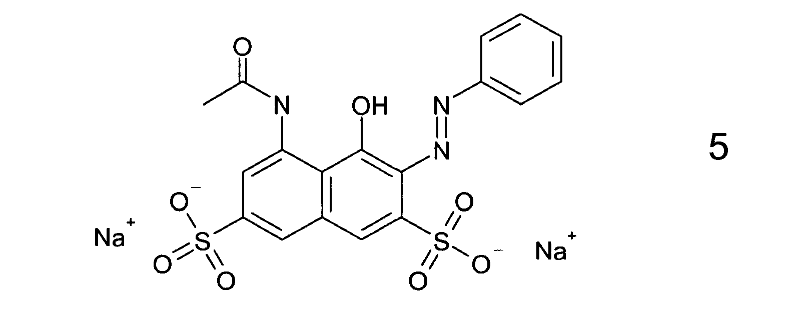
E129:

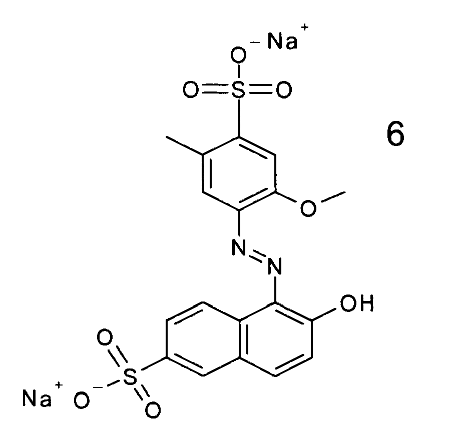

48. Verwendung nach einem der Ansprüche 1-20, **dadurch gekennzeichnet, dass** es sich um eine kohlenhydratarme, insbesondere kohlenhydratfreie, Zusammensetzung handelt.

49. Verwendung nach einem der Ansprüche1-20, **dadurch gekennzeichnet, dass** es sich bei der stofflichen Zusammensetzung um ein, insbesondere koffeinhaltiges, ACK gesüsstes Getränk handelt.

50. Verfahren nach einem der Ansprüche 22-31, **dadurch gekennzeichnet, dass** es sich um eine kohlenhydratarme, insbesondere kohlenhydratfreie, Zusammensetzung handelt.

51. Verfahren nach einem der Ansprüche 22-31, **dadurch gekennzeichnet, dass** es sich bei der stofflichen Zusammensetzung um ein, insbesondere koffeinhaltiges, ACK gesüsstes Getränk handet.

## Claims

1. Use of water-dispersible carotenoid nanoparticles as taste modulators in material compositions.

2. Use according to claim 1, **characterized in that** the carotenoid nanoparticles have a particle size of < 1 µm.

3. Use according to claim 2, **characterized in that** the carotenoid nanoparticles are liquid or solid.

4. Use according to any one of claims 1 to 3, **characterized in that** the carotenoids in the solid carotenoid nanoparticles are X-ray amorphous.

5. Use according to any one of claims 1 to 4, **characterized in that** the carotenoids in the liquid carotenoid nanoparticles are dissolved in a liquid medium or in the form of solubilized mixed micelles.

6. Use according to any one of claims 1 to 5, **characterized in that** the carotenoid nanoparticles exist in liquid or solid formulations, which comprise at least one additive approved for food and/or pharmaceuticals.

7. Use according to claim 6, **characterized in that** the additive is selected from the group consisting of protective colloids, stabilizers against oxidative degradation, emulsifiers, oils, plasticizers, means against caking and polyols.

8. Use according to any one of claims 1 to 7, **characterized in that** the carotenoid nanoparticles of the solid formulations are embedded into a matrix of at least one additive.

9. Use according to any one of claims 6 to 8, **characterized in that** the formulations of the carotenoid nanoparticles contain 0.5 to 30% (w/v) of carotenoid or carotenoids based on the respective total amount.

10. Use according to any one of claims 6 to 9, **characterized in that** the formulations of the carotenoid nanoparticles contain 10 to 80% (w/v) of protective colloid or protective colloids based on the respective total amount.

11. Use according to any one of claims 6 to 10, **characterized in that** the formulations of the carotenoid nanoparticles contain 20 to 70% (w/v) of plasticizer based on the respective total amount.

12. Use according to any one of claims 1 to 11, **characterized in that** the water-dispersible carotenoid nanoparticles effect the reduction of the bitter taste and the bitter after taste of the material compositions.

13. Use according to any one of claims 1 to 12, **characterized in that** the material compositions concern food, beverages, stimulants, sweeteners, animal food, cosmetics and pharmaceuticals.

14. Use according to any one of claims 1 to 13, **characterized in that** the material composition is a low-sugar composition having less than 10 g sugar per litre or kg of composition, respectively, in particular a sugar-free composition.

15. Use according to any one of claims 1 to 14, **characterized in that** the material compositions contain at least one high intensity sweetener HIS as sweetening agent.

16. Use according to any one of claims 1 to 15, **characterized in that** the carotenoid nanoparticles or formulations thereof are contained in solid material compositions in an amount which results in a concentration of 0.1 to 100 ppm.

17. Use according to any one of claims 1 to 15, **characterized in that** the carotenoid nanoparticles or formulations thereof are contained in liquid material compositions in an amount which results in a concentration of 0.1 to 100mg/l.

18. Use according to any one of claims 1 to 17, **characterized in that** the material compositions comprise at least one azo compound comprising at least one azo group, which reduces the bitter taste and bitter after taste.

19. Use according to claim 18, **characterized in that** the material compositions contain 0.1 to 100 ppm of an azo compound or azo compounds based on the respective total amount.

20. Use according to claim 18 or 19, **characterized in that** the azo compounds are selected from the group consisting of E110, E123, E128 and E129:
E123: 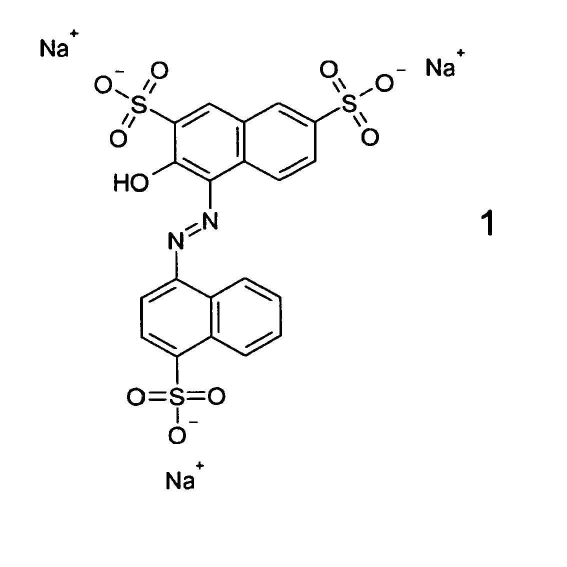
E110: 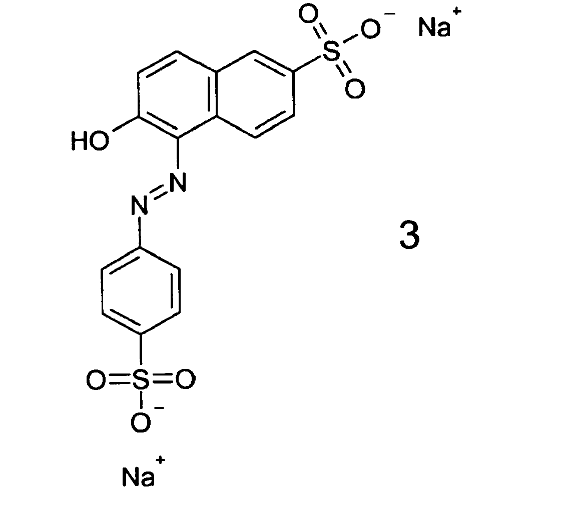
E128: 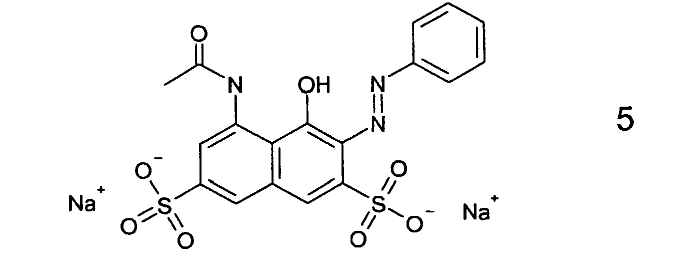
E129:

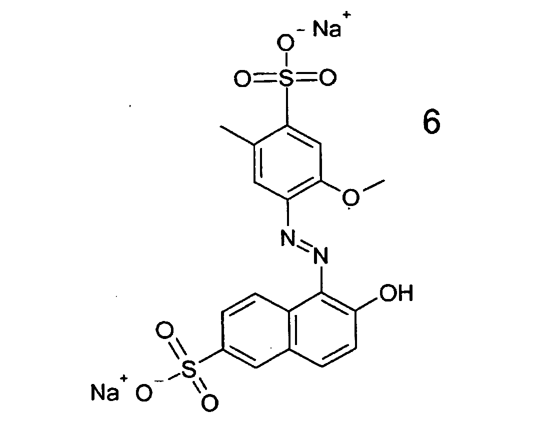

21. Use according to any one of claims 15 to 20, **characterized in that** the one or more HIS is or are selected from the group consisting of acesulfam potassium, aspartame, saccharine and its salts, cyclamate and its salts, aspartame-acesulfam-salt, sucralose, thaumatin, stevia, steviosid, and neohesperidin dihydrochalcone.

22. Process for modulating the taste of material compositions, **characterized in that** at least one type of water-dispersible carotenoid nanoparticles is added to the material compositions.

23. Process according to claim 22, **characterized in that** the water-dispersible carotenoid nanoparticles are added in an amount which results in a concentration of carotenoids of 0.1 to 100 ppm in solid material compositions and in a concentration of carotenoids of 0.1 to 100 mg/l in liquid material compositions.

24. Process according to claim 22 or 23, **characterized in that** the material compositions are food, beverages, stimulants, sweeteners, animal food, cosmetics and pharmaceuticals.

25. Process according to any one of claims 22 to 24, **characterized in that** the material compositions are low-sugar compositions having less than 10 g sugar per litre or kg, respectively, in particular sugar-free compositions.

26. Process according to any one of claims 22 to 25, **characterized in that** the material compositions contain at least one high intensity sweetener HIS as sweetening agent.

27. Process according to any one of claims 22 to 26, **characterized in that** at least one azo compound comprising at least one azo group, which reduces the bitter taste and bitter after taste is added to the material compositions.

28. Process according to claim 27, **characterized in that** 0.1 to 100 ppm of an azo composition or azo compositions are added to the material compositions based on their respective total amount.

29. Process according to claim 27 or 28, **characterized in that** the azo compounds are selected from the group consisting of E110, E123, E128 and E129:
E123: 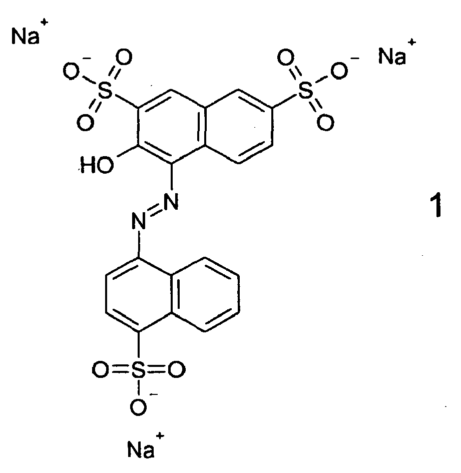
E110: 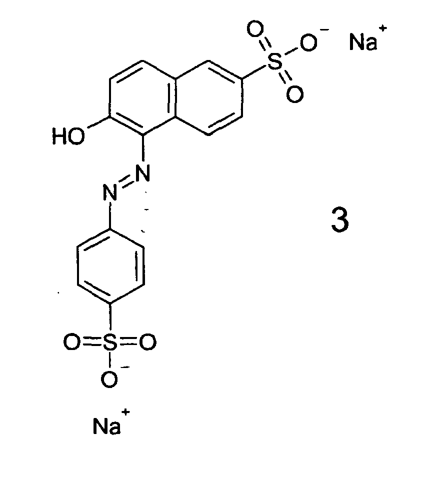
E128: 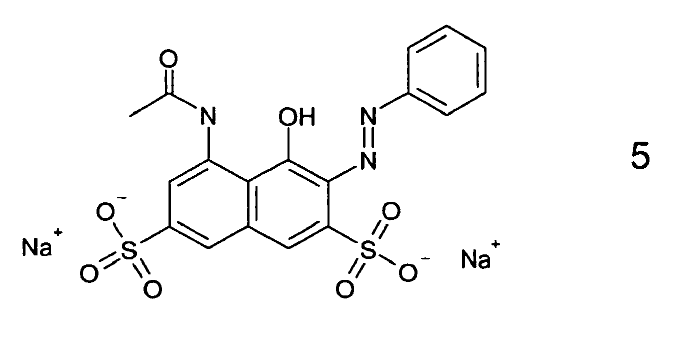
E129:

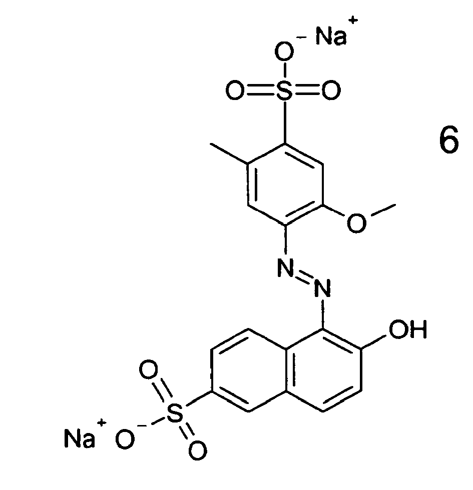

30. Process according to any one of claims 26 to 29, **characterized in that** the one or more HIS are selected from the group consisting of acesulfam potassium, aspartame, saccharine and its salts, cyclamate and its salts, aspartame-acesulfam-salt, sucralose, thaumatin, and neohesperidin dihydrochalcone.

31. Process according to any one of claims 26 to 30, **characterized in that** by the modulation of taste the bitter taste and the bitter after taste of the material compositions is reduced.

32. Taste modulators for material compositions, comprising
(A) at least one type of water-dispersible carotenoid nanoparticles
(B) at least one azo compound comprising at least one azo group.

33. Taste modulators according to claim 32, **characterized in that** the weight ratio of (A) to (B) = 10:1 to 1:20.

34. Taste modulators according to claim 32 or 33, **characterized in that** the carotenoid nanoparticles have a particle size of < 1 µm.

35. Taste modulators according to any one of claims 32 to 34, **characterized in that** the carotenoid nanoparticles are liquid or solid.

36. Taste modulators according to any one of claims 32 to 35, **characterized in that** the carotenoids in the solid carotenoid nanoparticles are X-ray amorphous.

37. Taste modulators according to any one of claims 32 to 36, **characterized in that** the carotenoids in the liquid carotenoid nanoparticles are dissolved in a liquid medium or in the form of solubilized mixed micelles.

38. Taste modulators according to any one of claims 32 to 37, **characterized in that** the carotenoid nanoparticles exist in liquid or solid formulations, which comprise at least one additive approved for food and/or pharmaceuticals.

39. Taste modulators according to claim 38, **characterized in that** the additive is selected from the group consisting of protective colloids, stabilizers against oxidative degradation, emulsifiers, oils, plasticizers, means against caking and polyols.

40. Taste modulators according to claim 38 or 39, **characterized in that** the carotenoid nanoparticles of the solid formulations are embedded into a matrix of at least one additive.

41. Taste modulators according to any one of claims 38 to 40, **characterized in that** the formulations of the carotenoid nanoparticles contain 0.5 to 30% (w/v) of carotenoid or carotenoids based on the respective total amount.

42. Taste modulators according to any one of claims 38 to 41, **characterized in that** the formulations of the carotenoid nanoparticles contain 10 to 80% (w/v) of protective colloid or protective colloids based on the respective total amount.

43. Taste modulators according to any one of claims 38 to 42, **characterized in that** the formulations of the carotenoid nanoparticles contain 20 to 70% (w/v) of plasticizer or plasticizers based on the respective total amount.

44. Taste modulators according to any one of claims 32 to 43, **characterized in that** the azo groups of the azo compounds (B) are linked to aryl groups.

45. Taste modulators according to claim 44, **characterized in that** the aryl groups are phenyl groups and/or naphthyl groups.

46. Taste modulators according to claim 44 or 45, **characterized in that** at least one aryl group is substituted with at least one sulfonic acid group and/or at least one hydroxyl group.

47. Taste modulators according to any one of claims 44 to 46, **characterized in that** the azo compounds (B) are selected from the group consisting of E110, E123, E128 and E129:
E123: 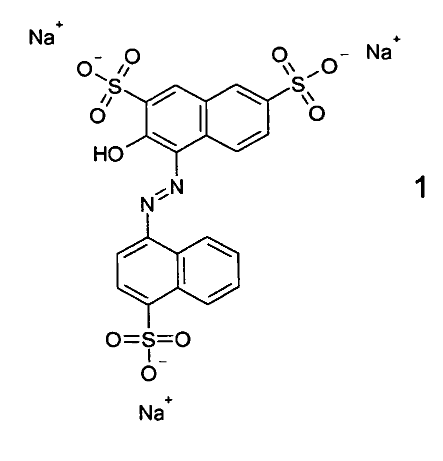
E110: 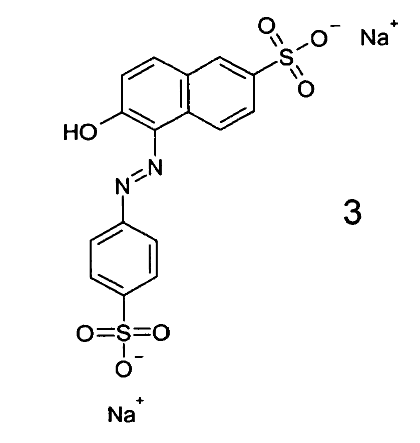
E128: 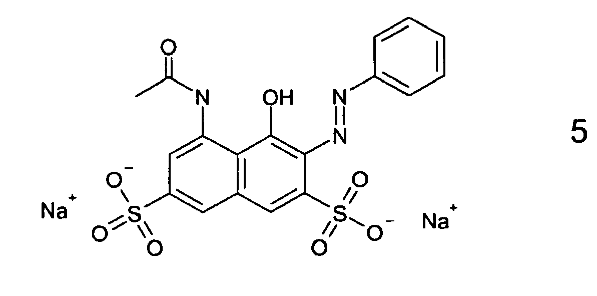
E129:

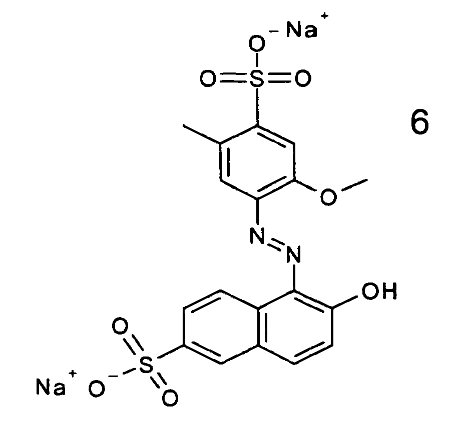

48. Use according to any one of claims 1 to 20, **characterized in that** a low carbohydrate, in particular a carbohydrate-free composition is concerned.

49. Use according to any one of claims 1 to 20, **characterized in that** the material composition concerns a particularly caffeine-containing, ACK-sweetened beverage.

50. Process according to any one of claims 22 to 31, **characterized in that** a low carbohydrate, in particular a carbohydrate-free composition is concerned.

51. Process according to any one of claims 22 to 31, **characterized in that** the material composition concerns a particularly caffeine-containing, ACK-sweetened beverage.

## Revendications

1. Utilisation de nanoparticules de caroténoïdes hydrodispersables comme modulateurs de goût dans des compositions matérielles.

2. Utilisation selon la revendication 1, **caractérisée en ce que** les nanoparticules de caroténoïdes ont une taille de particules < 1 µm.

3. Utilisation selon la revendication 2, **caractérisée en ce que** les nanoparticules de caroténoïdes sont liquides ou solides.

4. Utilisation selon l'une des revendications 1 à 3, **caractérisée en ce que** les caroténoïdes des nanoparticules de caroténoïdes solides sont amorphes aux rayons X.

5. Utilisation selon l'une des revendications 1 à 4, **caractérisée en ce que** les caroténoïdes des nanoparticules de caroténoïdes liquides se présentent sous forme dissoute dans un milieu liquide ou sous forme de micelles mélangées solubilisées.

6. Utilisation selon l'une des revendications 1 à 5, **caractérisée en ce que** les nanoparticules de caroténoïdes se présentent dans des formulations liquides ou solides qui contiennent au moins un additif alimentaire et/ou pharmaceutique homologué.

7. Utilisation selon la revendication 6, **caractérisée en ce que** l'additif est choisi dans le groupe constitué des colloïdes protecteurs, des stabilisateurs contre la dégradation par oxydation, des émulsifiants, des huiles, des plastifiants, des agents antiagglomérants et des polyols.

8. Utilisation selon l'une des revendications 1 à 7, **caractérisée en ce que** les nanoparticules de caroténoïdes des formulations solides sont incluses dans une matrice composée d'au moins un additif.

9. Utilisation selon l'une des revendications 6 à 8, **caractérisée en ce que** les formulations de nanoparticules de caroténoïdes contiennent de 0,5 à 30 % en poids de caroténoïde ou de caroténoïdes, par rapport à leur poids total respectif.

10. Utilisation selon l'une des revendications 6 à 9, **caractérisée en ce que** les formulations de nanoparticules de caroténoïdes contiennent de 10 à 80 % en poids de colloïde protecteur ou de colloïdes protecteurs, par rapport à leur poids total respectif.

11. Utilisation selon l'une des revendications 6 à 10, **caractérisée en ce que** les formulations de nanoparticules de caroténoïdes contiennent de 20 à 70 % en poids de plastifiant, par rapport à leur poids total respectif.

12. Utilisation selon l'une des revendications 1 à 11, **caractérisée en ce que** les nanoparticules de caroténoïdes hydrodispersables servent à réduire le goût amer et l'arrière-goût amer des compositions matérielles.

13. Utilisation selon l'une des revendications 1 à 12, **caractérisée en ce que** les compositions matérielles sont des produits alimentaires, boissons, produits de luxe, édulcorants, aliments pour animaux, produits cosmétiques et pharmaceutiques.

14. Utilisation selon l'une des revendications 1 à 13, **caractérisée en ce que** la composition matérielle est une composition pauvre en sucre, contenant moins de 10 g de sucre par litre ou par kg, et en particulier une composition exempte de sucre.

15. Utilisation selon l'une des revendications 1 à 14, **caractérisée en ce que** les compositions matérielles contiennent au moins un édulcorant de haute intensité (HIS) en tant qu'édulcorant.

16. Utilisation selon l'une des revendications 1 à 15, **caractérisée en ce que** les nanoparticules de caroténoïdes ou leurs formulations sont présentes dans les compositions matérielles solides en quantité telle qu'il en résulte une concentration de caroténoïdes de 0,1 à 100 ppm.

17. Utilisation selon l'une des revendications 1 à 15, **caractérisée en ce que** les nanoparticules de caroténoïdes ou leurs formulations sont présentes dans les compositions matérielles liquides en quantité telle qu'il en résulte une concentration de caroténoïdes de 0,1 à 100 mg/l.

18. Utilisation selon l'une des revendications 1 à 17, **caractérisée en ce que** les compositions matérielles contiennent au moins un composé azoïque avec au moins un groupe azoïque réduisant le goût et l'arrière-goût amers.

19. Utilisation selon la revendication 18, **caractérisée en ce que** les compositions matérielles contiennent de 0,1 à 100 ppm de composé azoïque ou de composés azoïques, par rapport à leur poids total respectif.

20. Utilisation selon la revendication 18 ou la revendication 19, **caractérisée en ce que** les composés azoïques sont choisis dans le groupe constitué de E110, E123, E128 et E129 :
E123 : 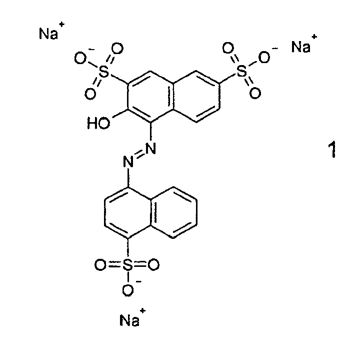
E110 : 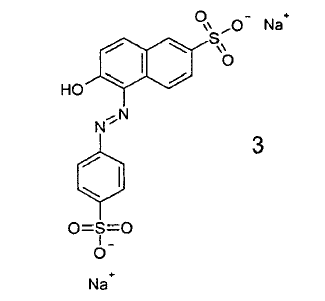
E128 : 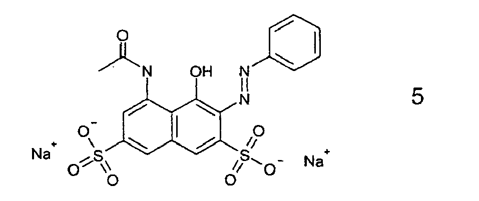
E129:

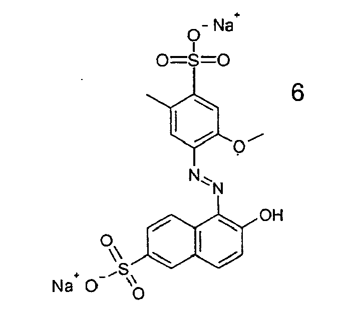

21. Utilisation selon l'une des revendications 15 à 20, **caractérisée en ce que** le ou les HIS est ou sont choisis dans le groupe composé de l'acésulfame de potassium, l'aspartame, la saccharine et ses sels, le cyclamate et ses sels, le sel d'aspartame-acésulfame, le sucralose, la thaumatine, la stévia, le stévioside et la néohespéridine-dihydrochalcone.

22. Procédé pour la modulation de goût de compositions matérielles, **caractérisé en ce que** l'on ajoute au moins un type de nanoparticules de caroténoïdes hydrodispersables aux compositions matérielles.

23. Procédé selon la revendication 22, **caractérisé en ce que** les nanoparticules de caroténoïdes hydrodispersables sont ajoutées en quantité telle qu'il en résulte une concentration de caroténoïdes de 0,1 à 100 ppm dans les compositions matérielles solides et une concentration de caroténoïdes de 0,1 à 100 mg/l dans les compositions matérielles liquides.

24. Procédé selon la revendication 22 ou 23, **caractérisé en ce que** les compositions matérielles sont des produits alimentaires, boissons, produits de luxe, édulcorants, aliments pour animaux, produits cosmétiques et pharmaceutiques.

25. Procédé selon la revendication 22 à 24, **caractérisé en ce que** les compositions matérielles sont des compositions pauvres en sucre, contenant moins de 10 g de sucre par litre ou par kg, et en particulier des compositions exemptes de sucre.

26. Procédé selon l'une des revendications 22 à 25, **caractérisé en ce que** les compositions matérielles contiennent au moins un édulcorant de haute intensité (HIS) en tant qu'édulcorant.

27. Procédé selon l'une des revendications 22 à 26, **caractérisé en ce qu'**au moins un composé azoïque avec au moins un groupe azoïque réduisant le goût et l'arrière-goût amers est encore ajouté aux compositions matérielles.

28. Procédé selon la revendication 27, **caractérisé en ce que** de 0,1 à 100 ppm de composé azoïque ou de composés azoïques sont ajoutés aux compositions matérielles, par rapport à leur poids total respectif.

29. Procédé selon la revendication 27 ou 28, **caractérisé en ce que** les composés azoïques sont choisis dans le groupe constitué de E110, E123, E128 et E129 :
E123 : 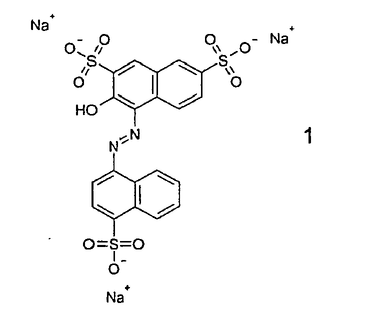
E110 : 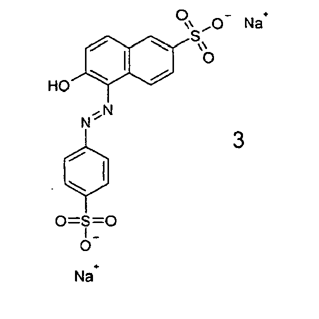
E128 : 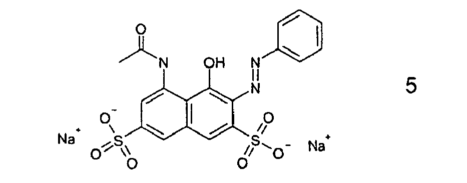
E129 :

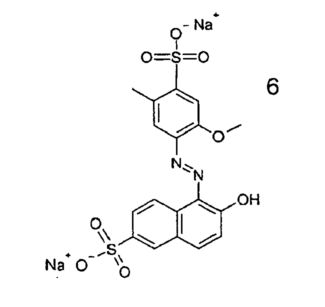

30. Procédé selon l'une des revendications 26 à 29, **caractérisé en ce que** le ou les HIS sont choisis dans le groupe constitué de l'acésulfame de potassium, l'aspartame, la saccharine et ses sels, le cyclamate et ses sels, le sel d'aspartame-acésulfame, le sucralose, la thaumatine et la néohespéridine-dihydrochalcone.

31. Procédé selon l'une des revendications 22 à 30, **caractérisé en ce que** la modulation de goût réduit le goût amer et l'arrière-goût amer des compositions matérielles.

32. Modulateurs de goût pour compositions matérielles, contenant :
(A) au moins un type de nanoparticules de caroténoïdes hydrodispersables ;
(B) au moins un composé azoïque, contenant au moins un groupe azoïque.

33. Modulateurs de goût selon la revendication 32, **caractérisés en ce que** le rapport de poids entre (A) et (B) = 10 : 1 à 1 : 20.

34. Modulateurs de goût selon la revendication 32 ou 33, **caractérisés en ce que** les nanoparticules de caroténoïdes ont une taille de particules < 1 µm.

35. Modulateurs de goût selon l'une des revendications 32 à 34, **caractérisés en ce que** les nanoparticules de caroténoïdes sont liquides ou solides.

36. Modulateurs de goût selon l'une des revendications 32 à 35, **caractérisés en ce que** les caroténoïdes des nanoparticules de caroténoïdes solides sont amorphes aux rayons X.

37. Modulateurs de goût selon l'une des revendications 32 à 36, **caractérisés en ce que** les caroténoïdes des nanoparticules de caroténoïdes liquides se présentent sous forme dissoute dans un milieu liquide ou sous forme de micelles mélangées solubilisées.

38. Modulateurs de goût selon l'une des revendications 32 à 37, **caractérisés en ce que** les nanoparticules de caroténoïdes se présentent dans des formulations liquides ou solides qui contiennent au moins un additif alimentaire et/ou pharmaceutique homologué.

39. Modulateurs de goût selon la revendication 38, **caractérisés en ce que** l'additif est choisi dans le groupe constitué des colloïdes protecteurs, des stabilisateurs contre la dégradation par oxydation, des émulsifiants, des huiles, des plastifiants, des agents antiagglomérants et des polyols.

40. Modulateurs de goût selon la revendication 38 ou 39, **caractérisés en ce que** les nanoparticules de caroténoïdes des formulations solides sont incluses dans une matrice composée d'au moins un additif.

41. Modulateurs de goût selon l'une des revendications 38 à 40, **caractérisés en ce que** les formulations de nanoparticules de caroténoïdes contiennent de 0,5 à 30 % en poids de caroténoïde ou de caroténoïdes, par rapport à leur poids total respectif.

42. Modulateurs de goût selon l'une des revendications 38 à 41, **caractérisés en ce que** les formulations de nanoparticules de caroténoïdes contiennent de 10 à 80 % en poids de colloïde protecteur ou de colloïdes protecteurs, par rapport à leur poids total respectif.

43. Modulateurs de goût selon l'une des revendications 38 à 42, **caractérisés en ce que** les formulations de nanoparticules de caroténoïdes contiennent de 20 à 70 % en poids de plastifiant ou de plastifiants, par rapport à leur poids total respectif.

44. Modulateurs de goût selon l'une des revendications 32 à 43, **caractérisés en ce que** les groupes azoïques des composés azoïques (B) sont liés à des groupes aryles.

45. Modulateurs de goût selon la revendication 44, **caractérisés en ce que** les groupes aryles sont des groupes phényles et/ou des groupes naphtyles.

46. Modulateurs de goût selon la revendication 44 ou 45, **caractérisés en ce qu'**au moins un groupe aryle est substitué par au moins un groupe acide sulfonique et/ou au moins un groupe hydroxyle.

47. Modulateurs de goût selon l'une des revendications 44 à 46, **caractérisés en ce que** les composés azoïques (B) sont choisis dans le groupe constitué de E110, E123, E128 et E129 :
E123 : 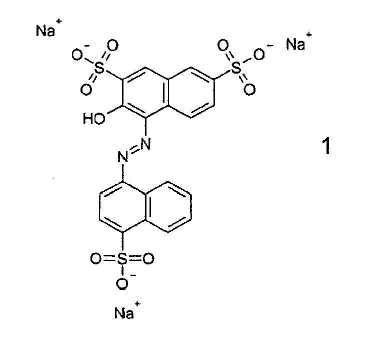
E110 : 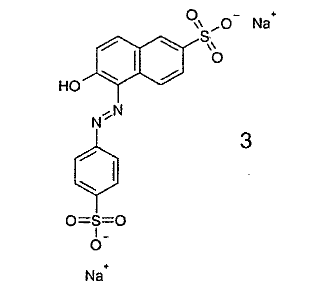
E128 : 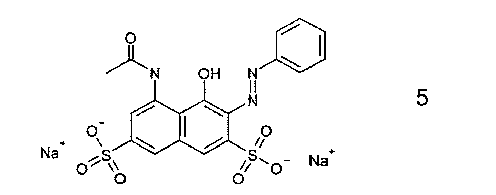
E129 :

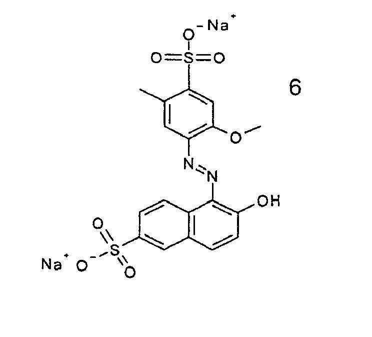

48. Utilisation selon l'une des revendications 1 à 20, **caractérisée en ce que** la composition est une composition pauvre en glucides, et en particulier exempte de glucides.

49. Utilisation selon l'une des revendications 1 à 20, **caractérisée en ce que** la composition matérielle est une boisson édulcorée à l'acésulfame de potassium, contenant en particulier de la caféine.

50. Procédé selon l'une des revendications 22 à 31, **caractérisé en ce que** la composition est une composition pauvre en glucides, et en particulier exempte de glucides.

51. Procédé selon l'une des revendications 22 à 31, **caractérisé en ce que** la composition matérielle est une boisson édulcorée à l'acésulfame de potassium, contenant en particulier de la caféine.
